(19) Europäisches Patentamt
European Patent Office
Office européen des brevets

(11) EP 4 398 865 B1

(12) EUROPEAN PATENT SPECIFICATION

(45) Date of publication and mention of the grant of the patent:
**29.10.2025 Bulletin 2025/44**

(21) Application number: **22772917.5**

(22) Date of filing: **01.09.2022**

(51) International Patent Classification (IPC):
***A61K 8/31*** (2006.01) ***A61K 8/81*** (2006.01)
***A61K 8/898*** (2006.01) ***A61Q 1/10*** (2006.01)

(52) Cooperative Patent Classification (CPC):
**A61K 8/8152; A61K 8/31; A61K 8/898; A61Q 1/10;**
A61K 2800/43; A61K 2800/884

(86) International application number:
**PCT/EP2022/074343**

(87) International publication number:
**WO 2023/036686 (16.03.2023 Gazette 2023/11)**

# (54) MAKEUP KIT COMPRISING AN AQUEOUS MAKEUP COMPOSITION AND A CONTINUOUS OILY-PHASE FIXING COMPOSITION WITH A HYDROPHOBIC FILM-FORMING POLYMER

MAKEUP-KIT MIT EINER WÄSSRIGEN MAKEUP-ZUSAMMENSETZUNG UND EINER KONTINUIERLICHEN ÖLPHASENFIXIERZUSAMMENSETZUNG MIT EINEM HYDROPHOBEN FILMBILDENDEN POLYMER

KIT DE MAQUILLAGE COMPRENANT UNE COMPOSITION DE MAQUILLAGE AQUEUSE ET UNE COMPOSITION DE FIXATION À PHASE HUILEUSE CONTINUE AVEC UN POLYMÈRE FILMOGÈNE HYDROPHOBE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2021 FR 2109496**

(43) Date of publication of application:
**17.07.2024 Bulletin 2024/29**

(73) Proprietor: **L'OREAL**
**75008 Paris (FR)**

(72) Inventors:
- **ILEKTI, Philippe**
**94152 CHEVILLY LARUE (FR)**
- **WOLLBRETT-BLITZ, Judith**
**94152 CHEVILLY LARUE (FR)**
- **PLOS, Grégory**
**94152 CHEVILLY LARUE (FR)**

(74) Representative: **L'Oreal**
**Service D.I.P.I.**
**9, rue Pierre Dreyfus**
**92110 Clichy (FR)**

(56) References cited:
**FR-A1- 2 936 420** **FR-A1- 3 088 206**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 398 865 B1

## Description

### Technical field

The present invention is directed toward proposing for the field of caring for and/or making up keratin materials, notably the eyebrows and the skin around the eyebrows, a novel two-composition makeup kit that is most particularly advantageous with regard to its technical performance and the esthetic result that it affords the user when it is applied to said keratin materials and in particular to the eyebrows.

Many eyebrow makeup products are known in the prior art for coloring keratin materials, in particular the eyebrows. They may be based on makeup products: pencils, hot-cast powders, ointments, etc. and also on the use of direct dyes, but these products do not durably color the skin around the eyebrows and the results change in the course of the day.

Products based on oxidation dyes also exist, but these products require mixing at the time of use. This type of product does not make it possible to preview the result before the rinsing step and also does not make it possible to correct the result obtained.

One solution also consists in applying a water-resistant makeup product based on the combination of an MQ-type silicone resin with a high molecular weight silicone resin, but the makeup result of this solution lasts for a maximum of two days (Wunderbrow 2® product range from Wonder 2).

A final solution known from the prior art consists in applying products containing a self-tanning active agent such as dihydroxyacetone DHA, optionally combined with direct dyes, but this solution is limited in terms of the colors offered and the colors are mainly orangey.

Users of coloring products for keratin materials such as the eyebrows are in search of a surface makeup gesture which has a semi-permanent result, i.e. a result that can be eliminated after a few days, while at the same time maintaining control over the final result and in particular making it possible to visualize said result in advance. Specifically, users wish to be able to make corrections or retouches in the event of overrun marking during application before the fixing of the result for several days.

FR2936420 A1 discloses a make-up kit for care of keratin fibres, particularly eyebrows and lashes, comprising a first aqueous make-up base coat composition comprising a silicone surfactant and a crosslinked polyelectrolyte, and optionally colourants, and a second composition which may comprise a volatile hydrocarbon oil.

The need remains to find a novel process for semi-permanently making up keratin materials, notably the eyebrows and the skin around the eyebrows, and also novel suitable compositions which make it possible to obtain long-lasting makeup for several days, which offer a wide range of colors and which make it possible to check the final result before it is fixed.

In the course of its research, the Applicant discovered, unexpectedly, that this objective was achieved by means of a two-step makeup process consisting in successively applying to said keratin materials:

a) at least one first coat with a first aqueous "base-coat" makeup composition (A) comprising at least one water-soluble dye; and

b) onto the coat formed by composition (A), applying at least one second coat of a second "top-coat" fixing composition (B) comprising an oily continuous phase containing:

i) at least one hydrophobic film-forming polymer other than silicone acrylate copolymers; and

ii) at least one volatile hydrocarbon-based oil.

The first step of applying the "base-coat" makeup composition (A) makes it possible to color the eyebrows and their contour and allows the consumer to correct possible application errors, change the color and/or shape contours and thus be able to preview the final result before fixing.

The second step of applying the "top-coat" fixing composition (B) makes it possible to fix the makeup obtained on the eyebrows and the skin around the eyebrows and to obtain a semi-permanent makeup with good staying power which has good resistance to rubbing, to sebum, to washing with a shower gel and a makeup remover such as micellar water for a period of more than 2 days, notably from 3 to 5 days.

This discovery forms the basis of the invention.

### Subjects of the invention

Thus, according to one of its aspects, the present invention relates to a kit for making up keratin materials, in particular the eyebrows and the skin around the eyebrows, comprising:

a) a first aqueous "base-coat" makeup composition (A) comprising at least one water-soluble dye; and
b) a second "top-coat" fixing composition (B) comprising a continuous oily phase containing:

i) at least one hydrophobic film-forming polymer other than silicone acrylate copolymers; and
ii) at least one volatile hydrocarbon-based oil.

The invention also relates to a cosmetic process for making up keratin materials, in particular the eyebrows and the skin around the eyebrows, comprising:

- at least a first step of applying a first coat with a composition (A) as defined previously to said keratin materials, and
- at least a second step of applying, to the coat formed by composition (A), at least one second coat of a composition (B) as defined previously.

**Definitions**

In the context of the present invention, the term "keratin materials" notably means the eyebrows and the skin around the eyebrows.
The term "physiologically acceptable" means compatible with the skin and/or its integuments, which has a pleasant color, odor and feel, and which does not cause any unacceptable discomfort (stinging, tautness or redness) liable to put the consumer off using this composition.
For the purposes of the present invention, the expression "composition comprising a continuous oily phase" refers to any composition which may be either an oily anhydrous composition or a water-in-oil emulsion, also known as an inverse emulsion. This composition may be in the form of an anhydrous composition or of an inverse emulsion containing less than 40% by weight of water, preferably less than 30% by weight of water or even less than 15% of water relative to its total weight. For the purposes of the present invention, the expression "anhydrous composition" denotes any composition containing less than 5% by weight of water, preferably less than 2% by weight of water, or even less than 0.5% of water, relative to its total weight, and notably a composition which is free of water.
The term "silicone acrylate copolymer" means a vinyl polymer grafted onto the main chain via an organosiloxane group.

**Makeup composition (A)**

According to the present invention, the semi-permanent makeup composition (A) comprises at least one aqueous phase containing at least one water-soluble dye, in particular a direct acid dye.

Aqueous phase

The makeup composition (A) according to the invention comprises an aqueous phase, which may preferably form a continuous phase of the composition.
The term "aqueous phase" means a phase comprising water and also all the water-soluble or water-miscible solvents and ingredients (miscibility in water of greater than 50% by weight at 25° C).
Preferably, composition (A) according to the invention comprises a continuous aqueous phase. The expression "composition with a *continuous* aqueous phase" means that the composition has a conductivity, measured at 25° C, of less than 23 μS/cm (microSiemens/cm), the conductivity being measured, for example, using an MPC227® conductimeter from Mettler Toledo and an Inlab730® conductivity measuring cell. The measuring cell is immersed in the composition so as to remove the air bubbles that might be formed between the two electrodes of the cell. The conductivity reading is taken once the conductimeter value has stabilized. A mean is determined on at least three successive measurements.
The aqueous phase comprises water. It may also comprise at least one pro-penetrating solvent.
The term "pro-penetrating compound" means any substance which is capable of promoting the penetration of at least one other substance such as a dye into keratin materials such as the skin and the eyebrows.
The pro-penetrating solvents that may be used according to the present invention are preferably chosen from glycols such as propanediol, propylene glycol, butylene glycol, pentylene glycol; cyclic glycol esters such as propylene carbonate of the structure:

[Chem 1]

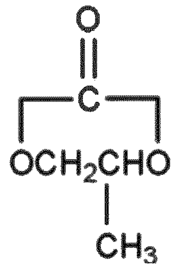

butylene carbonate of the structure

[Chem 2]

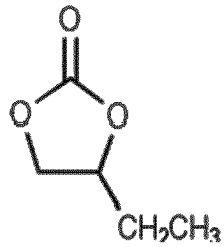

benzyl alcohol
and mixtures thereof.

The pro-penetrating solvent is present in composition (A) in a content of less than or equal to 30% by weight, preferably less than or equal to 25% by weight and more preferentially ranging from 4% to 25% by weight relative to the total weight of composition (A).

Water is preferably present in composition (A) according to the present patent application in a content of greater than or equal to 40% by weight, more preferentially ranging from 45% to 90% by weight, in particular ranging from 50% to 85% by weight relative to the total weight of composition (A). This content of aqueous phase includes not only the water deliberately added to the composition, but also the water that may be introduced by other ingredients.

The pH of the makeup composition (A) according to the invention is preferably less than 6, more preferentially ranging from 2 to less than 6, and more particularly ranging from 2 to 5.

The pH can be obtained and/or adjusted conventionally by adding alkaline agents, such as aqueous ammonia, monoethanolamine, diethanolamine, triethanolamine, isopropanolamine, 2-amino-2-methyl-1-propanol, 1,3-propanediamine, an alkali metal or ammonium carbonate or bicarbonate, for example sodium bicarbonate, an organic carbonate, such as guanidine carbonate, or alternatively an alkali metal hydroxide, such as sodium hydroxide or potassium hydroxide, it being possible, of course, for all these compounds to be taken alone or as a mixture.

To obtain and/or adjust the pH, use may also be made of acidifying agents, such as hydrochloric acid, acetic acid, lactic acid, citric acid and phosphoric acid.

Citric acid will preferably be used.

Direct acid dye

A composition (A) according to the invention includes at least one water-soluble dye, in particular a direct acid dye.

The term "direct acid dye" means an acid dye which is present in free form in the composition. In particular, it is not supported on a support, such as alumina. Thus, preferably, the direct acid dye is dissolved in the aqueous phase.

In addition, it is compatible with application around/on the eyebrows.

The direct acid dye is present in a concentration of less than 5% by weight, preferably less than 3% by weight and more particularly between 0.01% and 3% by weight relative to the total weight of the composition.

It is chosen from dyes containing an anthraquinone, azine, azo, polymethine, triarylmethane or xanthene nucleus, and dyes comprising at least one $-NO_2$ group.

The dyes with an anthraquinone nucleus are preferably those having the following formulae:

[Chem 3]

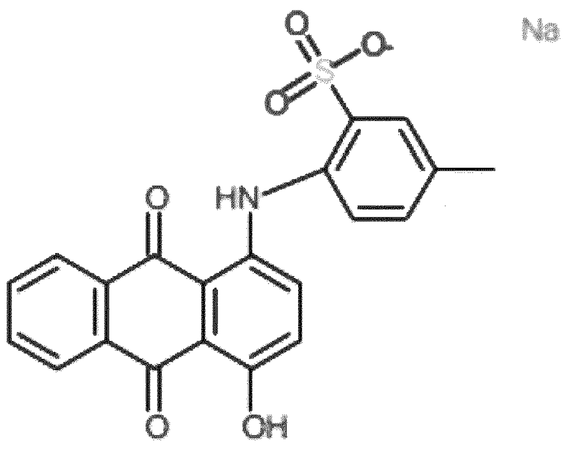

CI60730·(Acid·Violet·43)¶

[Chem 4]

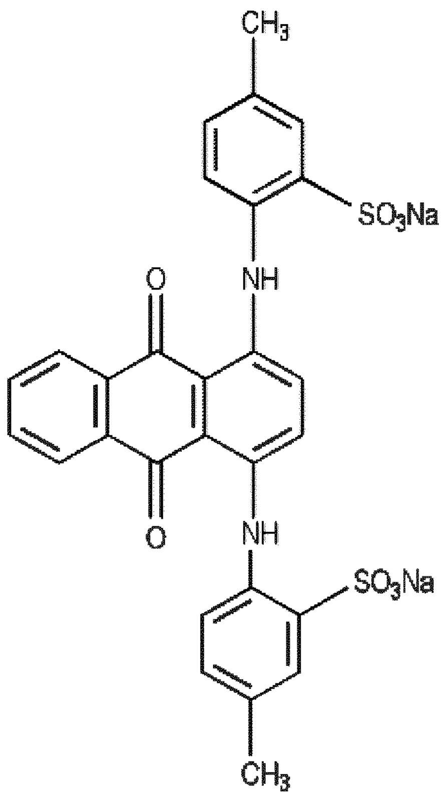

CI61570 (Acid Green 25)

[Chem 5]

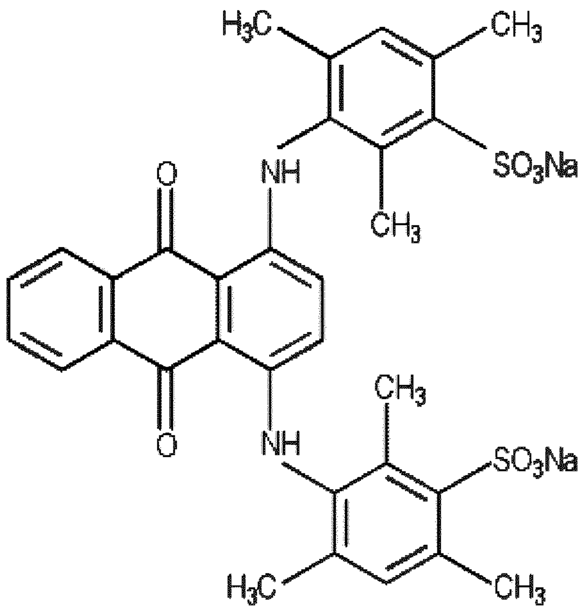

CI61585 (Acid Blue 80)

[Chem 6]

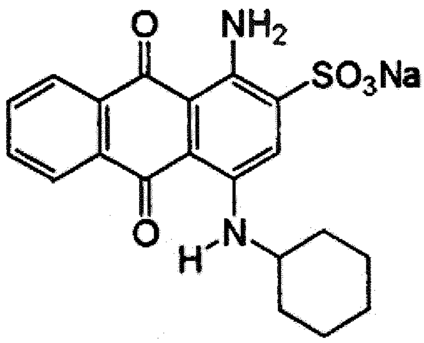

CI62045 (Acid Blue 62)

The dye with an azine nucleus is preferably the one having the following formula:

[Chem 7]

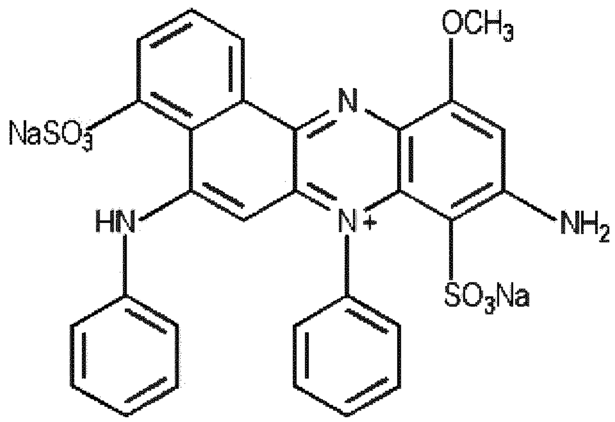

CI50325 (Acid Violet 50)

The dyes with an azo nucleus are preferably those having the following formulae:

[Chem 8]

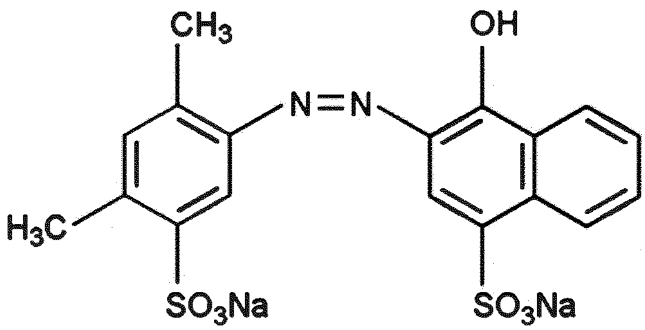

CI14700 (FD&C Red 4)

[Chem 9]

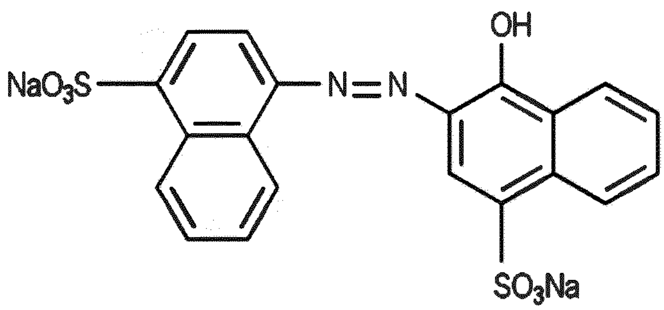

CI14720 (Acid Red 4)

[Chem 10]

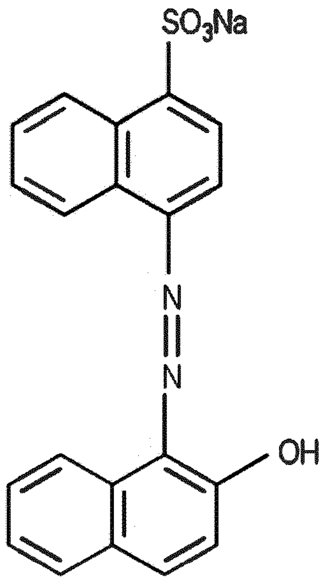

CI15620 (Acid Red 88)

[Chem 11]

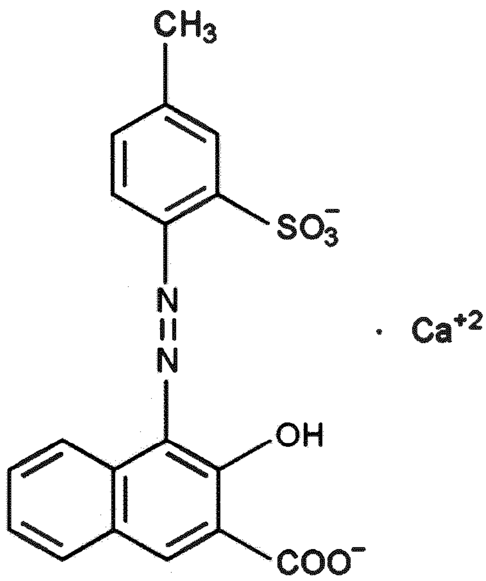

CI15850 (D&C Red 7)

[Chem 12]

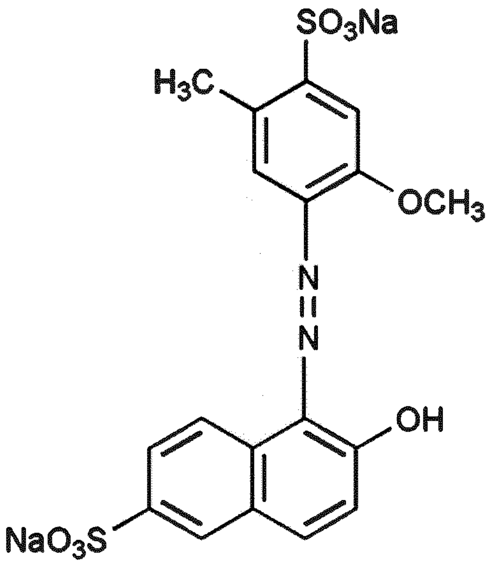

CI16305 (FD&C Red 40)

[Chem 13]

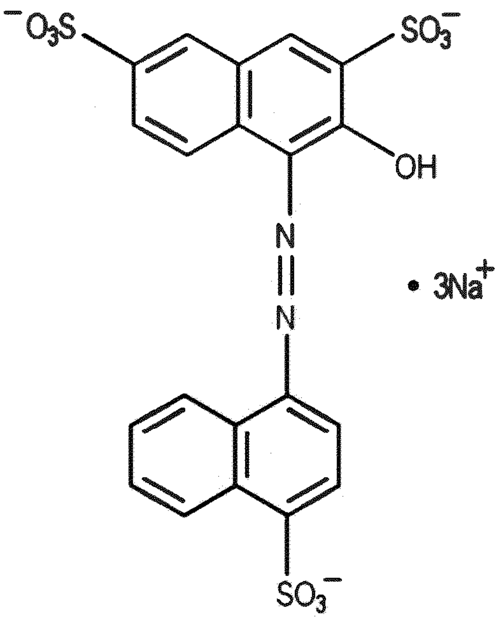

CI16185 (Acid Red 27)

[Chem 14]

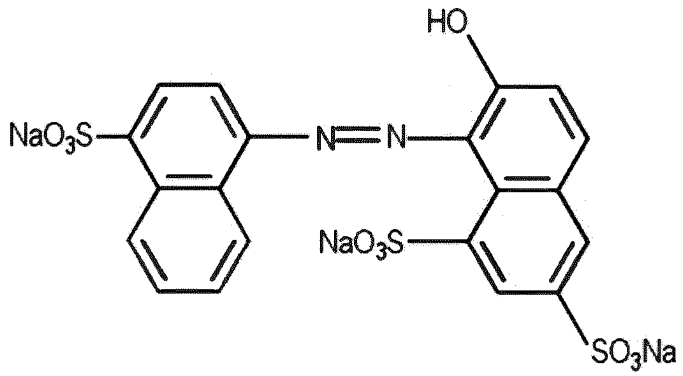

CI16255 (Acid Red 18)

[Chem 15]

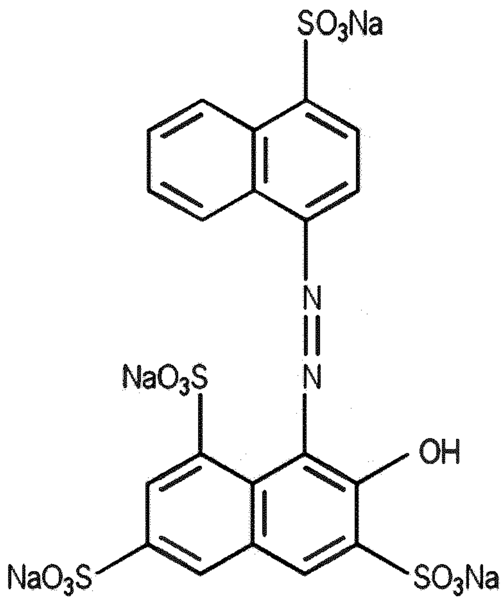

CI16290 (Acid Red 41)

[Chem 16]

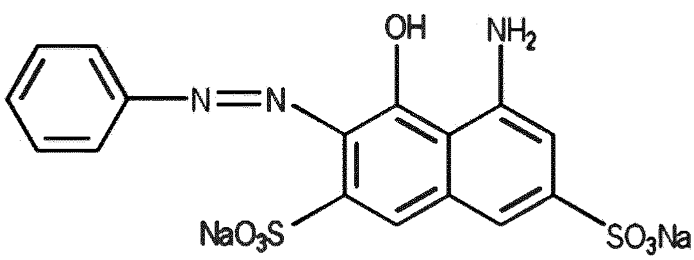

CI17200 (Acid Red 33)

[Chem 17]

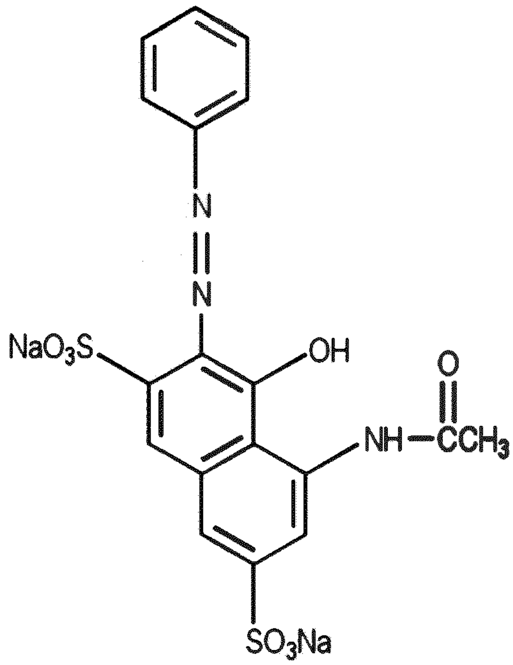

CI18050 (Acid Red 1)

[Chem 18]

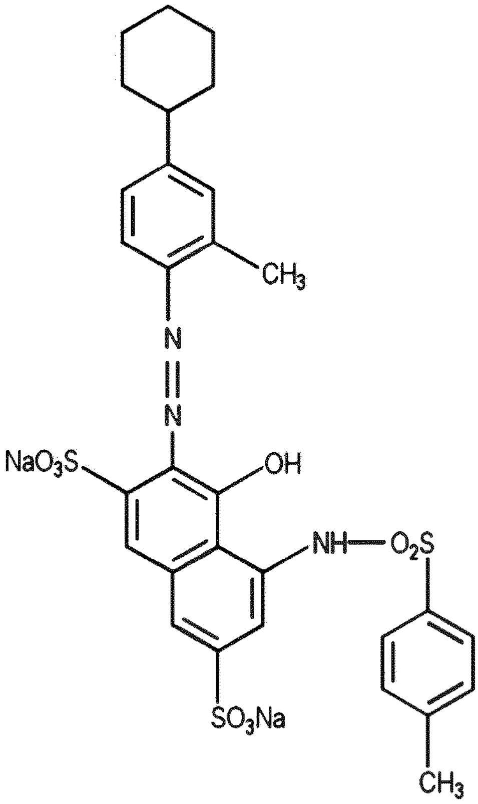

CI18130 (Acid Red 155)

[Chem 19]

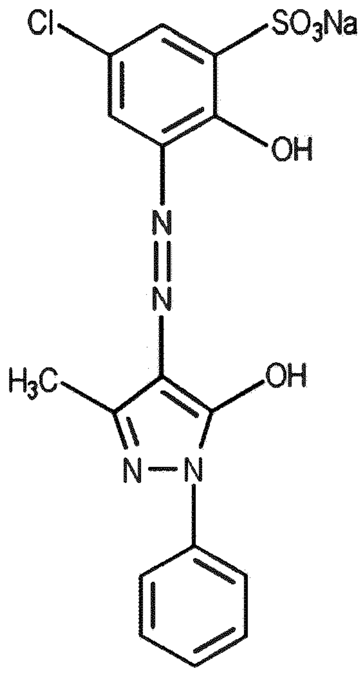

CI18736 (Acid Red 180)

[Chem 20]

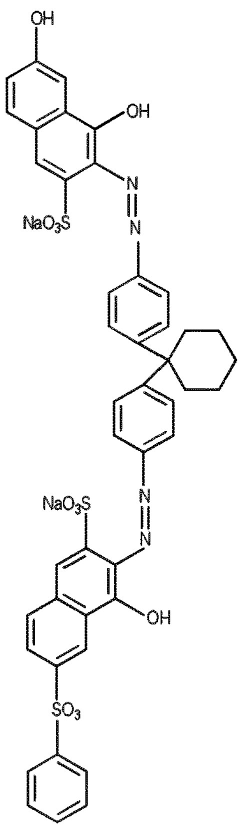

CI24790 (Acid Red 163)

[Chem 21]

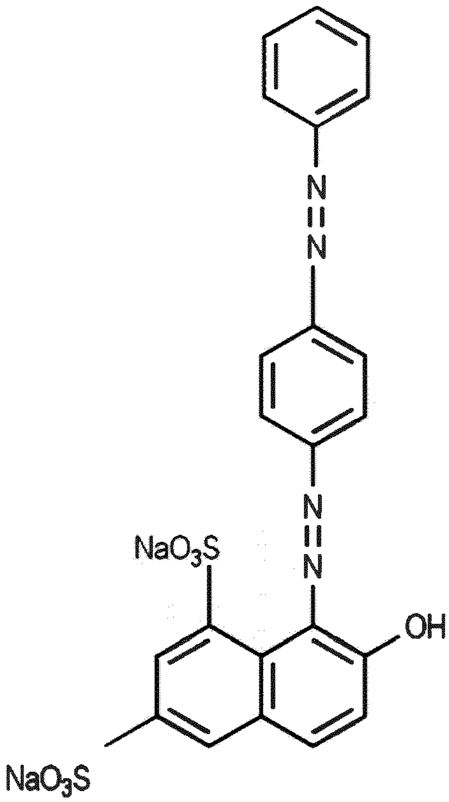

CI27290 (Acid Red 73)

[Chem 22]

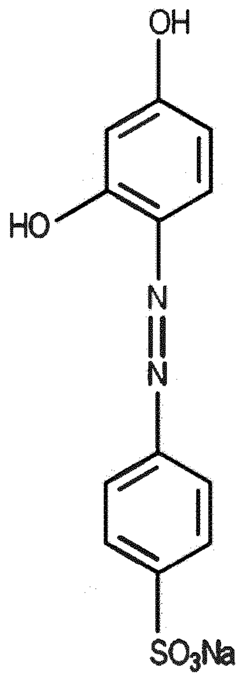

CI27290 (Acid Orange 6)

[Chem 23]

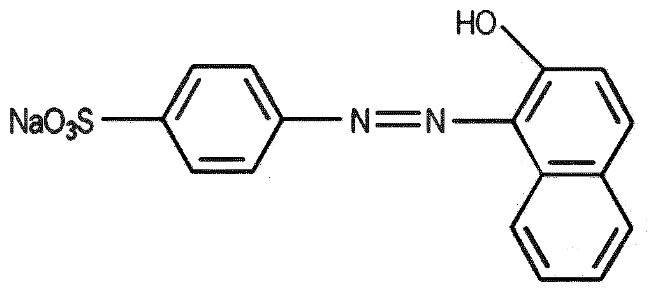

CI15510 (Acid Orange 7)

[Chem 24]

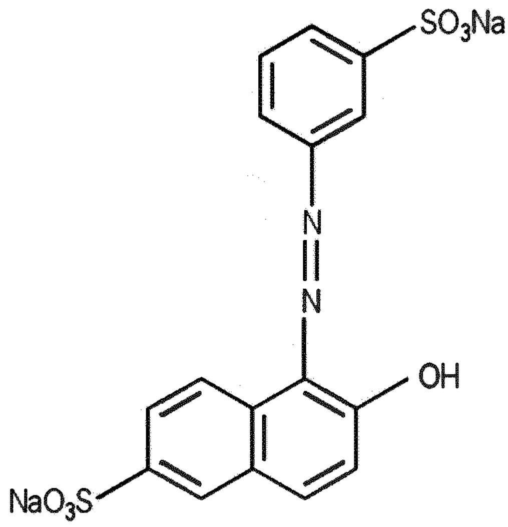

CI15980 (Food Orange 2)

[Chem 25]

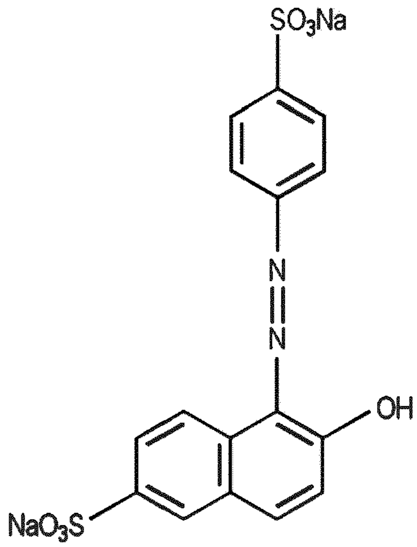

CI15985 (Food Yellow 3)

[Chem 26]

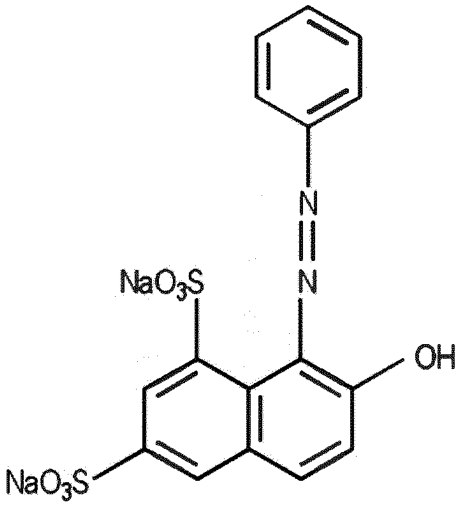

CI16230 (Acid Orange 10)

[Chem 27]

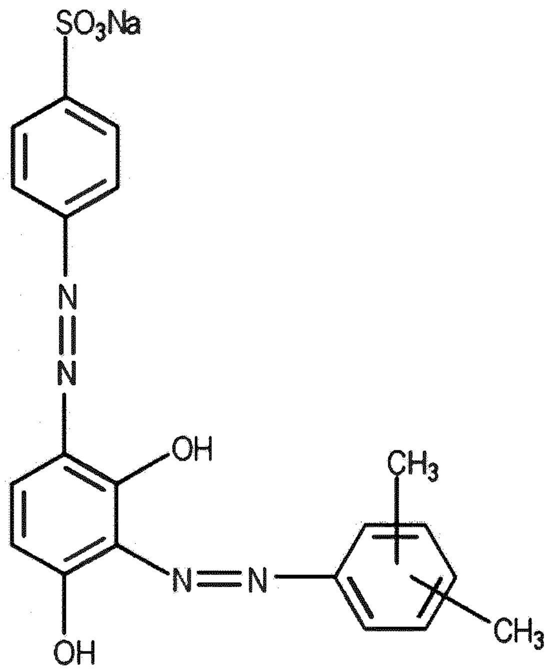

CI20170 (Acid Orange 24)

[Chem 28]

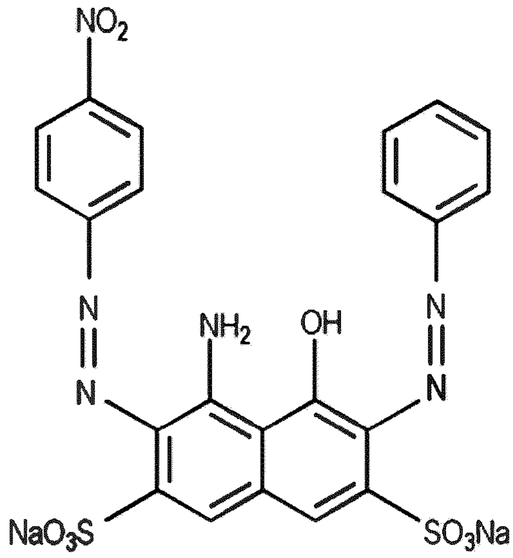

CI20470 (Acid Black 1)

[Chem 29]

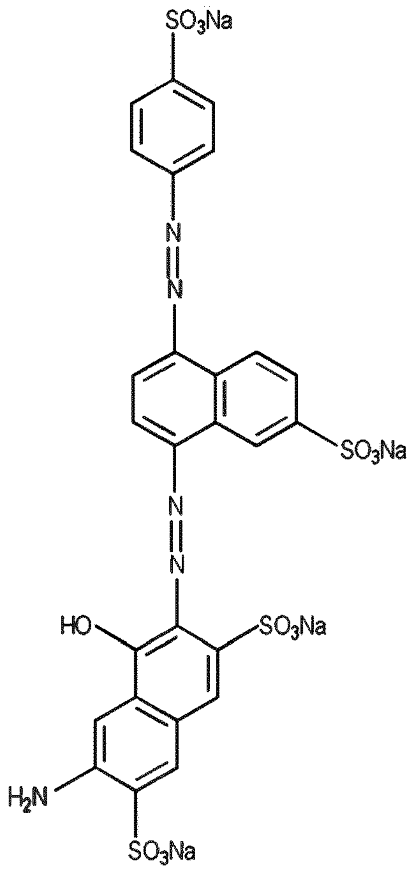

CI27755 (Food Black 2)

[Chem 30]

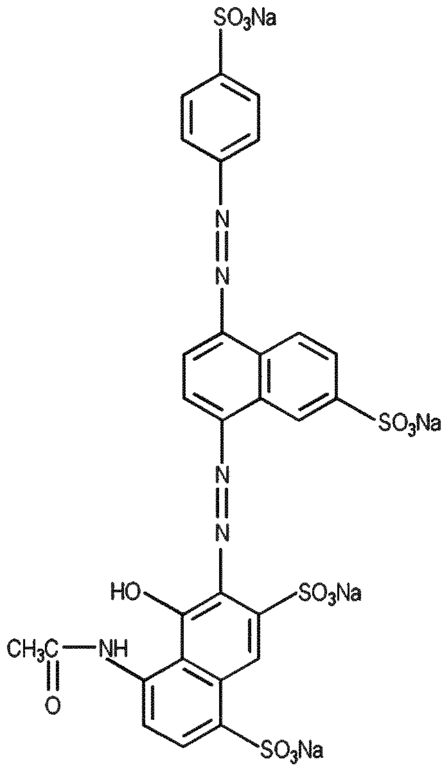

CI278440 (Food Black 1)

[ Chem 31]

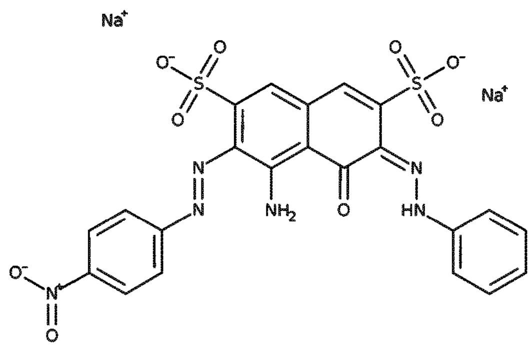

CI50420 (Acid Black 2)

[Chem 32]

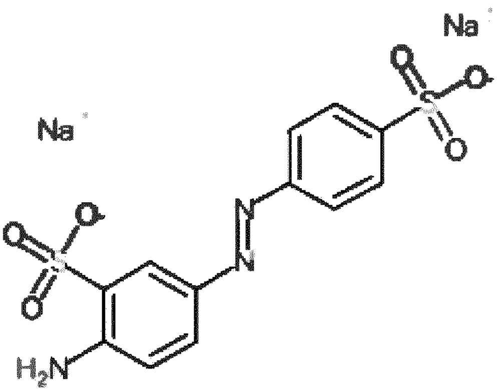

CI13015 (Acid Yellow 9)

[Chem 33]

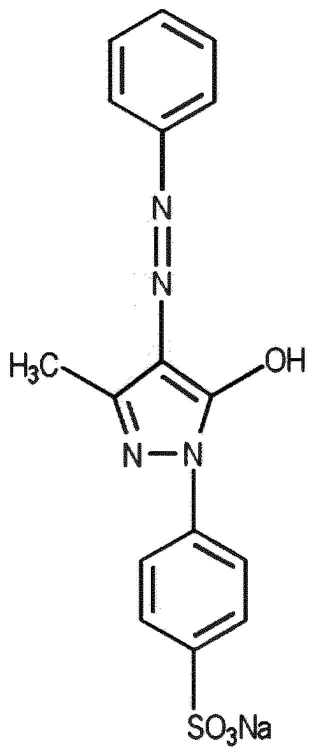

CI18820 (Acid Yellow 11)

[Chem 34]

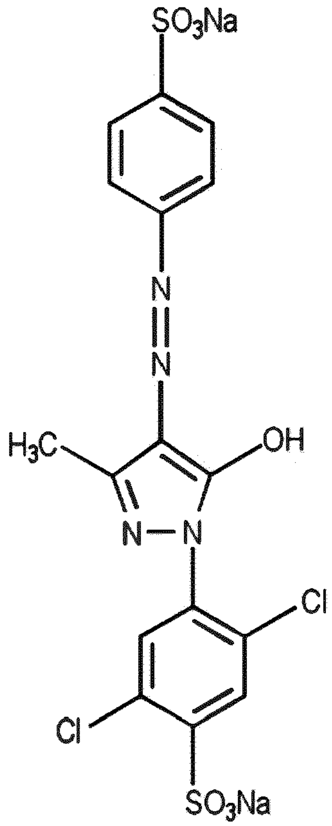

CI18965 (Acid Yellow 17)

[Chem 35]

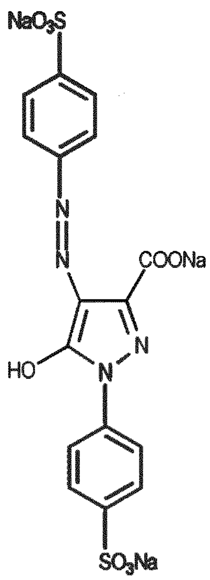

CI19140 (Acid Yellow 23)

The dye with a polymethine nucleus is preferably the one having the following formula:

[Chem 36]

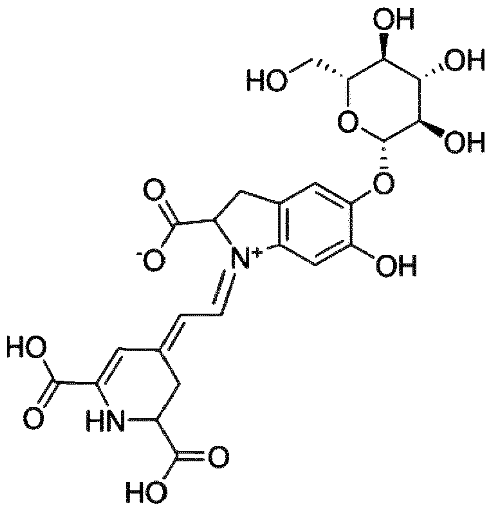

Betanin

The dyes with a triarylmethane nucleus are preferably those having the following formulae:

[Chem 37]

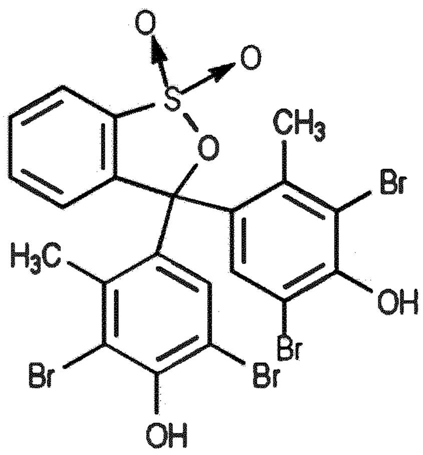

Bromocresol Green

[Chem 38]

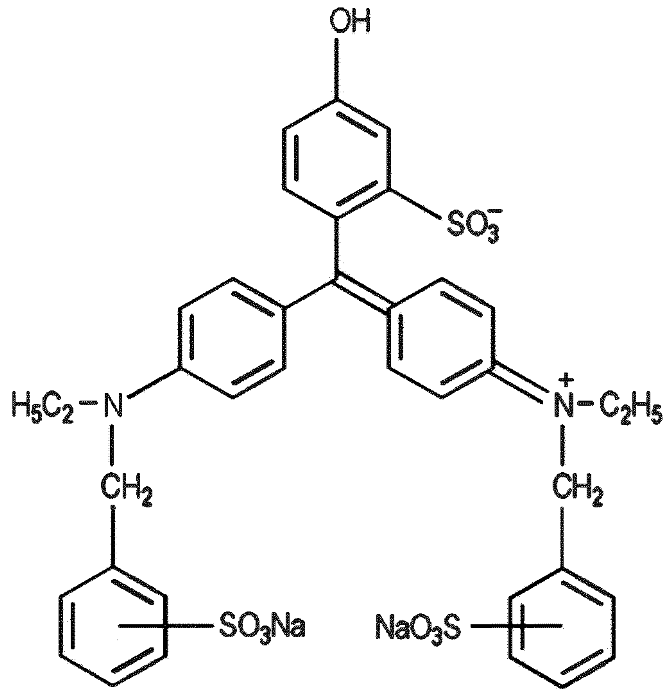

CI14053 (Food Green 3)

[Chem 39]

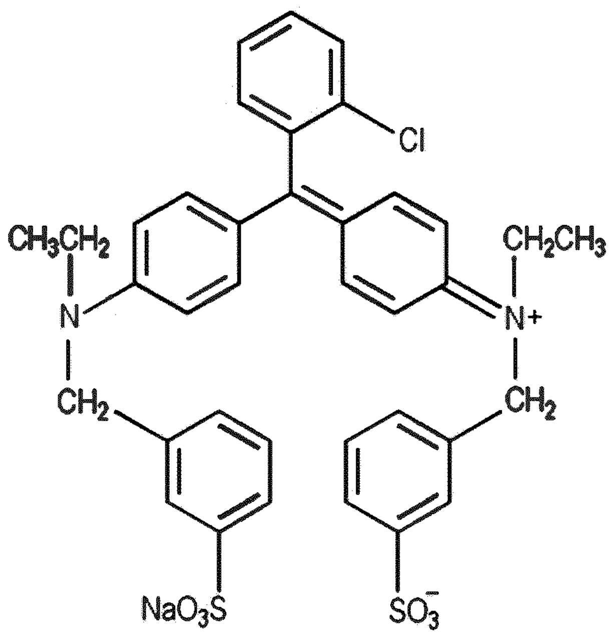

CI42100 (Acid Green 9)

[Chem 40]

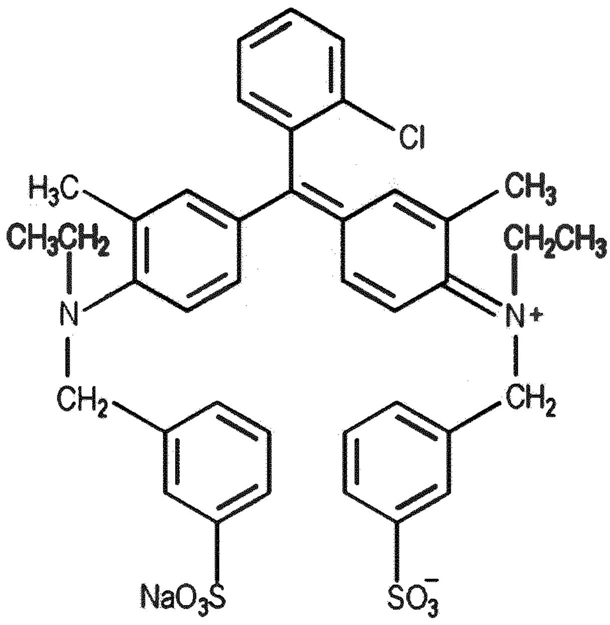

CI42170 (Acid Green 22)

[Chem 41]

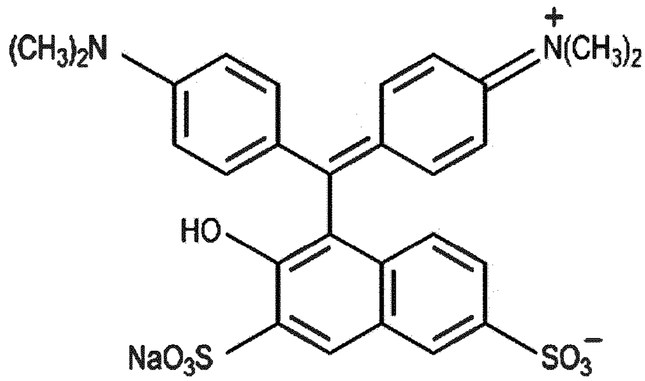

CI44090 (Acid Green 50)

[Chem 42]

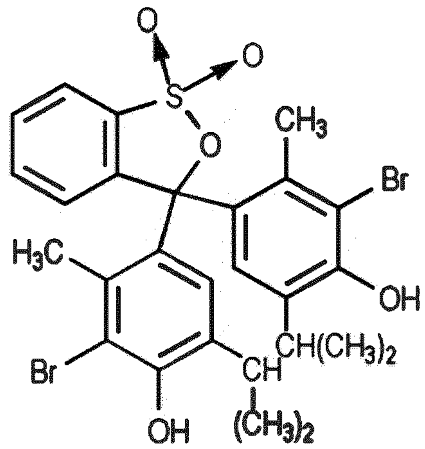

Bromothymol Blue

[Chem 43]

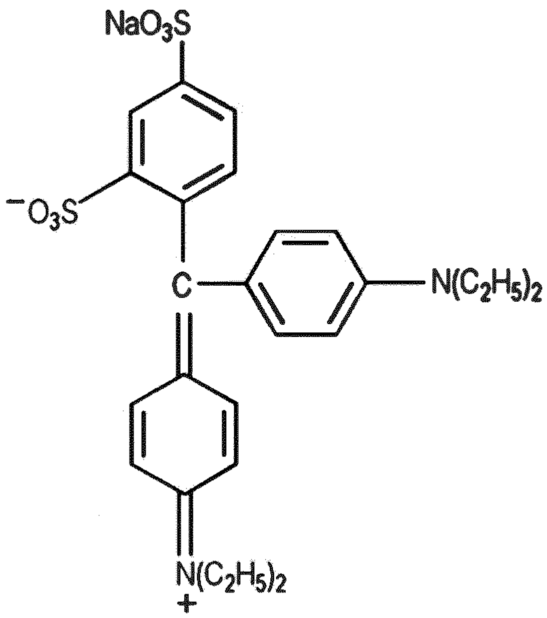

CI42045 (Acid Blue 1)

[Chem 44]

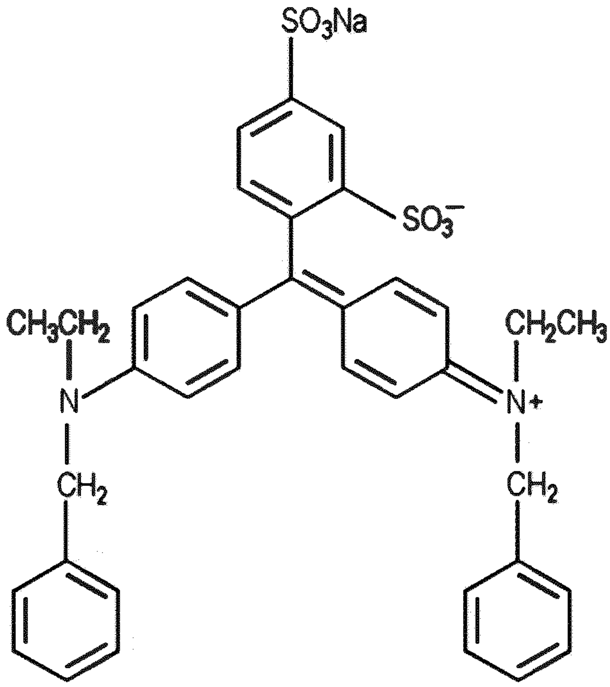

CI42080 (Acid Blue 7)

[Chem 45]

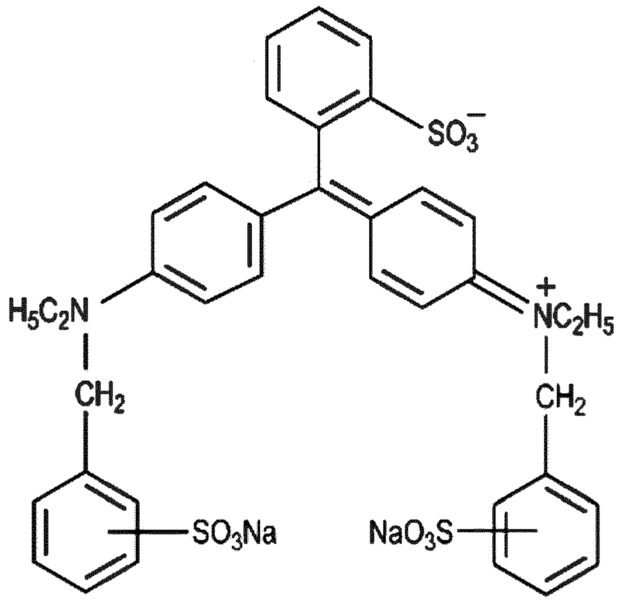

CI42092 (Acid Blue 9)

[Chem 46]

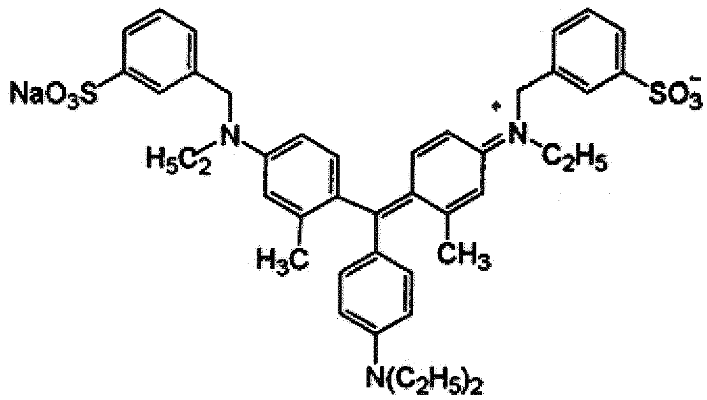

CI42735 (Acid Blue 104)

CI42735 (Acid Blue 104)

The dyes with a xanthene nucleus are preferably those having the following formulae:

[Chem 47]

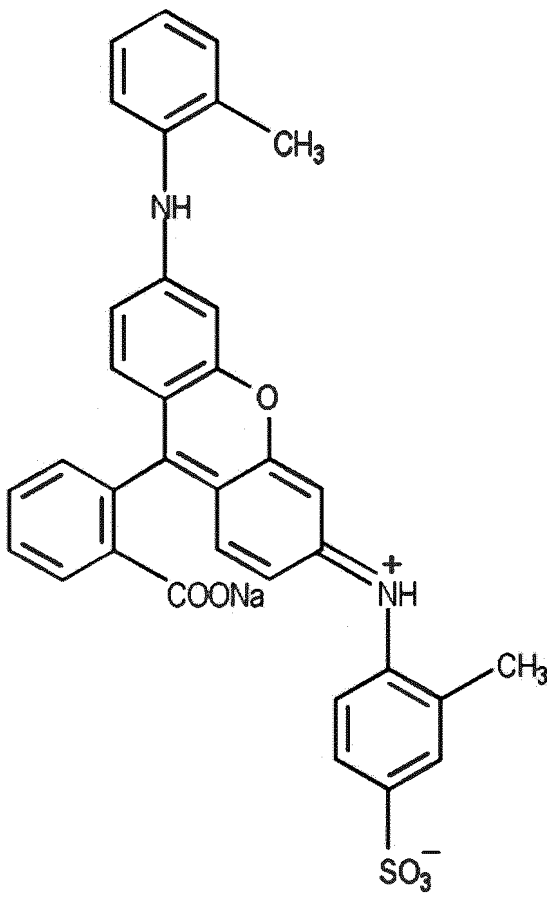

CI45190 (Acid Violet 9)

[Chem 48]

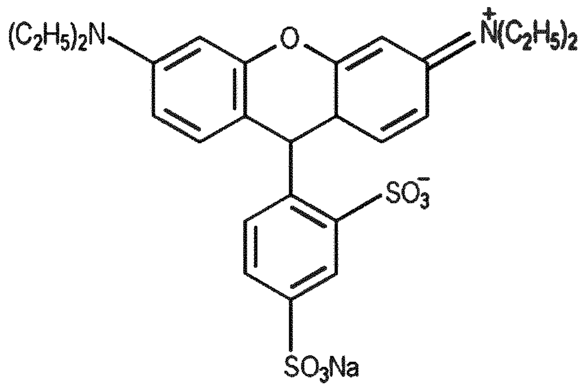

CI45100 (Acid Red 52)

[Chem 49]

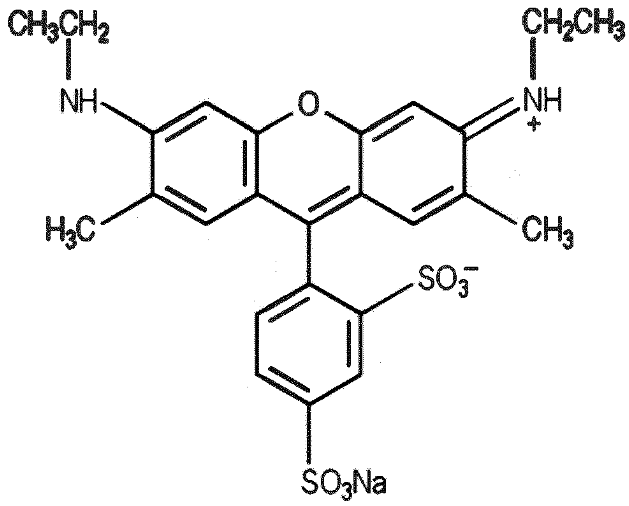

CI45220 (Acid Red 50)

[Chem 50]

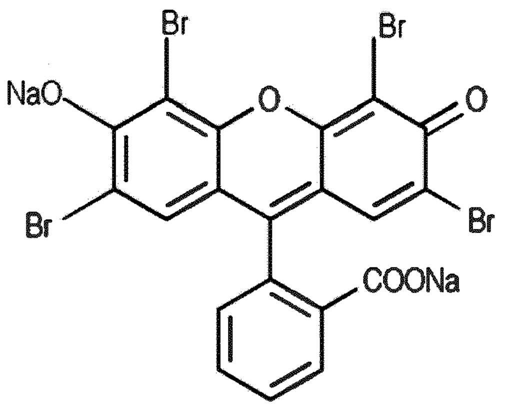

CI45380 (Acid Red 87)

[Chem 51]

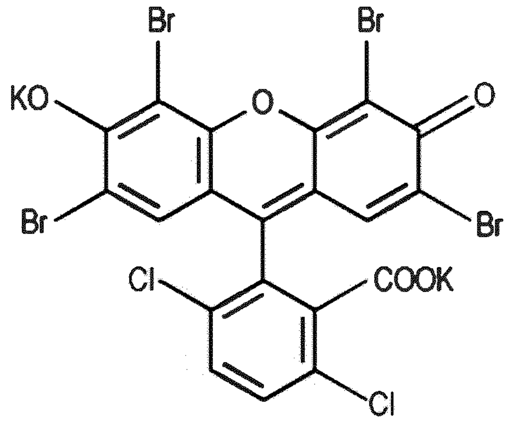

CI45405 (Acid Red 98)

[Chem 52]

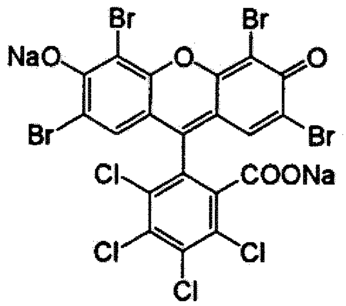

CI45410 (Acid Red 92)

[Chem 53]

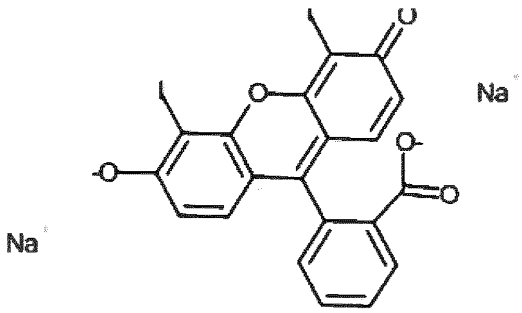

CI45425 (Acid Red 95)

[Chem 54]

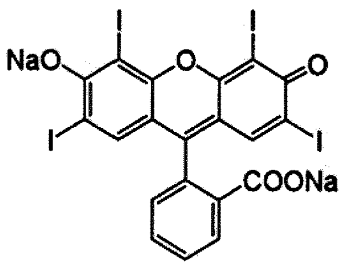

CI45430 (Acid Red 51)

[Chem 55]

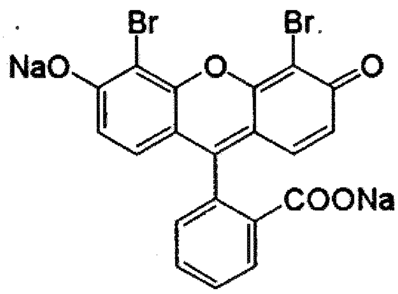

CI45370 (Acid Orange 11)

[Chem 56]

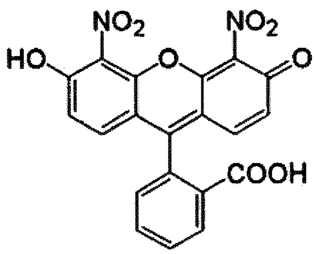

CI45396 (Solvent Orange 16)

[Chem 57]

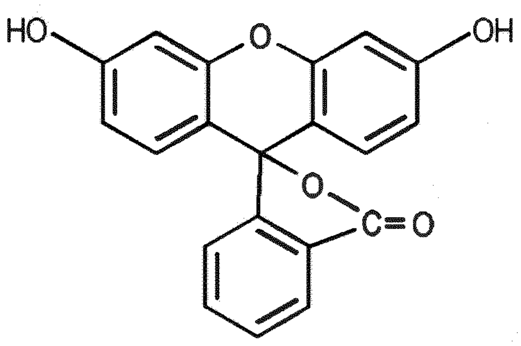

CI45350 (Acid Yellow 73)

[Chem 58]

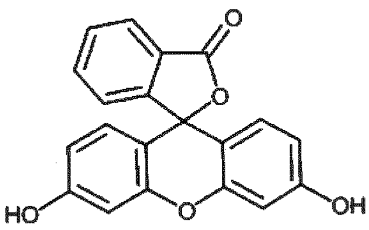

CI45340 (Acid Yellow 73 Fluorescein)

Finally, the dye comprising at least one $-NO_2$ group is preferably the one having the following formula:

[Chem 59]

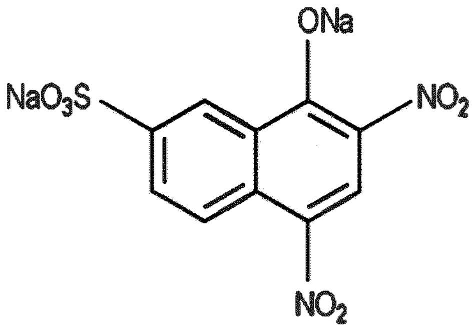

CI10316 (Acid Yellow 1)

Preferably, the dye is chosen from dyes having an anthraquinone, azo, triarylmethane or xanthene nucleus.

Preferably, the dye is chosen from:

[Chem 60]

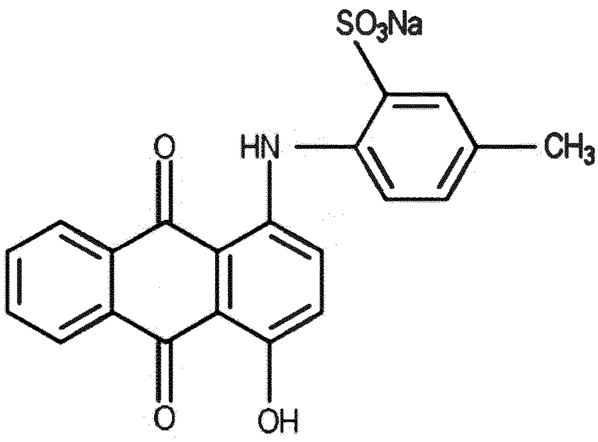

CI60730 (Acid Violet 43)

[Chem 61]

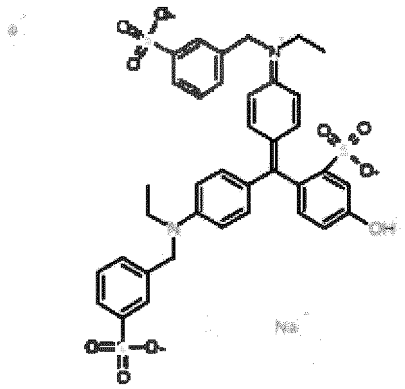

CI42053 (Food Green 3)

[Chem 62]

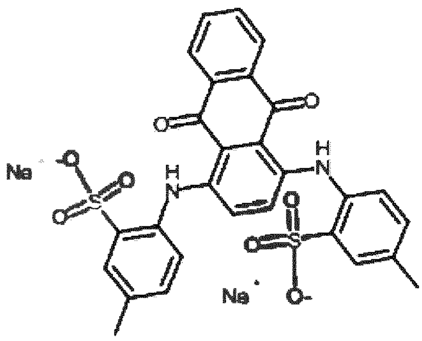

CI61570 (Acid Green 25)

[Chem 63]

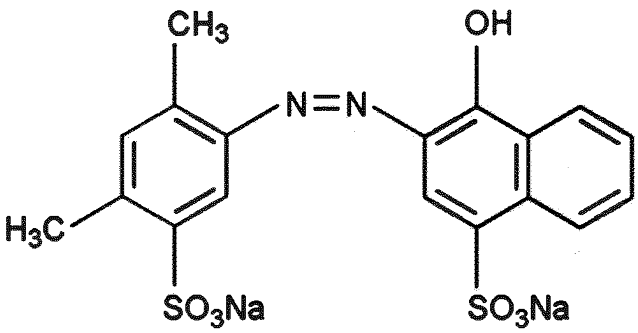

CI14700 (FD&C Red 4)

[Chem 64]

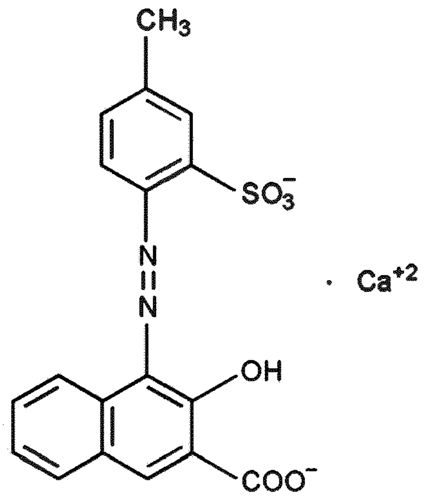

CI15850 (D&C Red 7)

[Chem 65]

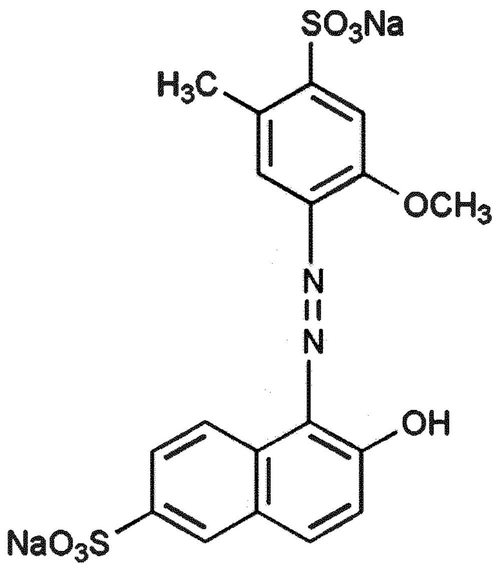

CI16305 (FD&C Red 40)

[Chem 66]

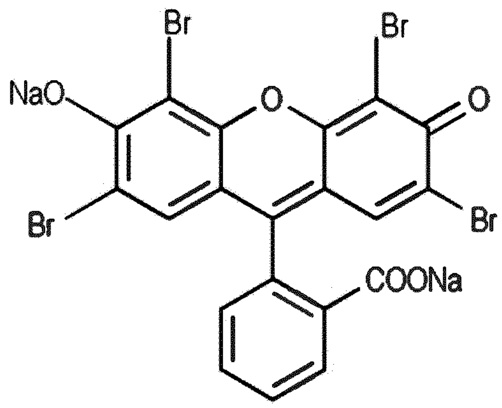

CI45380 (Acid Red 87)

[Chem 67]

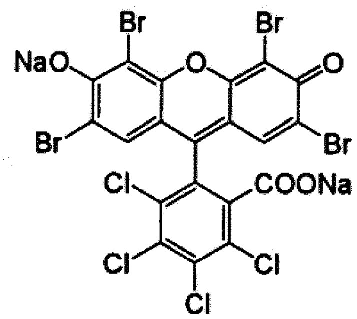

CI45410 (Acid Red 92)

[Chem 68]

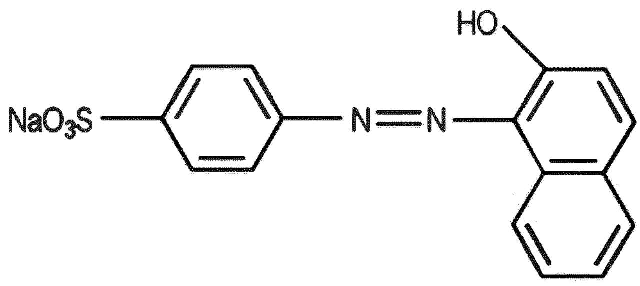

CI15510 (Acid Orange 7)

[Chem 69]

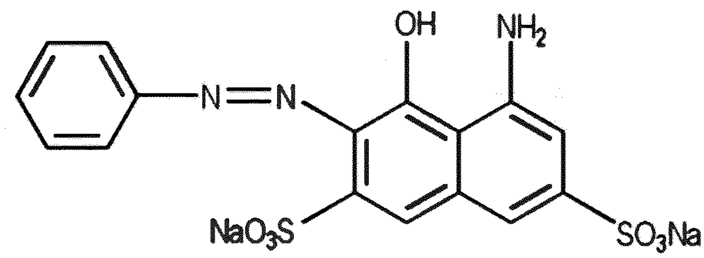

CI17200 (Acid Red 33)

[Chem 70]

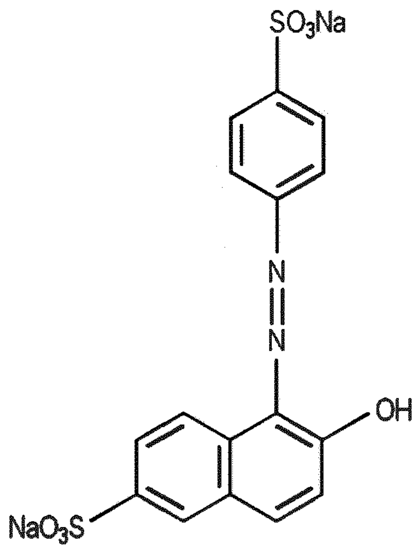

CI15985 (Food Yellow 3)

[Chem 71]

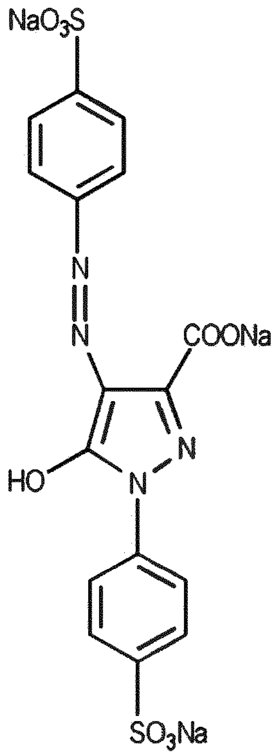

CI19140 (Acid Yellow 23)

[Chem 72]

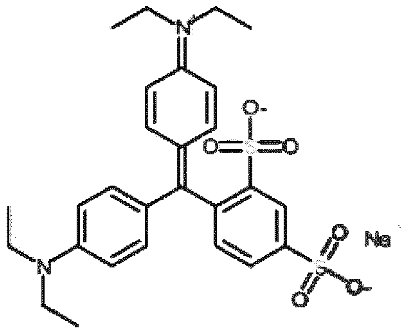

CI42045 (Acid Blue 1)

[Chem 73]

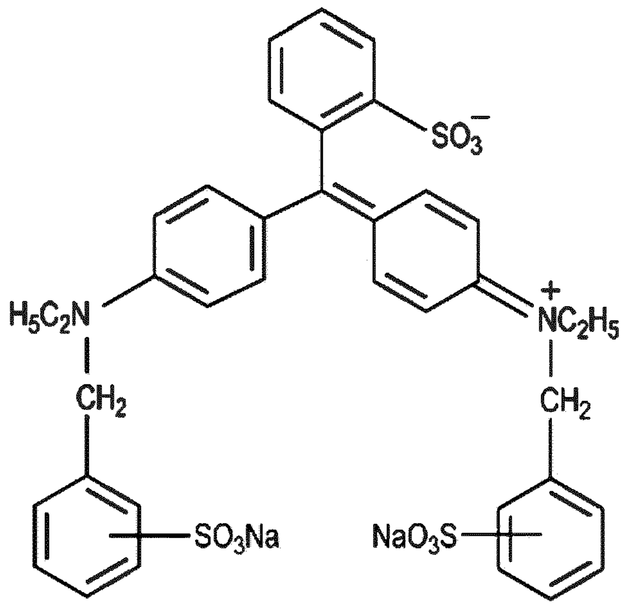

CI42092(Acid Blue 9)

and mixtures thereof.

Preferably, the dye is chosen from dyes having an anthraquinone, azo or triarylmethane nucleus.

More preferentially, such dyes are described above.

Preferably, the dye is chosen from:

[Chem 74]

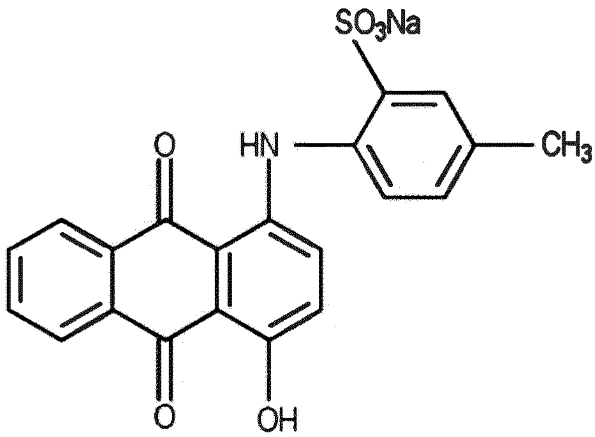

CI60730 (Acid Violet 43)

[Chem 75]

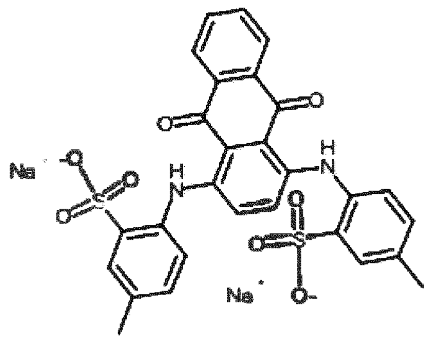

CI61570 (Acid Green 25)

[Chem 76]

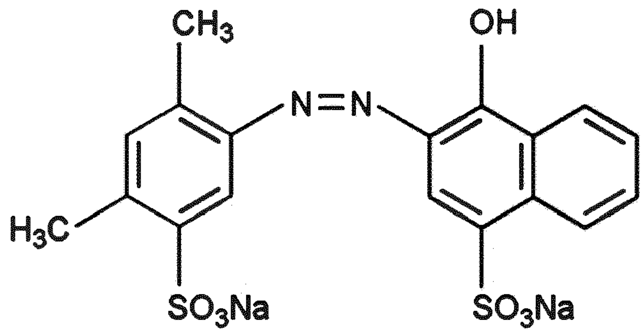

CI14700 (FD&C Red 4)

[Chem 77]

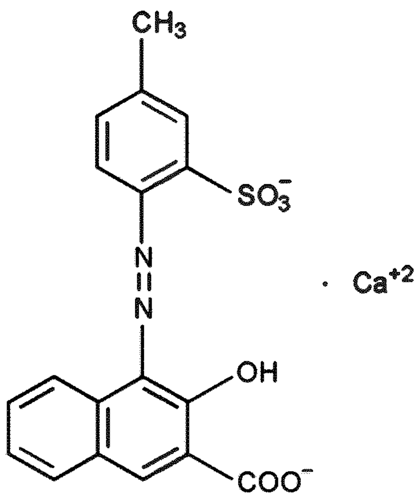

CI15850 (D&C Red 7)

[Chem 78]

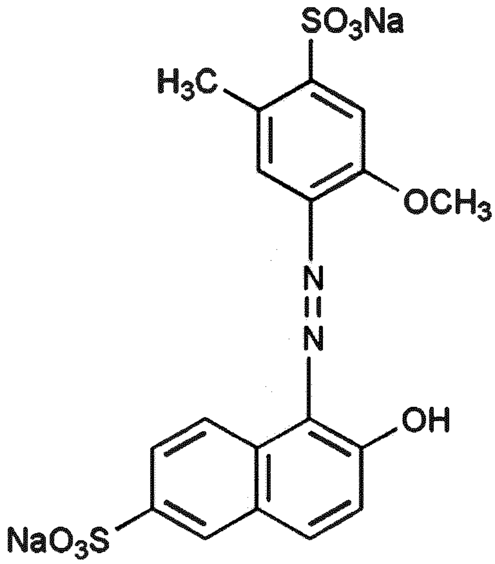

CI16305 (FD&C Red 40)

[Chem 79]

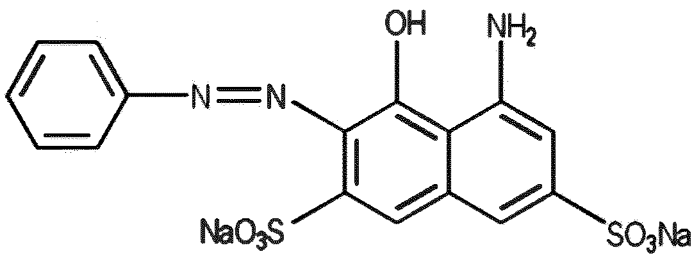

CI17200 (Acid Red 33)

[Chem 80]

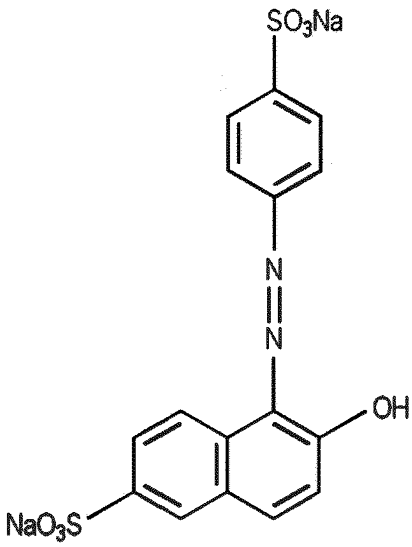

CI15985 (Food Yellow 3)

[Chem 81]

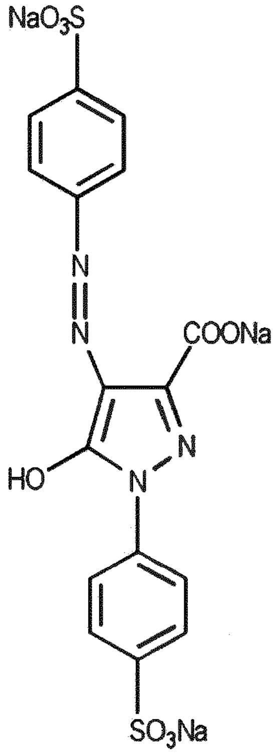

CI19140 (Acid Yellow 23)

[Chem 82]

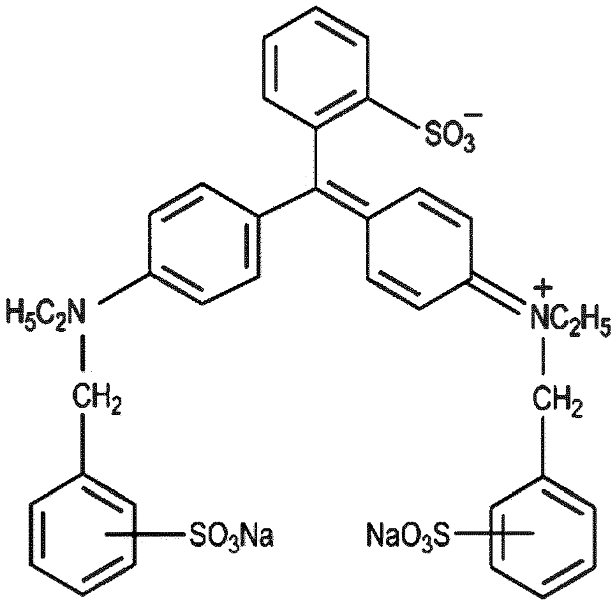

CI42092(Acid Blue 9)

and mixtures thereof.

According to a particular form of the invention, use will preferentially be made of one or more dyes chosen from Acid FD & C Red 40 (CI 16035), Acid Red 33 (CI 17200), Acid Blue 1 (CI 42045), Acid Yellow 23 (CI 19140), and mixtures

thereof.

Besides the compounds described above, composition (A) according to the invention may also comprise the various ingredients as described below.

## Solids content

Composition (A) according to the invention advantageously has a solids content of less than or equal to 30.0% and more preferentially less than or equal to 25.0% relative to the total weight of composition (A).

For the purposes of the present invention, the solids content denotes the content of nonvolatile matter.

The amount of solids content (abbreviated as SC) of a composition according to the invention is measured using a Halogen Moisture Analyzer HR 73 commercial halogen desiccator from Mettler Toledo. The measurement is performed on the basis of the weight loss of a sample dried by halogen heating, and thus represents the percentage of residual matter once the water and the volatile matter have evaporated off.

This technique is fully described in the machine documentation supplied by Mettler Toledo.

The measuring protocol is as follows:
Approximately 2 g of the composition, referred to hereinbelow as the sample, are spread out on a metal crucible, which is placed in the halogen desiccator mentioned above. The sample is then subjected to a temperature of 105° C until a constant weight is obtained. The wet mass of the sample, corresponding to its initial mass, and the dry mass of the sample, corresponding to its mass after halogen heating, are measured using a precision balance.

The experimental error associated with the measurement is of the order of plus or minus 2%.

The solids content is calculated in the following manner:

[Math 1]

$$Solids\ content\ (expressed\ as\ weight\ \%) = 100\ \times \frac{Dry\ mass}{Wet\ mass}$$

## Fixing composition (B)

The fixing composition (B) according to the invention comprises a continuous oily phase containing:

i) at least one hydrophobic film-forming polymer other than silicone acrylate copolymers; and
ii) at least one volatile hydrocarbon-based oil.

### Oily phase

The term "oil" means a water-immiscible nonaqueous compound that is liquid at room temperature (25° C) and at atmospheric pressure (760 mmHg).

The term "hydrocarbon-based oil" means an oil predominantly including carbon and hydrogen atoms and not comprising any silicon atoms.

The term "volatile oil" means any oil with a vapor pressure of greater than or equal to 0.13 Pa measured at 25° C.

### Hydrocarbon-based volatile oil

The hydrocarbon-based oil of composition (B) is preferably chosen from hydrocarbon-based oils containing from 8 to 16 carbon atoms and better still from 12 to 16 carbon atoms.

Advantageously, the hydrocarbon-based oil of composition (B) is apolar (thus formed solely from carbon and hydrogen atoms).

The volatile hydrocarbon-based oil may be chosen from:

- branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isohexadecane and, for example, the oils sold under the Isopar or Permethyl trade names;
- linear $C_{11}$-$C_{14}$ alkanes, for instance n-dodecane ($C_{12}$) and n-tetradecane ($C_{14}$) sold by Sasol under the references, respectively, Parafol 12-97 and Parafol 14-97, and also mixtures thereof, the undecane-tridecane mixture, mixtures of n-undecane ($C_{11}$) and of n-tridecane ($C_{13}$) obtained in examples 1 and 2 of patent application WO 2008/155059 from the company Cognis, and mixtures thereof.

Preferentially, the volatile hydrocarbon-based oil is isododecane.
More particularly, the content of volatile hydrocarbon-based oil(s) is greater than 20% by weight, preferably an amount ranging from 30% to 70% by weight and better still an amount ranging from 35% to 60% by weight, relative to the weight of composition (B).

## Viscosity

A composition (B) according to the invention is advantageously creamy at an ambient temperature of 20° C.
It is characterized by a viscosity of less than 60 Pa.s, or even preferably less than 45 Pa.s, measured at an ambient temperature of 20° C using a Rheomat RM100® viscometer.
Preferably, the viscosity of the compositions according to the invention ranges from 2.0 to 60.0 Pa.s, or even preferably from 2.5 to 45.0 Pa.s, measured at the ambient temperature of 20° C using a Rheomat RM100® viscometer.
Composition (B) may be manufactured via the known processes generally used in the cosmetics field.
Such compositions are notably prepared according to the general knowledge of a person skilled in the art.

## Hydrophobic film-forming polymers

Among the hydrophobic film-forming polymers, the ones that will preferably be chosen are those chosen from:

(i) silicone resins
(ii) silicone polyamides
(iii) pseudo-blocks
(iv) oily dispersions; the respective definitions of which are indicated below.

### Silicone resin

Composition (B) according to the invention comprises at least one silicone resin.
More generally, the term "resin" means a compound whose structure is three-dimensional. "Silicone resins" are also known as "siloxane resins". Thus, for the purposes of the present invention, a polydimethylsiloxane is not a silicone resin.
The nomenclature of silicone resins (also known as siloxane resins) is known under the name "MDTQ", the resin being described as a function of the various siloxane monomer units it comprises, each of the letters "MDTQ" characterizing a type of unit.
The letter "M" represents the Monofunctional unit of formula $R_1R_2R_3SiO_{1/2}$, the silicon atom being connected to only one oxygen atom in the polymer comprising this unit.
The letter "D" means a Difunctional unit $R_1R_2SiO_{2/2}$ in which the silicon atom is connected to two oxygen atoms.
The letter "T" represents a Trifunctional unit $R_1SiO_{3/2}$.
Such resins are described, for example, in the Encyclopedia of Polymer Science and Engineering, vol. 15, John Wiley & Sons, New York, (1989), pp. 265-270, and US 2 676 182, US 3 627 851, US 3 772 247, US 5 248 739 or US 5 082 706, US 5 319 040, US 5 302 685 and US 4 935 484.
In the units M, D and T defined previously, R, namely $R_1$, $R_2$ and $R_3$, represents a hydrocarbon-based radical (notably alkyl) containing from 1 to 10 carbon atoms, a phenyl group, a phenylalkyl group or a hydroxyl group.
Finally, the letter Q means a Quadrifunctional unit $SiO_{4/2}$ in which the silicon atom is bonded to four hydrogen atoms, which are themselves bonded to the rest of the polymer.
Various silicone resins with different properties can be obtained from these different units, the properties of these polymers varying as a function of the type of monomer (or units), of the nature and number of the R radical, of the length of the polymer chain, of the degree of branching and of the size of the pendent chains.
As silicone resins that may be used in the compositions according to the invention, use may be made, for example, of silicone resins of MQ type, of T type or of MQT type.

### MQ resins:

As examples of silicone resins of MQ type, mention may be made of the alkyl siloxysilicates of formula $[(R_1)_3SiO_{1/2}]_x(SiO_{4/2})_y$ (MQ units) in which x and y are integers ranging from 50 to 80, and such that the group $R_1$ represents a radical as defined previously, and is preferably an alkyl group containing from 1 to 8 carbon atoms or a hydroxyl group, preferably a methyl group.
As examples of MQ silicone resins of trimethyl siloxysilicate type, mention may be made of those sold under the reference SR1000® by the company General Electric, under the reference TMS 803® by the company Wacker, or under

the name KF-7312J® by the company Shin-Etsu or DC749® or DC593® by the company Dow Corning.

T resins:

Examples of silicone resins of type T that may be mentioned include the polysilsesquioxanes of formula $(RSiO_{3/2})_x$ (T units) in which x is greater than 100 and such that the group R is an alkyl group containing from 1 to 10 carbon atoms, said polysilsesquioxanes also possibly comprising Si-OH end groups.
Polymethylsilsesquioxane resins that may preferably be used are those in which R represents a methyl group, for instance those sold:

- by the company Wacker under the reference Resin MK, such as Belsil PMS MK®: polymer comprising $CH_3SiO_{3/2}$ repeating units (T units), which may also comprise up to 1% by weight of $(CH_3)_2SiO_{2/2}$ units (D units) and having an average molecular weight of about 10 000 g/mol, or
- by the company Shin-Etsu under the references KR-220L®, which are composed of units T of formula $CH_3SiO_{3/2}$ and contain Si-OH (silanol) end groups, under the reference KR-242A®, which comprise 98% of units T and 2% of dimethyl units D and contain Si-OH end groups, or else under the reference KR-251®, comprising 88% of units T and 12% of dimethyl units D and contain Si-OH end groups.

MQT resins:

Resins comprising MQT units that are notably known are those mentioned in US 5 110 890.
A preferred form of resins of MQT type are MQT-propyl (also known as MQTPr) resins. Such resins that may be used in the compositions according to the invention are notably the resins described and prepared in patent application WO 2005/075 542, the content of which is incorporated herein by reference.
The MQ-T-propyl resin preferably comprises the following units:

(i) $(R1_3SiO_{1/2})_a$
(ii) $(R2_2SiO_{2/2})_b$
(iii) $(R3SiO_{3/2})_c$ and
(iv) $(SiO_{4/2})_d$

with

R1, R2 and R3 independently representing a hydrocarbon-based radical (notably alkyl) containing from 1 to 10 carbon atoms, a phenyl group, a phenylalkyl group or a hydroxyl group and preferably an alkyl radical containing from 1 to 8 carbon atoms or a phenyl group,
a, b, c and d being mole fractions,
a being between 0.05 and 0.5,
b being between 0 and 0.3,
c being greater than zero,
d being between 0.05 and 0.6,
a + b + c + d = 1,
on condition that more than 40 mol% of the groups R3 of the siloxane resin are propyl groups.

Preferably, the siloxane resin comprises the units:

(i) $(R1_3SiO_{1/2})_a$
(ii) $(R3SiO_{3/2})_c$ and
(iv) $(SiO_{4/2})_d$
with R1 and R3 independently representing an alkyl group containing from 1 to 8 carbon atoms, R1 preferably being a methyl group and R3 preferably being a propyl group,
a being between 0.05 and 0.5 and preferably between 0.15 and 0.4,
c being greater than zero, preferably between 0.15 and 0.4,
d being between 0.05 and 0.6, preferably between 0.2 and 0.6 or alternatively between 0.2 and 0.55,
a + b + c + d = 1, and a, b, c and d being mole fractions,
provided that more than 40 mol% of the R3 groups of the siloxane resin are propyl groups.

The siloxane resins that may be used according to the invention may be obtained via a process comprising the

reaction of:

A) an MQ resin comprising at least 80 mol% of units $(R1_3SiO_{1/2})_a$ and $(SiO_{4/2})_d$,

R1 representing an alkyl group containing from 1 to 8 carbon atoms, an aryl group, a carbinol group or an amino group,
a and d being greater than zero,
the ratio a/d being between 0.5 and 1.5; and

B) a T-propyl resin comprising at least 80 mol% of units $(R3SiO_{3/2})_c$,

R3 representing an alkyl group containing from 1 to 8 carbon atoms, an aryl group, a carbinol group or an amino group,
c being greater than zero,
provided that at least 40 mol% of the R3 groups are propyl groups,
in which the mass ratio A/B is between 95/5 and 15/85 and preferably the mass ratio A/B is 30/70.

Advantageously, the mass ratio A/B is between 95/5 and 15/85. Preferably, the ratio A/B is less than or equal to 70/30. These preferred ratios have proven to afford comfortable deposits.

Preferably, the composition according to the invention comprises, as silicone resin, at least one resin of MQ type, more particularly of trimethyl siloxysilicate type, such as those sold under the reference SR1000® by the company General Electric, under the reference TMS 803® by the company Wacker, or under the name KF-7312J® by the company Shin-Etsu or DC749® or DC593® by the company Dow Corning.

According to a particular embodiment of the invention, the silicone resin is present in composition (B) in a resin solids content ranging from 4% to 35% by weight relative to the total weight of the composition, preferably ranging from 6% to 30% by weight and more preferentially from 8% to 25% by weight relative to the total weight of composition (B).

Silicone polyamide

The second composition (B) of the makeup kit according to the invention comprises at least one silicone polyamide.

The silicone polyamides are preferably solid at room temperature (25° C) and atmospheric pressure (760 mmHg).

For the purposes of the invention, the term "polymer" means a compound containing at least two repeating units, preferably at least three repeating units and better still ten repeating units.

The silicone polyamides of the composition of the invention may be polymers of the polyorganosiloxane type, for instance those described in US-A-5 874 069, US-A-5 919 441, US-A-6 051 216 and US-A-5 981 680. According to the invention, the silicone polymers may belong to the following two families:

(1) polyorganosiloxanes including at least two amide groups, these two groups being located in the polymer chain, and/or
(2) polyorganosiloxanes including at least two amide groups, these two groups being located on grafts or branches.

According to a first variant, the silicone polymers are polyorganosiloxanes as defined above in which the units that are capable of establishing hydrogen interactions are located in the polymer chain.

The silicone polymers may be more particularly polymers comprising at least one unit corresponding to the general formula I(I):

[Chem 83]

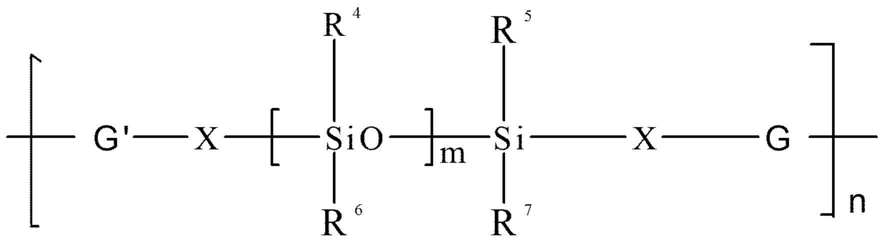

(I)

in which: G' represents C(O) when G represents -C(O)-NH-Y-NH-, and G' represents -NH- when G represents -NH-C(O)-Y-C(O)-,

$R^4$, $R^5$, $R^6$ and $R^7$, which may be identical or different, represent a group chosen from:

saturated or unsaturated, $C_1$ to $C_{40}$ linear, branched or cyclic hydrocarbon-based groups, which may contain in their chain one or more oxygen, sulfur and/or nitrogen atoms, and which may be partially or totally substituted with fluorine atoms,
$C_6$ to $C_{10}$ aryl groups, optionally substituted with one or more $C_1$ to $C_4$ alkyl groups, polyorganosiloxane chains possibly containing one or more oxygen, sulfur and/or nitrogen atoms,
the groups X, which may be identical or different, represent a linear or branched $C_1$ to $C_{30}$ alkylenediyl group, possibly containing in its chain one or more oxygen and/or nitrogen atoms,
Y is a saturated or unsaturated $C_1$ to $C_{50}$ linear or branched alkylene, arylene, cycloalkylene, alkylarylene or arylalkylene divalent group, which may include one or more oxygen, sulfur and/or nitrogen atoms, and/or may bear as substituent one of the following atoms or groups of atoms: fluorine, hydroxyl, $C_3$ to $C_8$ cycloalkyl, $C_1$ to $C_{40}$ alkyl, $C_5$ to $C_{10}$ aryl, phenyl optionally substituted with one to three $C_1$ to $C_3$ alkyl, $C_1$ to $C_5$ hydroxyalkyl and $C_1$ to $C_6$ aminoalkyl groups, or Y represents a group corresponding to formula (1):

[Chem 84]

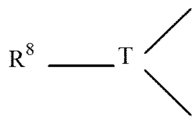

(1)

in which:

T represents a linear or branched, saturated or unsaturated, $C_3$ to $C_{24}$ trivalent or
tetravalent hydrocarbon-based group optionally substituted with a polyorganosiloxane chain, and possibly containing one or more atoms chosen from O, N and S, or T represents a trivalent atom chosen from N, P and Al, and $R^8$ represents a linear or branched $C_1$-$C_{50}$ alkyl group or a polyorganosiloxane chain, possibly including one or more ester, amide, urethane, thiocarbamate, urea, thiourea and/or sulfonamide groups, which may possibly be linked to another chain of the polymer,
n is an integer ranging from 2 to 500 and preferably from 2 to 200, and m is an integer ranging from 1 to 1000, preferably from 1 to 700 and better still from 6 to 200. Preferably, m is an integer ranging from 50 to 150.

According to one embodiment of the invention, 80% of the groups $R^4$, $R^5$, $R^6$ and $R^7$ of the polymer are preferably chosen from methyl, ethyl, phenyl and 3,3,3-trifluoropropyl groups. According to another embodiment, 80% of the groups $R^4$, $R^5$, $R^6$ and $R^7$ of the polymer are methyl groups.

According to the invention, Y may represent various divalent groups, furthermore optionally including one or two free valencies to establish bonds with other units of the polymer or copolymer. Preferably, Y represents a group chosen from:

linear $C_1$ to $C_{20}$ and preferably $C_1$ to $C_{10}$ alkylene groups,
$C_{30}$ to $C_{56}$ branched alkylene groups possibly including rings and unconjugated unsaturations,
$C_5$-$C_6$ cycloalkylene groups,
phenylene groups optionally substituted with one or more $C_1$ to $C_{40}$ alkyl groups, $C_1$ to $C_{20}$ alkylene groups including from 1 to 5 amide groups,
$C_1$ to $C_{20}$ alkylene groups including one or more substituents chosen from hydroxyl, $C_3$ to $C_8$ cycloalkane, $C_1$ to $C_3$ hydroxyalkyl and $C_1$ to $C_6$ alkylamine groups,

polyorganosiloxane chains of formula (2) or (3):

[Chem 85]

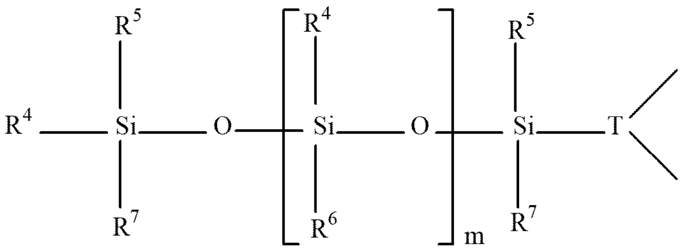

(2)

[Chem 86]

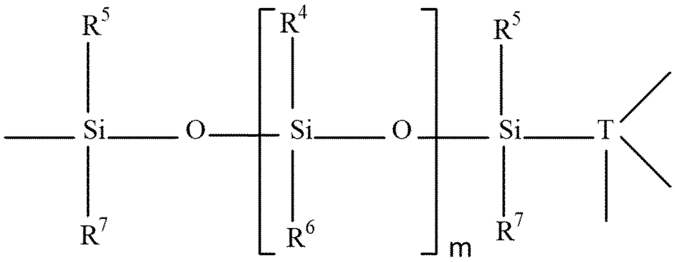

(3)

in which $R^4$, $R^5$, $R^6$, $R^7$, T and m are as defined above.

According to the second variant, the polyorganosiloxanes may be polymers comprising at least one unit corresponding to formula (II):

[Chem 87]

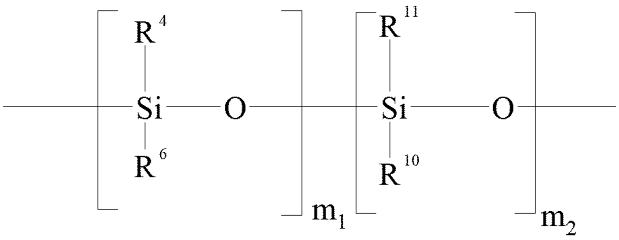

(II)

in which:

$R^4$ and $R^6$, which may be identical or different, are as defined above for formula (I),
$R^{10}$ represents a group as defined above for $R^4$ and $R^6$, or represents a group of formula -X-G-$R^{12}$ in which X and G are as defined above for formula (I) and R12 represents a hydrogen atom or a linear, branched or cyclic, saturated or unsaturated, $C_1$-$C_{50}$ hydrocarbon-based group optionally including in its chain one or more atoms chosen from O, S and N, optionally substituted with one or more fluorine atoms and/or one or more hydroxyl groups, or a phenyl group optionally substituted with one or more $C_1$-$C_4$ alkyl groups,
$R^{11}$ represents a group of formula -X-G-$R^{12}$ in which X, G and $R^{12}$ are as defined above,
$m_1$ is an integer ranging from 1 to 998, and
$m_2$ is an integer ranging from 2 to 500.

According to a particular embodiment of the invention, the silicone polyamide may be a homopolymer, that is to say a polymer including several identical units, in particular units of formula (I) or of formula (II).

According to another particular embodiment of the invention, it is also possible to use a silicone polyamide formed

from a copolymer including several different units of formula (I), that is to say a polymer in which at least one from among $R^4$, $R^5$, $R^6$, $R^7$, X, G, Y, m and n is different in one of the units. The copolymer may also be formed from several units of formula (II), in which at least one from among $R^4$, $R^6$, $R^{10}$, $R^{11}$, m1 and m2 is different in at least one of the units.

It is also possible to use a polymer including at least one unit of formula (I) and at least one unit of formula (II), the units of formula (I) and the units of formula (II) possibly being identical to or different than each other.

According to one variant of the invention, it is also possible to use a polymer furthermore comprising at least one hydrocarbon-based unit including two groups that are capable of establishing hydrogen interactions, chosen from ester, amide, sulfonamide, carbamate, thiocarbamate, urea, urethane, thiourea, oxamido, guanidino and biguanidino groups, and combinations thereof.

These copolymers may be block polymers or grafted polymers.

According to an advantageous embodiment of the invention, the groups that are capable of establishing hydrogen interactions are amide groups of formulae

-C(O)NH- and -HN-C(O)-.

In this case, the film-forming agent may be a polymer comprising at least one unit of formula (III) or (IV):

[Chem 88]

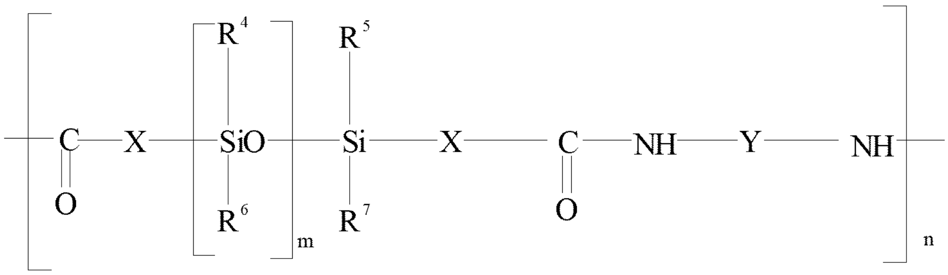

(III)

or

[Chem 89]

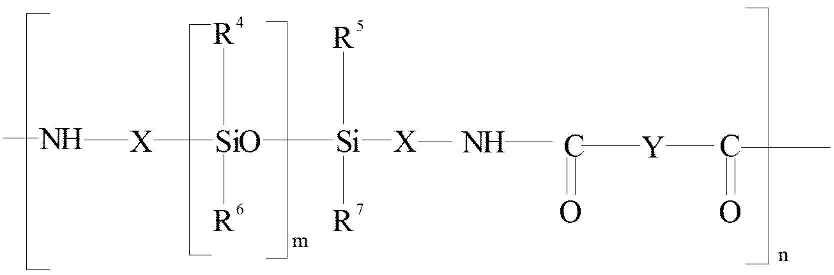

(IV)

in which $R^4$, $R^5$, $R^6$, $R^7$, X, Y, m and n are as defined above.

In these polyamides of formula (III) or (IV), m ranges from 1 to 700, in particular from 15 to 500 and notably from 50 to 200, and n ranges in particular from 1 to 500, preferably from 1 to 100 and better still from 4 to 25,

X is preferably a linear or branched alkylene chain containing from 1 to 30 carbon atoms, in particular 1 to 20 carbon atoms, notably from 5 to 15 carbon atoms and more particularly 10 carbon atoms, and

Y is preferably an alkylene chain that is linear or branched, or which may include rings and/or unsaturations, containing from 1 to 40 carbon atoms, in particular 1 to 20 carbon atoms and better still from 2 to 6 carbon atoms, in particular 6 carbon atoms.

In formulae (III) and (IV), the alkylene group representing X or Y may optionally contain in its alkylene part at least one of the following components: one to five amide, urea, urethane or carbamate groups,

a $C_5$ or $C_6$ cycloalkyl group, and

a phenylene group optionally substituted with 1 to 3 identical or different $C_1$ to $C_3$ alkyl groups.

In formulae (III) and (IV), the alkylene groups may also be substituted with at least one component chosen from

the group consisting of:

- a hydroxyl group,
- a $C_3$ to $C_8$ cycloalkyl group,
- one to three $C_1$ to $C_{40}$ alkyl groups,
- a phenyl group optionally substituted with one to three $C_1$ to $C_3$ alkyl groups,
- a $C_1$ to $C_3$ hydroxyalkyl group, and
- a $C_1$ to $C_6$ aminoalkyl group.

In these formulae (III) and (IV), Y may also represent:

[Chem 90]

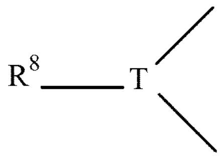

in which $R^8$ represents a polyorganosiloxane chain and T represents a group of formula:

[Chem 91]

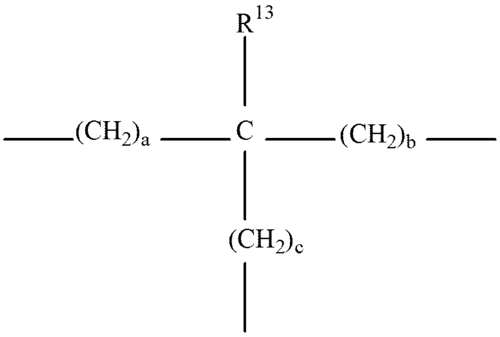

ou

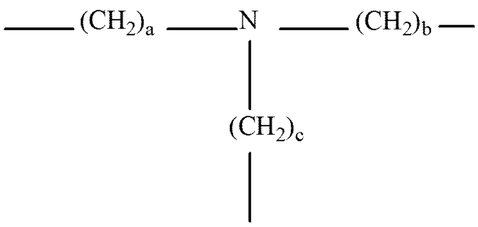

in which a, b and c are, independently, integers ranging from 1 to 10, and $R^{13}$ is a hydrogen atom or a group such as those defined for $R^4$, $R^5$, $R^6$ and $R^7$.

In formulae (III) and (IV), $R^4$, $R^5$, $R^6$ and $R^7$ preferably represent, independently, a linear or branched $C_1$ to $C_{40}$ alkyl group, preferably a $CH_3$, $C_2H_5$, n-$C_3H_7$ or isopropyl group, a polyorganosiloxane chain or a phenyl group optionally substituted with one to three methyl or ethyl groups.

According to a preferred embodiment, the silicone polyamide comprises at least one unit of formula (III) or (IV).

As has been seen previously, the polymer may comprise identical or different units of formula (III) or (IV).

Thus, the polymer may be a polyamide containing several units of formula (III) or (IV) of different lengths, i.e. a polyamide corresponding to formula (V):

[Chem 92]

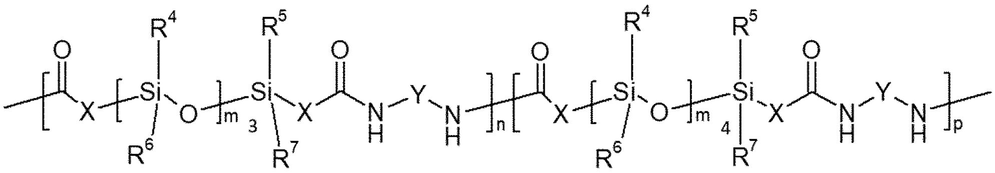

(V)

in which X, Y, n and $R^4$ to $R^7$ have the meanings given above, $m_3$ and $m_4$, which are different, are chosen in the range from 1 to 1000, and p is an integer ranging from 2 to 300.

In this formula, the units may be structured to form either a block copolymer, or a statistical copolymer or an

alternating copolymer. In this copolymer, the units may be not only of different lengths, but also of different chemical structures, for example containing different groups Y. In this case, the polymer may correspond to formula (VI):

[Chem 93]

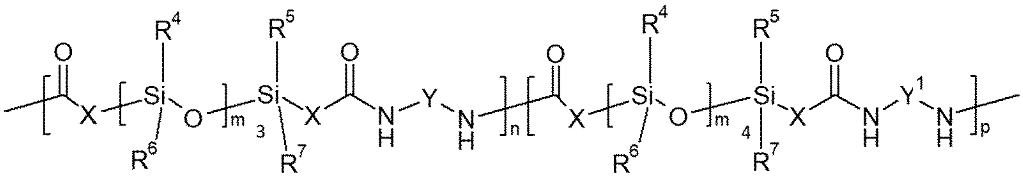

(VI)

in which $R^4$ to $R^7$, X, Y, $m_3$, $m_4$, n and p have the meanings given above and Y1 is different than Y but is chosen from the groups defined for Y.

As previously, the various units may be structured to form either a block copolymer, or a statistical copolymer or an alternating copolymer.

In this first embodiment of the invention, the film-forming agent may also be formed from a grafted copolymer. Thus, the polyamide containing silicone units may be grafted and optionally crosslinked with silicone chains containing amide groups. Such polymers may be synthesized with trifunctional amines.

In this case, the polymer may comprise at least one unit of formula (VII):

[Chem 94]

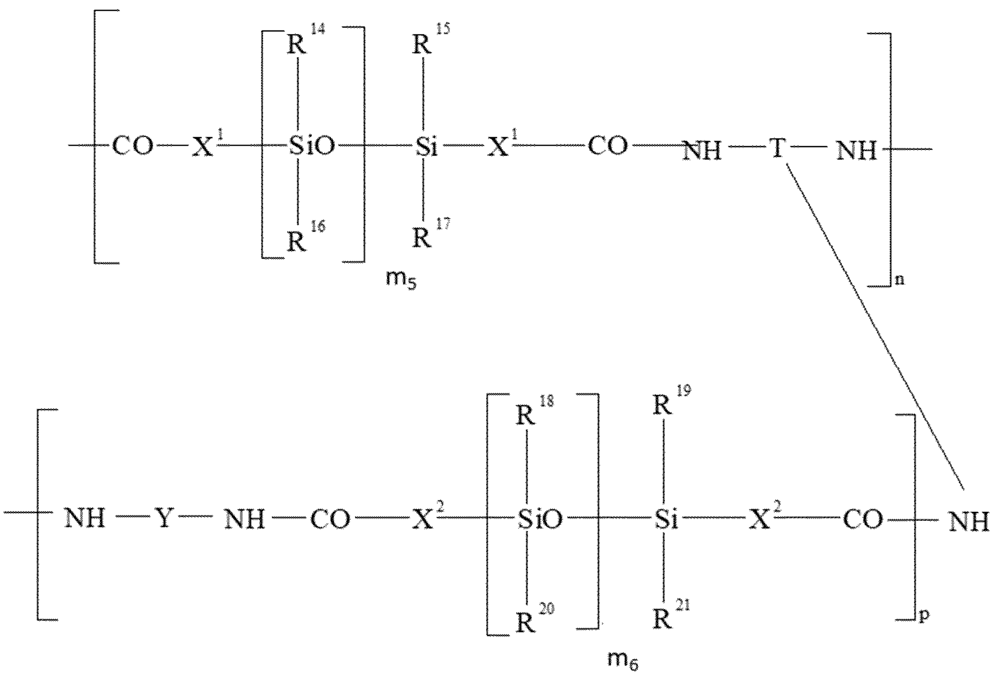

(VII)

in which $X^1$ and $X^2$, which are identical or different, have the meaning given for X in formula (I), n is as defined in formula (I), Y and T are as defined in formula (I), $R^{14}$ to $R^{21}$ are groups chosen from the same group as $R^4$ to $R^7$, $m_5$ and $m_6$ are numbers in the range from 1 to 1000, and p is an integer ranging from 2 to 500.

In formula (VII), it is preferred that:

p is in the range from 1 to 25 and better still from 1 to 7,

$R^{14}$ to $R^{21}$ are methyl groups,

T corresponds to one of the following formulae:

[Chem 95]

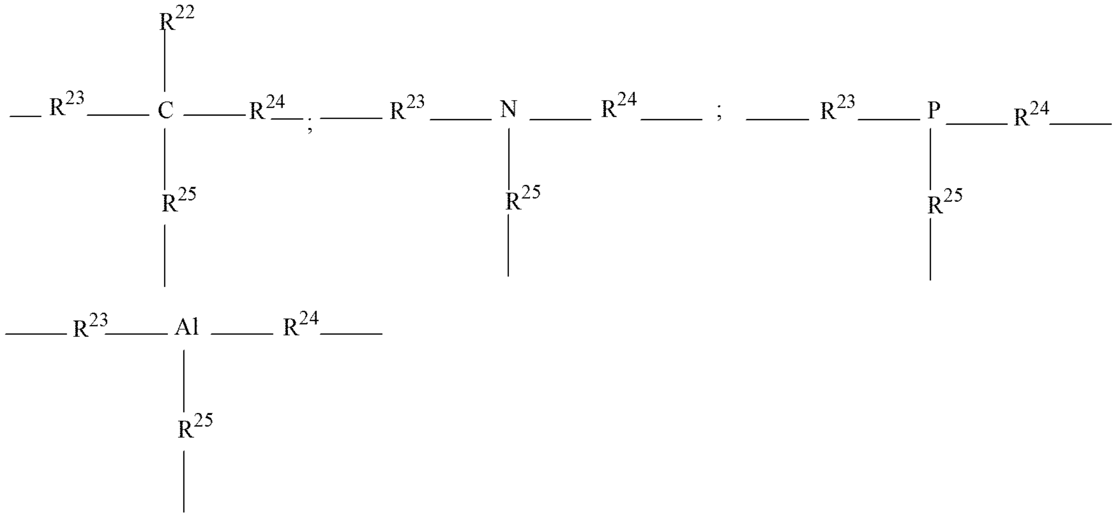

in which $R^{22}$ is a hydrogen atom or a group chosen from the groups defined for $R^4$ to $R^7$, and $R^{23}$, $R^{24}$ and $R^{25}$ are, independently, linear or branched alkylene groups, and more preferably corresponds to the formula:

[Chem 96]

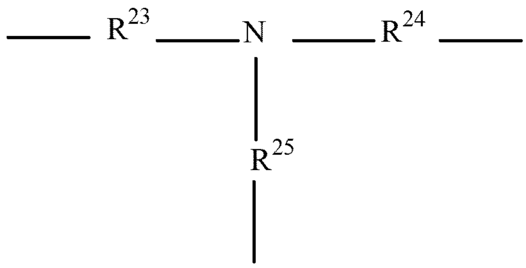

in particular with $R^{23}$, $R^{24}$ and $R^{25}$ representing $-CH_2-CH_2-$,
$m_1$ and $m_2$ range from 15 to 500 and better still from 15 to 45,
$X^1$ and $X^2$ represent $-(CH_2)_{10}-$, and
Y represents $-CH_2-$.

These polyamides containing a grafted silicone unit of formula (VII) may be copolymerized with polyamide-silicones of formula (II) to form block copolymers, alternating copolymers or statistical copolymers. The weight percentage of grafted silicone units (VII) in the copolymer may range from 0.5% to 30% by weight.

According to the invention, as has been seen previously, the siloxane units may be in the main chain or backbone of the polymer, but they may also be present in grafted or pendent chains. In the main chain, the siloxane units may be in the form of segments as described above. In the pendent or grafted chains, the siloxane units may appear individually or in segments.

According to one embodiment variant of the invention, a copolymer of silicone polyamide and of hydrocarbon-based polyamide, or a copolymer including units of formula (III) or (IV) and hydrocarbon-based polyamide units, may be used. In this case, the polyamide-silicone units may be located at the ends of the hydrocarbon-based polyamide.

According to a preferred embodiment, the silicone polyamide comprises units of formula (III).

[Chem 97]

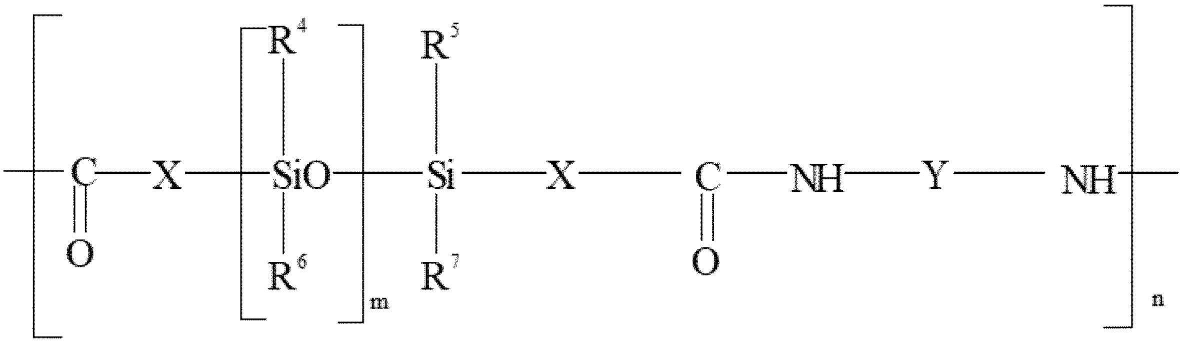

(III)

in which $R^4$, $R^5$, $R^6$ and $R^7$ represent, independently, a linear or branched $C_1$ to $C_{40}$ alkyl group, preferably a $CH_3$, $C_2H_5$, n-$C_3H_7$ or isopropyl group, a polyorganosiloxane chain or a phenyl group optionally substituted with one to three methyl or ethyl groups, and m ranges from 1 to 700, in particular from 15 to 500 and notably from 50 to 200 and n ranges in particular from 1 to 500, preferably from 1 to 100 and better still from 4 to 25.

Preferably, according to this embodiment, the groups $R^4$, $R^5$, $R^6$ and $R^7$ represent methyl groups, one from among X and Y represents an alkylene group of 6 carbon atoms and the other represents an alkylene group of 11 carbon atoms, n representing the degree of polymerization (DP) of the polymer.

Examples of such silicone polyamides that may be mentioned include the compounds sold by the company Dow Corning under the names DC 2-8179 Gellant® (DP 100) and DC 2-8178 Gellant® (DP 15), the INCI name of which is Nylon-611/dimethicone copolymer.

Advantageously, the composition according to the invention comprises at least one polydimethylsiloxane block polymer of general formula (I) with an m value of about 100.

The m value corresponds to the degree of polymerization of the silicone portion of the polymer.

More preferably, the composition according to the invention comprises at least one polymer comprising at least one unit of formula (III) in which m ranges from 50 to 200, in particular from 75 to 150 and is preferably about 100.

As examples of silicone polymers that may be used, mention may be made of one of the silicone polyamides obtained in accordance with Examples 1 to 3 of US-A-5 981 680.

According to a preferred embodiment, use is made of a polyamide silicone having the INCI name: Nylon-611/dimethicone copolymer sold by the company Dow Corning under the name DC 2-8179 Gellant® (DP 100).

The polymers and/or copolymers used in the composition of the invention advantageously have a temperature of transition from the solid state to the liquid state ranging from 45 to 190° C. Preferably, they have a temperature of transition from the solid state to the liquid state ranging from 70 to 130° C and better still from 80 to 105° C.

The content of silicone polyamide, expressed as active material, preferably ranges from 5% to 30% by weight, more preferentially from 8% to 25% by weight and more particularly from 10% to 25% by weight relative to the weight of composition (B).

According to a particular form of the invention, composition (B) according to the invention may comprise at least one mixture of a silicone polyamide as described previously in combination with a silicone resin as defined previously.

Preferentially, said mixture consists of a silicone polyamide having the INCI name: Nylon-611/Dimethicone Polymer with an MQ silicone resin having the INCI name Trimethoxysiloxysilicate.

According to a particular form of the invention, the silicone polyamide/silicone resin mixture is present in composition (B) in a silicone polyamide/silicone resin mass ratio ranging from 0.3 to 0.7 and more preferentially from 0.4 to 0.6. According to a particular form of the invention, the mixture of silicone polyamide and silicone resin is present in composition (B) in a content ranging from 8% to 50% by weight, preferably from 10% to 45% by weight and more preferentially from 12% to 40% by weight, relative to the weight of composition (B).

Pseudo-blocks

According to one embodiment, composition (B) according to the invention may comprise at least one block ethylenic copolymer (also known as a block ethylenic polymer), containing at least one first block with a glass transition temperature (Tg) of greater than or equal to 40° C and being totally or partly derived from one or more first monomers, which are such that the homopolymer prepared from these monomers has a glass transition temperature of greater than or equal to 40° C, and at least one second block with a glass transition temperature of less than or equal to 20° C and being

derived totally or partly from one or more second monomers, which are such that the homopolymer prepared from these monomers has a glass transition temperature of less than or equal to 20° C, said first block and said second block being connected together via a statistical intermediate segment comprising at least one of said first constituent monomers of the first block and at least one of said second constituent monomers of the second block, and said block copolymer having a polydispersity index I of greater than 2.

The block polymer used according to the invention thus comprises at least one first block and at least one second block.

The term *"at least* one block" means one or more blocks.

The term "block" polymer means a polymer comprising at least two different blocks and preferably at least three different blocks.

The term "ethylenic polymer" means a polymer obtained by polymerization of ethylenically unsaturated monomers.

The block ethylenic polymer used according to the invention is prepared exclusively from monofunctional monomers.

This means that the block ethylenic polymer used according to the present invention does not contain any multifunctional monomers, which make it possible to break the linearity of a polymer so as to obtain a branched or even crosslinked polymer, as a function of the content of multifunctional monomer. The polymer used according to the invention does not, either, contain any macromonomers (the term "macromonomer" means a monofunctional monomer containing a pendent group of polymeric nature, and preferably having a molecular mass of greater than 500 g/mol, or alternatively a polymer including on only one of its ends a polymerizable (or ethylenically unsaturated) end group), which are used in the preparation of a grafted polymer.

It is pointed out that, in the text hereinabove and hereinbelow, the terms "first" and "second" blocks do not in any way condition the order of said blocks in the structure of the polymer.

The first block and the second block of the polymer used in the invention may be advantageously mutually incompatible.

The term "mutually incompatible blocks" means that the mixture formed from a polymer corresponding to the first block and from a polymer corresponding to the second block is not miscible in the polymerization solvent that is in major amount by weight for the block polymer, at room temperature (25° C) and atmospheric pressure ($10^5$ Pa), for a content of the mixture of said polymers of greater than or equal to 5% by weight, relative to the total weight of the mixture of said polymers and of said polymerization solvent, it being understood that:

i) said polymers are present in the mixture in a content such that the respective weight ratio ranges from 10/90 to 90/10, and that

ii) each of the polymers corresponding to the first and second blocks has an average (weight-average or number-average) molar mass equal to that of the block polymer ± 15%.

In the case of a mixture of polymerization solvents, and in the event that two or more solvents are present in identical mass proportions, said polymer mixture is immiscible in at least one of them.

Needless to say, in the case of a polymerization performed in a single solvent, this solvent is the solvent that is in major amount.

The block polymer according to the invention comprises at least one first block and at least one second block that are connected together via an intermediate segment comprising at least one constituent monomer of the first block and at least one constituent monomer of the second block. The intermediate segment (also known as the intermediate block) has a glass transition temperature Tg that is between the glass transition temperatures of the first and second blocks.

The intermediate segment is a block comprising at least one constituent monomer of the first block and at least one constituent monomer of the second block of the polymer allowing these blocks to be "compatibilized".

Advantageously, the intermediate segment comprising at least one constituent monomer of the first block and at least one constituent monomer of the second block of the polymer is a statistical polymer.

Preferably, the intermediate block is derived essentially from constituent monomers of the first block and of the second block.

The term "essentially" means at least 85%, preferably at least 90%, better still 95% and even better still 100%.

The block polymer according to the invention is advantageously a film-forming block ethylenic polymer.

The term "ethylenic polymer" means a polymer obtained by polymerization of ethylenically unsaturated monomers.

The term "film-forming polymer" means a polymer that is capable of forming, by itself or in the presence of an auxiliary film-forming agent, a continuous deposit on a support, notably on keratin materials.

Preferentially, the polymer according to the invention does not comprise any silicon atoms in its backbone. The term "backbone" means the main chain of the polymer, as opposed to the pendent side chains.

Preferably, the polymer according to the invention is not water-soluble, i.e. the polymer is not soluble in water or in a mixture of water and linear or branched lower monoalcohols containing from 2 to 5 carbon atoms, for instance ethanol, isopropanol or n-propanol, without modifying the pH, at an active material content of at least 1% by weight, at room temperature (25° C).

Preferably, the polymer according to the invention is not an elastomer.

The term "non-elastomeric polymer" means a polymer which, when it is subjected to a constraint intended to pull it (for example by 30% relative to its initial length), does not return to a length substantially identical to its initial length when the constraint ceases.

The polydispersity index of the polymer of the invention is greater than 2.

Advantageously, the block polymer used in the compositions according to the invention has a polydispersity index I of greater than 2, for example ranging from 2 to 9, preferably greater than or equal to 2.5, for example ranging from 2.5 to 8 and better still greater than or equal to 2.8, and in particular ranging from 2.8 to 6.

The polydispersity index I of the polymer is equal to the ratio of the weight-average mass Mw to the number-average mass Mn.

The weight-average molar mass (Mw) and number-average molar mass (Mn) are determined by gel permeation liquid chromatography (THF solvent, calibration curve established with linear polystyrene standards, refractometric detector).

The weight-average mass (Mw) of the polymer according to the invention is preferably less than or equal to 300 000 g/mol; it ranges, for example, from 35 000 to 200 000 g/mol and better still from 45 000 to 150 000 g/mol.

The number-average mass (Mn) of the polymer according to the invention is preferably less than or equal to 70 000 g/mol; it ranges, for example, from 10 000 to 60 000 g/mol and better still from 12 000 to 50 000 g/mol.

Preferably, the polydispersity index of the polymer according to the invention is greater than 2, for example ranging from 2 to 9, preferably greater than or equal to 2.5, for example ranging from 2.5 to 8, and better still greater than or equal to 2.8, and notably ranging from 2.8 to 6.

First block with a Tg of greater than or equal to 40° C

The block with a Tg of greater than or equal to 40° C has, for example, a Tg ranging from 40 to 150° C, preferably greater than or equal to 50° C, for example ranging from 50° C to 120° C and better still greater than or equal to 60° C, for example ranging from 60° C to 120° C.

The glass transition temperatures indicated for the first and second blocks may be theoretical Tg values determined from the theoretical Tg values of the constituent monomers of each of the blocks, which may be found in a reference manual such as the Polymer Handbook, 3rd Edition, 1989, John Wiley, according to the following relationship, known as Fox's law:

$$1/Tg = \Sigma \ (\omega_i / Tg_i),$$

$\omega_i$ being the mass fraction of the monomer i in the block under consideration and $Tg_i$ being the glass transition temperature of the homopolymer of the monomer i.

Unless otherwise indicated, the Tg values indicated for the first and second blocks in the present patent application are theoretical Tg values.

The difference between the glass transition temperatures of the first and second blocks is generally greater than 10° C, preferably greater than 20° C and better still greater than 30° C.

The block with a Tg of greater than or equal to 40° C may be a homopolymer or a copolymer.

The block with a Tg of greater than or equal to 40° C may be derived totally or partially from one or more monomers which are such that the homopolymer prepared from these monomers has a glass transition temperature of greater than or equal to 40° C. This block may also be referred to as a "rigid block".

In the case where this block is a homopolymer, it is derived from monomers which are such that the homopolymers prepared from these monomers have glass transition temperatures of greater than or equal to 40° C. This first block may be a homopolymer consisting of only one type of monomer (for which the Tg of the corresponding homopolymer is greater than or equal to 40° C).

In the case where the first block is a copolymer, it may be totally or partially derived from one or more monomers, the nature and concentration of which are chosen such that the Tg of the resulting copolymer is greater than or equal to 40° C. The copolymer may comprise, for example:

- monomers which are such that the homopolymers prepared from these monomers have Tg values of greater than or equal to 40° C, for example a Tg ranging from 40° C to 150° C, preferably greater than or equal to 50° C, for example

ranging from 50° C to 120° C and better still greater than or equal to 60° C, for example ranging from 60° C to 120° C, and

- monomers which are such that the homopolymers prepared from these monomers have Tg values of less than 40° C, chosen from monomers with a Tg of between 20° C and 40° C and/or monomers with a Tg of less than or equal to 20° C, for example a Tg ranging from -100° C to 20° C, preferably less than 15° C, notably ranging from -80° C to 15° C and better still less than 10° C, for example ranging from -50° C to 0° C, as described later.

The first monomers whose homopolymers have a glass transition temperature of greater than or equal to 40° C are chosen, preferably, from the following monomers, also known as the main monomers:

- the methacrylates of formula $CH_2 = C(CH_3)-COOR_1$, in which $R_1$ represents a linear or branched unsubstituted alkyl group containing from 1 to 4 carbon atoms, such as a methyl, ethyl, propyl or isobutyl group or $R_1$ represents a $C_4$ to $C_{12}$ cycloalkyl group, preferably a $C_8$ to $C_{12}$ cycloalkyl, such as isobornyl methacrylate,
- the acrylates of formula $CH_2 = CH-COOR_2$ in which $R_2$ represents a $C_4$ to $C_{12}$ cycloalkyl group such as an isobornyl group or a tert-butyl group,
- (meth)acrylamides of formula:

[Chem 98]

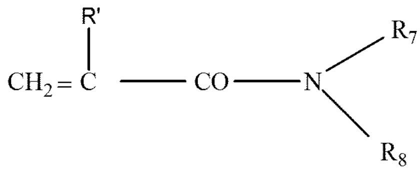

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and R' denotes H or methyl. Examples of monomers that may be mentioned include N-butylacrylamide, N-tert-butylacrylamide, N-isopropylacrylamide, N,N-dimethylacrylamide and N,N-dibutylacrylamide,
- and mixtures thereof.

The first block is advantageously obtained from at least one acrylate monomer of formula $CH_2=CH-COOR_2$ and from at least one methacrylate monomer of formula $CH_2=C(CH_3)-COOR_2$ in which $R_2$ represents a $C_4$ to $C_{12}$ cycloalkyl group, preferably a $C_8$ to $C_{12}$ cycloalkyl, such as isobornyl. The monomers and the proportions thereof are preferably chosen such that the glass transition temperature of the first block is greater than or equal to 40° C.

According to one embodiment, the first block is obtained from:

i) at least one acrylate monomer of formula $CH_2=CH-COOR_2$ in which $R_2$ represents a $C_4$ to $C_{12}$ cycloalkyl group, preferably a $C_8$ to $C_{12}$ cycloalkyl, such as isobornyl,
ii) and at least one methacrylate monomer of formula $CH_2 = C(CH_3)-COOR'_2$ in which $R'_2$ represents a $C_4$ to $C_{12}$ cycloalkyl group, preferably a $C_8$ to $C_{12}$ cycloalkyl group, such as isobornyl.

According to one embodiment, the first block is obtained from at least one acrylate monomer of formula $CH_2=CH-COOR_2$ in which $R_2$ represents a $C_8$ to $C_{12}$ cycloalkyl group, such as isobornyl, and from at least one methacrylate monomer of formula $CH_2=C(CH_3)-COOR'_2$ in which $R'_2$ represents a $C_8$ to $C_{12}$ cycloalkyl group, such as isobornyl.

Preferably, $R_2$ and $R'_2$ represent, independently or simultaneously, an isobornyl group.

Preferably, the block copolymer comprises from 50% to 80% by weight of isobornyl methacrylate/acrylate, from 10% to 30% by weight of isobutyl acrylate and from 2% to 10% by weight of acrylic acid.

The first block may be obtained exclusively from said acrylate monomer and from said methacrylate monomer.

The acrylate monomer and the methacrylate monomer are preferably in mass proportions of between 30/70 and 70/30, preferably between 40/60 and 60/40 and notably of the order of 50/50.

The proportion of the first block advantageously ranges from 20% to 90%, better still from 30% to 80% and even better still from 60% to 80% by weight of the polymer.

According to one embodiment, the first block is obtained by polymerization of isobornyl methacrylate and isobornyl acrylate.

Second block with a glass transition temperature of less than 20° C

The second block advantageously has a glass transition temperature Tg of less than or equal to 20° C, for example, a Tg ranging from -100° C to 20° C, preferably less than or equal to 15° C, notably ranging from -80° C to 15° C and better still less than or equal to 10° C, for example ranging from -100° C to 10° C, notably ranging from -30° C to 10° C.
The second block is totally or partially derived from one or more second monomers, which are such that the homopolymer prepared from these monomers has a glass transition temperature of less than or equal to 20° C.
This block may also be referred to as a "flexible block".
The monomer with a Tg of less than or equal to 20° C (known as the second monomer) is preferably chosen from the following monomers:

- the acrylates of formula $CH_2 = CHCOOR_3$,
  $R_3$ representing a linear or branched, unsubstituted $C_1$ to $C_{12}$ alkyl group, with the exception of the tert-butyl group, in which one or more heteroatoms chosen from O, N and S are optionally intercalated,
- the methacrylates of formula $CH_2 = C(CH_3)\text{-}COOR_4$,
  $R_4$ representing a linear or branched, unsubstituted $C_6$ to $C_{12}$ alkyl group, in which one or more heteroatoms chosen from O, N and S are optionally intercalated;
- the vinyl esters of formula $R_5\text{-}CO\text{-}O\text{-}CH = CH_2$,
  in which $R_5$ represents a linear or branched $C_4$ to $C_{12}$ alkyl group;
- ethers of vinyl alcohol and of a $C_4$ to $C_{12}$ alcohol,
- N-($C_4$ to $C_{12}$)alkyl acrylamides, such as N-octylacrylamide,
- and mixtures thereof.

The preferred monomers with a Tg of less than or equal to 20° C are isobutyl acrylate, 2-ethylhexyl acrylate or mixtures thereof in all proportions.
Each of the first and second blocks may contain in small proportion at least one constituent monomer of the other block.
Thus, the first block may contain at least one constituent monomer of the second block, and vice versa.
Each of the first and/or second blocks may comprise, in addition to the monomers indicated above, one or more other monomers known as additional monomers, which are different from the main monomers mentioned previously.
The nature and amount of this or these additional monomer(s) are chosen such that the block in which they are present has the desired glass transition temperature.
This additional monomer is chosen, for example, from:

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof,
- the methacrylates of formula $CH_2 = C(CH_3)\text{-}COOR_6$,
  in which $R_6$ represents a linear or branched alkyl group containing from 1 to 4 carbon atoms, such as a methyl, ethyl, propyl or isobutyl group, said alkyl group being substituted with one or more substituents chosen from hydroxyl groups (for instance 2-hydroxypropyl methacrylate and 2-hydroxyethyl methacrylate) and halogen atoms (Cl, Br, I or F), such as trifluoroethyl methacrylate,
- the methacrylates of formula $CH_2 = C(CH_3)\text{-}COOR_9$,
  $R_9$ representing a linear or branched $C_6$ to $C_{12}$ alkyl group in which one or more heteroatoms chosen from O, N and S are optionally intercalated, said alkyl group being substituted with one or more substituents chosen from hydroxyl groups and halogen atoms (Cl, Br, I or F);
- the acrylates of formula $CH_2 = CHCOOR_{10}$,
  $R_{10}$ representing a linear or branched $C_1$ to $C_{12}$ alkyl group substituted with one or more substituents chosen from hydroxyl groups and halogen atoms (Cl, Br, I or F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit 5 to 10 times, for example methoxy-POE, or $R_{10}$ represents a polyoxyethylenated group comprising from 5 to 10 ethylene oxide units.

In particular, the first block may comprise as additional monomer:

- (meth)acrylic acid, preferably acrylic acid,
- tert-butyl acrylate,
- the methacrylates of formula $CH_2 = C(CH_3)\text{-}COOR_1$,
  in which $R_1$ represents a linear or branched unsubstituted alkyl group containing from 1 to 4 carbon atoms, such as a

methyl, ethyl, propyl or isobutyl group,

- (meth)acrylamides of formula:

[Chem 99]

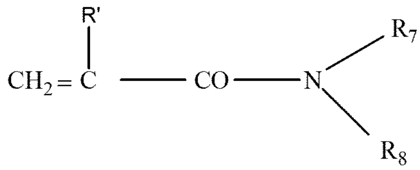

in which $R_7$ and $R_8$, which may be identical or different, each represent a hydrogen atom or a linear or branched $C_1$ to $C_{12}$ alkyl group such as an n-butyl, t-butyl, isopropyl, isohexyl, isooctyl or isononyl group; or $R_7$ represents H and $R_8$ represents a 1,1-dimethyl-3-oxobutyl group, and
R' denotes H or methyl. Examples of monomers that may be mentioned include N-butylacrylamide, N-tert-butylacrylamide, N-isopropylacrylamide, N,N-dimethylacrylamide and N,N-dibutylacrylamide,
and mixtures thereof.

The additional monomer may represent 0.5% to 30% by weight relative to the weight of the polymer. According to one embodiment, the polymer of the invention does not contain any additional monomer.

Preferably, the polymer of the invention comprises at least isobornyl acrylate and isobornyl methacrylate monomers in the first block and isobutyl acrylate and acrylic acid monomers in the second block.

Preferably, the polymer comprises at least isobornyl acrylate and isobornyl methacrylate monomers in equivalent weight proportion in the first block and isobutyl acrylate and acrylic acid monomers in the second block.

Preferably, the polymer comprises at least isobornyl acrylate and isobornyl methacrylate monomers in equivalent weight proportion in the first block, and isobutyl acrylate and acrylic acid monomers in the second block, the first block representing 70% by weight of the polymer.

Preferably, the polymer comprises at least isobornyl acrylate and isobornyl methacrylate monomers in equivalent weight proportion in the first block and isobutyl acrylate and acrylic acid monomers in the second block. Preferably, the block with a Tg of greater than 40° C represents 70% by weight of the polymer, and acrylic acid represents 5% by weight of the polymer.

According to one embodiment, the first block does not comprise any additional monomer.

According to a preferred embodiment, the second block comprises acrylic acid as additional monomer. In particular, the second block is advantageously obtained from an acrylic acid monomer and from at least one other monomer with a Tg of less than or equal to 20° C.

According to a preferred embodiment, the composition according to the invention comprises at least one copolymer comprising at least one acrylate monomer of formula $CH_2=CH-COOR_2$ in which $R_2$ represents a $C_8$ to $C_{12}$ cycloalkyl group and/or at least one methacrylate monomer of formula $CH_2=C(CH_3)-COOR'_2$ in which $R'_2$ represents a $C_8$ to $C_{12}$ cycloalkyl group at least one second acrylate monomer of formula $CH_2=CHCOOR_3$, in which $R_3$ is an unsubstituted linear or branched $C_1$ to $C_{12}$ alkyl group, with the exception of the tert-butyl group, and at least one acrylic acid monomer.

Preferably, the copolymer used in the compositions according to the invention is obtained from at least one isobornyl methacrylate monomer, at least one isobornyl acrylate monomer, at least one isobutyl acrylate monomer and at least one acrylic acid monomer.

Advantageously, the copolymer used in the invention comprises from 50% to 80% by weight of isobornyl methacrylate/acrylate mixture, from 10% to 30% by weight of isobutyl acrylate and from 2% to 10% by weight of acrylic acid.

The block copolymer may advantageously comprise more than 2% by weight of acrylic acid monomers, and notably from 2% to 15% by weight, for example from 3% to 15% by weight, in particular from 4% to 15% by weight or even from 4% to 10% by weight of acrylic acid monomers, relative to the total weight of said copolymer.

The constituent monomers of the second block and the proportions thereof are chosen such that the glass transition temperature of the second block is less than or equal to 20° C.

<u>Intermediate segment</u>

The intermediate segment (also known as the intermediate block) connects the first block and the second block of the polymer used according to the present invention. The intermediate segment results from the polymerization:

i) of the first monomer(s), and optionally of the additional monomer(s), which remain available after their polymerization to a maximum degree of conversion of 90% to form the first block,
ii) and of the second monomer(s), and optionally of the additional monomer(s), added to the reaction mixture.

The formation of the second block is initiated when the first monomers no longer react or are no longer incorporated into the polymer chain either because they are all consumed or because their reactivity no longer allows them to be.

Thus, the intermediate segment comprises the first available monomers, resulting from a degree of conversion of these first monomers of less than or equal to 90%, during the introduction of the second monomer(s) during the synthesis of the polymer.

The intermediate segment of the block polymer is a statistical polymer (which may also be referred to as a statistical block). This means that it comprises a statistical distribution of the first monomer(s) and of the second monomer(s) and also of the additional monomer(s) that may be present.

Thus, the intermediate segment is a statistical block, as are the first block and the second block if they are not homopolymers (i.e. if they are both formed from at least two different monomers).

Process for preparing the copolymer:

The block ethylenic copolymer according to the invention is prepared by free radical polymerization, according to the techniques that are well known for this type of polymerization.

The free radical polymerization is performed in the presence of an initiator, the nature of which is adapted, in a known manner, as a function of the desired polymerization temperature and of the polymerization solvent. In particular, the initiator may be chosen from initiators bearing a peroxide function, redox couples or other free radical polymerization initiators known to those skilled in the art.

In particular, examples of initiators bearing a peroxide function that may be mentioned include:

a) peroxyesters such as tert-butyl peroxyacetate, tert-butyl perbenzoate, tert-butyl peroxy-2-ethylhexanoate (Trigonox 21S® from AkzoNobel) or 2,5-bis(2-ethylhexanoylperoxy)-2,5-dimethylhexane (Trigonox 141 from AkzoNobel);
b) peroxydicarbonates such as diisopropyl peroxydicarbonate;
c) peroxy ketones such as methyl ethyl ketone peroxide;
d) hydroperoxides such as aqueous hydrogen peroxide solution ($H_2O_2$) or tert-butyl hydroperoxide;
e) diacyl peroxides such as acetyl peroxide or benzoyl peroxide;
f) dialkyl peroxides such as di-tert-butyl peroxide;
g) mineral peroxides such as potassium peroxodisulfate ($K_2S_2O_8$).

As initiator in the form of a redox couple, mention may be made of the potassium thiosulfate + potassium peroxodisulfate couple, for example.

According to a preferred embodiment, the initiator is chosen from organic peroxides comprising from 8 to 30 carbon atoms. Preferably, the initiator used is 2,5-bis(2-ethylhexanoylperoxy)-2,5-dimethylhexane sold under the reference Trigonox® 141 by the company AkzoNobel.

The block copolymer used according to the invention is prepared by free radical polymerization and not by controlled or living polymerization. In particular, the polymerization of the block ethylenic copolymer is performed in the absence of control agents, and in particular in the absence of control agents conventionally used in living or controlled polymerization processes, such as nitroxides, alkoxyamines, dithioesters, dithiocarbamates, dithiocarbonates or xanthates, trithiocarbonates or copper-based catalysts, for example.

Block copolymers such as those described previously are notably described in patent applications EP-A-1411069 and EP-A-1882709.

The synthetic solvent used for the polymerization of the film-forming copolymer is generally chosen from volatile oils with a flash point below 80° C, for instance isododecane.

The composition according to the invention preferably comprises from 0.5% to 40% by weight, advantageously from 1% to 40% by weight, notably from 2% to 30% by weight or even from 2% to 20% by weight of block ethylenic copolymer by weight of active material, relative to the total weight of the composition.

Preferably, the composition according to the invention comprises at least 2% by weight of active material (i.e. solids) of block ethylenic copolymer, relative to the total weight of the composition.

Oily dispersions

Composition (B) according to the invention comprises at least one oily dispersion comprising at least:

i) particles including:

a) at least one ethylenic copolymer of $a_1$) ($C_1$-$C_4$)alkyl ($C_1$-$C_4$)(alkyl)acrylate, and of $a_2$) ethylenic monomers comprising one or more carboxyl, anhydride, phosphoric acid, sulfonic acid and/or aryl, such as benzyl, groups; in particular,

$a_2$) is a ($C_1$-$C_4$)(alkyl)acrylic acid, more particularly copolymers of ($C_1$-$C_4$)alkyl (meth)acrylate and of (meth) acrylic acid; and

ii) at least one polymeric stabilizer chosen from:

b) polymers of ($C_3$-$C_{12}$)cycloalkyl ($C_1$-$C_6$)(alkyl)acrylate monomers such as isobornyl (meth)acrylates; and
c) copolymers of $c_1$) ($C_3$-$C_{12}$)cycloalkyl ($C_1$-$C_6$)(alkyl)acrylate such as ($C_1$-$C_4$)alkyl (meth)acrylate; and

iii) at least one hydrocarbon-based oil, preferably a volatile hydrocarbon-based oil.

The dispersions according to the invention are therefore constituted of particles, which are generally spherical, of at least one polymer i) which is surface-stabilized with one or more stabilizers ii), in a nonaqueous medium.

In order to obtain the dispersion, it is proposed to polymerize particular monomers that are capable of forming the polymeric core i) in the presence of a polymeric statistical stabilizer ii) comprising in major amount a portion ii) that is soluble and in minor amount a portion i) that is insoluble in the dispersion medium, i.e. in the hydrocarbon-based liquid fatty substance(s) iii) optionally containing iv) water.

Preferably, said particles i) are not or are only sparingly crosslinked.

The polymer particles i) and the stabilizer(s) ii) are preferably in the hydrocarbon-based liquid fatty substance(s) iii) in an amount of between 2% and 40% by weight, notably between 4% and 35% by weight of soluble monomer (the one forming the stabilizer(s) ii)) and between 60% and 98% by weight, notably from 65% to 96% by weight of insoluble monomer (the one forming the particles i)).

*Polymer particles i)*

The particle(s) i) of the dispersion of the process of the invention are constituted of one or more ethylenic copolymers derived from the polymerization $a_1$) of at least one ($C_1$-$C_4$)alkyl ($C_1$-$C_4$)(alkyl)acrylate monomer and $a_2$) of at least one ethylenic monomer comprising one or more carboxyl, anhydride, phosphoric acid, sulfonic acid and/or aryl groups; in particular, $a_2$) is a ($C_1$-$C_4$)(alkyl)acrylic acid.

Preferably, the particle(s) i) are constituted of an ethylenic polymeric core derived from copolymers a), as defined previously.

The term *"ethylenic copolymer"* means a polymer resulting from the polymerization of two monomers: of ($C_1$-$C_4$) alkyl ($C_1$-$C_4$)(alkyl)acrylate monomer $a_1$) and of ethylenic monomer $a_2$) comprising one or more carboxyl, anhydride, phosphoric acid, sulfonic acid and/or aryl, such as phenyl, groups.

The term *"ethylenic monomer"* means a compound comprising at least one ethylenic unsaturation -($R_a$) C=C($R_b$)-, -C($R_a$)=C($R_b$)-$R_c$ or >C=C($R_a$)-$R_b$, with $R_a$, $R_b$, and $R_c$, which may be identical or different, representing a hydrogen atom or a ($C_1$-$C_4$)alkyl group such as methyl, preferably hydrogen.

The ethylenic compound may also be a cyclic compound, which is preferably 5- or 6-membered, and comprising an ethylenic unsaturation.

The term "ethylenic monomer comprising *one or more carboxyl, anhydride, phosphoric acid, sulfonic acid and/or aryl groups"* means that the monomer is substituted on the one of the carbon atoms of the polymeric monomer with one or more groups, preferably a single group, chosen from carboxyl -C(O)-OH, phosphoric acid -O-P(O)$(OH)_2$ or -P(O)$(OH)_2$, and sulfonic acid -O-$S(O)_2$-OH or -$S(O)_2$-OH, maleic anhydride, and aryl such as phenyl.

According to one embodiment of the invention, the particle(s) i) include a) ethylenic copolymers of $a_1$) ($C_1$-$C_4$) alkyl ($C_1$-$C_4$)(alkyl)acrylate and of $a_2$) ethylenic monomers comprising one or more carboxyl, anhydride, phosphoric acid, sulfonic acid and/or aryl, such as benzyl, groups.

More particularly, the ethylenic monomers $a_2$) comprising one or more carboxyl, anhydride, phosphoric acid, sulfonic acid and/or aryl groups are chosen from (1), (2), (3), and (4):

(1) $R_1(R_2)C=C(R_3)$-Acid with $R_1$, $R_2$ and $R_3$ representing a hydrogen atom or a $CO_2H$, $H_2PO_4$ or $SO_3H$ group, and Acid representing a carboxyl, phosphoric acid or sulfonic acid group, preferably a carboxyl group; preferably, (1) represents (5) $H_2C=C(R)$-C(O)-O-H with R representing a hydrogen atom or a ($C_1$-$C_4$)alkyl group such as methyl;
(2) $H_2C=C(R)$-C(O)-N(R')-Alk-Acid with R and R', which may be identical or different, representing a hydrogen atom or a ($C_1$-$C_4$)alkyl group; Alk represents a ($C_1$-$C_6$)alkylene group optionally substituted with at least one group chosen

from Acid as defined previously and hydroxyl; and Acid is as defined previously, preferably carboxyl or sulfonic acid;
(3) Ar-($R_a$)C=C($R_b$)-$R_c$ with $R_a$, $R_b$ and $R_c$, which may be identical or different, representing a hydrogen atom or a ($C_1$-$C_4$)alkyl group, and Ar representing an aryl group, preferably benzyl, optionally substituted with at least one acid group $CO_2H$, $H_2PO_4$ or $SO_3H$, preferably substituted with a $CO_2H$ or $SO_3H$ group;
(4) maleic anhydride of formulae (4a) and (4b):

[Chem 100]

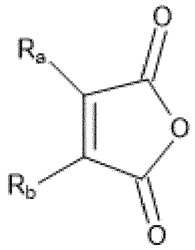

(4a)

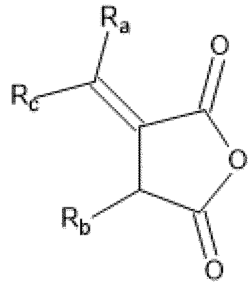

(4b)

in which formulae (4a) and (4b) $R_a$, $R_b$ and $R_c$, which may be identical or different, represent a hydrogen atom or a ($C_1$-$C_4$)alkyl group; preferably, $R_a$, $R_b$ and $R_c$ represent a hydrogen atom. Preferentially, the ethylenically unsaturated anhydride monomer of the invention is of formula (4b) and more preferentially is maleic anhydride; and more particularly $a_2$) is chosen from (1) and (4), in particular (4).

According to one particular variant of the invention, $a_2$) is a ($C_1$-$C_4$)(alkyl)acrylic acid, more particularly b) is (are) copolymers of ($C_1$-$C_4$)alkyl (meth)acrylate and of (meth)acrylic acid.

According to another variant of the invention, $a_2$) is chosen from crotonic acid, maleic anhydride, itaconic acid, fumaric acid, maleic acid, styrenesulfonic acid, vinylbenzoic acid, vinylphosphoric acid, acrylic acid, acrylamidopropanesulfonic acid, acrylamidoglycolic acid, and salts thereof; more preferentially, $a_2$) represents maleic anhydride.

The salts may be chosen from salts of alkali metals, for example sodium or potassium; salts of alkaline-earth metals, for example calcium, magnesium or strontium; metal salts, for example zinc, aluminum, manganese or copper; ammonium salts of formula $NH_4^+$; quaternary ammonium salts; salts of organic amines, for instance salts of methylamine, diethylamine, trimethylamine, triethylamine, ethylamine, 2-hydroxyethylamine, bis(2-hydroxyethyl)amine or tris(2-hydroxyethyl)amine; lysine or arginine salts.

According to a preferred embodiment of the invention, the ($C_1$-$C_4$)alkyl ($C_1$-$C_4$)(alkyl)acrylate monomer(s) $a_1$) are chosen from those of formula (VIII):
[Chem 101]

**$H_2C=C(R)-C(O)-O-R'$** **(VIII)**

in which formula (VIII):

- R represents a hydrogen atom or a ($C_1$-$C_4$)alkyl group such as methyl, and
- R' represents a ($C_1$-$C_4$)alkyl group such as methyl, ethyl, n-propyl, i-propyl, n-butyl, i-butyl, t-butyl, preferably methyl, ethyl or i-butyl,

preferably, (VIII) represents a $C_1$-$C_4$ alkyl acrylate such as methyl acrylate, ethyl acrylate, isobutyl acrylate and isobutyl methacrylate.

According to another particular embodiment of the invention, the polymer constituting the particles i) is an ethylenic acrylate copolymer a) resulting from the polymerization:

- $a_1$) of at least one monomer of formula (VIII) as defined previously, preferably a $C_1$-$C_4$ alkyl acrylate such as methyl acrylate, ethyl acrylate, isobutyl acrylate and isobutyl methacrylate; and
- $a_2$) of at least one monomer chosen from (1), (2), (3) and (4) as defined previously and preferentially of formula (IX) and also the salts thereof:
[Chem 102]

$$H_2C=C(R)-C(O)-O-H \qquad (IX)$$

in which formula (IX) R is as defined previously; in particular, (IX) represents acrylic acid.

According to a preferred embodiment of the invention, the amount of $a_2$) notably of compound (IX) and in particular of acrylic acid is greater than 2.5% by weight relative to the total weight of the particle i), more particularly between 3% and 35% by weight relative to the weight of monomers of the particle i), even more particularly between 5% and 25% by weight and the polymer of the particles i), better still between 10% and 15% by weight and the polymer of the particles i).

In particular, a) is a copolymer derived from the copolymerization $a_2$) of acrylic acid with $a_1$) one or more $C_1$-$C_4$ alkyl (meth)acrylate monomers, preferably at least two different $C_1$-$C_4$ alkyl (meth)acrylate monomers, in particular chosen from methyl (meth)acrylate, ethyl (meth)acrylate, butyl (meth)acrylate and isobutyl (meth)acrylate.

According to another preferred embodiment of the invention, the polymer constituting the particles i) is an ethylenic acrylate copolymer a) resulting from the polymerization:

- of at least two different monomers: of formula (VIII) as defined previously, preferably a $C_1$-$C_4$ alkyl acrylate such as methyl acrylate, ethyl acrylate, isobutyl (meth)acrylate; and
- of at least one monomer chosen from (1), (2), (3) and (4) as defined previously, preferably of formula (IX) as defined previously.

According to a particular embodiment of the invention, the polymer of the particles i) is a polymer derived from $C_1$-$C_4$ alkyl (meth)acrylate monomers $a_1$).

Advantageously, a linear or branched, preferably linear, $C_1$-$C_4$ alkyl acrylate, in particular ($C_1$-$C_3$)alkyl acrylate, monomer is used. Preferentially, a) is chosen from methyl acrylate and ethyl acrylate.

The monomers $a_1$) are preferably chosen from methyl (meth)acrylate, ethyl (meth)acrylate, n-propyl (meth) acrylate, isopropyl (meth)acrylate, n-butyl (meth)acrylate, isobutyl (meth)acrylate and tert-butyl (meth)acrylate, more preferentially chosen from methyl (meth)acrylate and ethyl (meth)acrylate.

According to another particular embodiment of the invention, the polymer constituting the particles i) is an acrylate copolymer a) resulting:

$a_1$) from at least one monomer of formula (VIII) as defined previously; and

$a_2$) from at least one ethylenically unsaturated anhydride monomer, notably of formula (4) as defined previously.

Particularly, the polymer of the particles a) is a polymer $a_1$) of linear or branched, preferably linear, $C_1$-$C_4$ alkyl (meth)acrylate, in particular ($C_1$-$C_3$)alkyl (meth)acrylate, and $a_2$) of an ethylenically unsaturated anhydride monomer.

The monomers $a_1$) are preferably chosen from methyl (meth)acrylate, ethyl (meth)acrylate, n-propyl (meth) acrylate, isopropyl (meth)acrylate, n-butyl (meth)acrylate, isobutyl (meth)acrylate and tert-butyl (meth)acrylate.

Advantageously, use is made of one or two different linear or branched, preferably linear, $C_1$-$C_4$ alkyl acrylate, in particular ($C_1$-$C_3$)alkyl acrylate monomers $a_1$). Preferentially, $a_1$) is chosen from methyl acrylate and ethyl acrylate.

According to a particular embodiment, use is made of one or two different branched ($C_3$-$C_4$)alkyl (meth)acrylate monomers. Preferentially, $a_1$) is chosen from isobutyl acrylate and isobutyl methacrylate.

According to a particular form of the invention, the ethylenically unsaturated anhydride compound(s) $a_2$) of the invention are chosen from derivatives of maleic anhydride (4a) and of itaconic anhydride (4b) as defined previously.

More preferentially, the ethylenically unsaturated anhydride monomer of the invention is of formula (4a) and even more preferentially is maleic anhydride.

The polymer of the particles may be chosen from: methyl acrylate/maleic anhydride copolymers; ethyl acrylate/maleic anhydride copolymers; and methyl acrylate/ethyl acrylate/maleic anhydride copolymers.

According to a preferred embodiment of the invention, the copolymer(s) of the particles i) of the oily dispersion comprise from 80% to 95% by weight of ingredient $a_1$) and from 5% to 20% by weight of ingredient $a_2$), relative to the total weight of the copolymer(s).

More particularly, the polymer of the particles i) preferably comprises from 80% to 93% by weight of ingredient $a_1$), notably of $C_1$-$C_4$ alkyl (meth)acrylate, and from 7% to 20% by weight of ingredient $a_2$) relative to the total weight of polymer i). More preferentially, the polymer of the particles i) preferably comprises from 85% to 93% by weight, more particularly 87% to 92% by weight, even more preferentially from 85% to 90% by weight of ingredient $a_1$), notably of $C_1$-$C_4$ alkyl (meth) acrylate, and from 10% to 20% by weight of ingredient $a_2$) relative to the total weight of the polymer.

Advantageously, the polymer of the particles i) is a non-crosslinked polymer.

According to one embodiment of the invention, the copolymers i) are chosen from:

- copolymers of methyl acrylate/ethyl acrylate/acrylic acid and its salts;
- copolymers of methyl acrylate/ethyl acrylate/maleic anhydride;
- copolymers of methyl acrylate/acrylic acid and its salts;
- copolymers of ethyl acrylate/acrylic acid and its salts;
- copolymers of methyl acrylate/maleic anhydride;
- copolymers of ethyl acrylate/maleic anhydride;
- copolymers of isobutyl acrylate/acrylic acid and its salts; and
- copolymers of isobutyl acrylate/isobutyl methacrylate/acrylic acid and its salts.

The polymer of the particles i) of the dispersion preferably has a number-average molecular weight ranging from 2000 to 10 000 000 g/mol.

The polymer of the particles i) may be present in the oily dispersion in a content ranging from 20% to 60% by weight relative to the total weight of the dispersion, in particular between 21% and 58.5% by weight relative to the total weight of the dispersion, preferably ranging from 30% to 50% by weight relative to the total weight of the dispersion, more preferentially ranging from 36% to 42% by weight relative to the total weight of the oily dispersion.

Preferably, the particle(s) i) are constituted of a copolymer of $a_1$) and $a_2$)

According to a particular embodiment of the invention, the particles i) are constituted of copolymers resulting from the polymerization of monomers $a_1$) and $a_2$) with an $a_1$) / $a_2$) weight ratio inclusively between 4.4 and 20, more particularly between 5.5 and 19, preferably between 6.5 and 16 and even more preferentially between 6.6 and 15.6.

The polymer particle(s) i) of the oily dispersion preferably have a number-mean size ranging from 5 to 600 nm, notably ranging from 10 to 500 nm and better still ranging from 20 to 400 nm.

*The stabilizer(s) ii)*

The oily dispersion according to the invention also comprises one or more stabilizers ii). Preferably, only one type of stabilizer ii) is used in the invention.

The stabilizer(s) of the invention are constituted of ethylenic polymers chosen from b) polymers of ($C_3$-$C_{12}$) cycloalkyl ($C_1$-$C_6$)(alkyl)acrylate monomers; and c) copolymers of $c_1$) ($C_3$-$C_{12}$)cycloalkyl ($C_1$-$C_6$)(alkyl)acrylate and $c_2$) ($C_1$-$C_4$)alkyl ($C_1$-$C_4$)(alkyl)acrylate. Preferably, the stabilizer(s) of the invention are constituted of ethylenic polymers chosen from c) copolymers of $c_1$) ($C_3$-$C_{12}$)cycloalkyl ($C_1$-$C_6$)(alkyl)acrylate and $c_2$) ($C_1$-$C_4$)alkyl ($C_1$-$C_4$)(alkyl)acrylate.

According to a preferred embodiment of the invention, the stabilizer ii) is constituted of ethylenic polymers chosen from:

b) polymers of monomers of formula $H_2C=C(R)-C(O)-O-R''$ (X) with R representing a hydrogen atom or ($C_1$-$C_4$)alkyl group such as methyl, and R"
representing a ($C_5$-$C_{10}$)cycloalkyl group such as norbornyl or isobornyl, preferably isobornyl; and
c) the copolymers of $c_1$) $H_2C=C(R)-C(O)-O-R''$ (X) in which R and R" have the same meanings indicated previously in b) and of
$c_2$) $H_2C=C(R)-C(O)-O-R'$ (VIII) in which R has the same meaning as that in formula (X) and R' represents a ($C_1$-$C_4$) alkyl group.

According to a particular embodiment, the stabilizer(s) of the invention are constituted of ethylenic polymers chosen from b) polymers of ($C_3$-$C_{12}$)cycloalkyl ($C_1$-$C_6$)(alkyl)acrylate monomers, particularly of formula (X) as defined previously.

According to another particular embodiment of the invention, the stabilizer(s) of the invention are constituted of ethylenic polymers chosen from c) copolymers of $c_1$) ($C_3$-$C_{12}$)cycloalkyl ($C_1$-$C_6$)(alkyl)acrylate, particularly of formula (X) as defined previously and of $c_2$) ($C_1$-$C_4$)alkyl ($C_1$-$C_4$)(alkyl)acrylate, particularly of formula (VIII) as defined previously.

According to a particular form of the invention, the ingredient $c_2$) is a ($C_1$-$C_4$)alkyl ($C_1$-$C_4$)(alkyl)acrylate monomer, notably of formula (VIII), more particularly chosen from methyl (meth)acrylate and ethyl (meth)acrylate, more preferentially methyl (meth)acrylate.

According to another particular form of the invention, the ingredient $c_2$) is a mixture of several different ($C_1$-$C_4$) alkyl ($C_1$-$C_4$)(alkyl)acrylate monomers, notably two different ($C_1$-$C_4$)alkyl ($C_1$-$C_4$)(alkyl)acrylate monomers, notably of formula (VIII), more particularly chosen from methyl (meth)acrylate and ethyl (meth)acrylate.

Particularly, the stabilizer ii) is chosen from b) polymers of ($C_3$-$C_{12}$)cycloalkyl ($C_1$-$C_6$)(alkyl)acrylate monomers; and c) statistical copolymers of $c_1$) ($C_3$-$C_{12}$)cycloalkyl ($C_1$-$C_6$)(alkyl)acrylate and of $c_2$) ($C_1$-$C_4$)alkyl ($C_1$-$C_4$)(alkyl)acrylate with a $c_1$)/$c_2$) weight ratio of greater than 4. Advantageously, said weight ratio ranges from 4.5 to 19. More advantageously, said $c_1$)/$c_2$) weight ratio ranges from 5 to 15 and more preferentially said weight ratio ranges from 5.5 to 12.

More particularly, the stabilizer ii) is a polymer chosen from b) isobornyl (meth)acrylate homopolymer and c)

statistical copolymers of $c_1$) isobornyl (meth)acrylate and of $c_2$) $C_1$-$C_4$ alkyl (meth)acrylate preferably present in an isobornyl (meth)acrylate/$C_1$-$C_4$ alkyl (meth)acrylate ($c_1$)/$c_2$)) weight ratio of greater than 4. Advantageously, said $c_1$)/$c_2$) weight ratio ranges from 4.5 to 19. Advantageously, said
$c_1$)/$c_2$) weight ratio ranges from 5 to 15 and more preferentially said $c_1$)/$c_2$) weight ratio ranges from 5.5 to 12.

For these statistical copolymers, the defined weight ratio makes it possible to obtain a polymer dispersion that is stable, notably after storage for seven days at room temperature.

Advantageously, the stabilizer is chosen from: b) isobornyl acrylate homopolymers, c) statistical copolymers of isobornyl acrylate/methyl acrylate, statistical copolymers of isobornyl acrylate/ethyl acrylate, and statistical copolymers of isobornyl acrylate/methyl acrylate/ethyl acrylate in the weight ratio described previously.

The stabilizing polymer ii) preferably has a number-average molecular weight ranging from 10 000 to 400 000 g/mol, preferably ranging from 20 000 to 200 000 g/mol.

The stabilizer ii) is in contact with the surface of the polymer particles i) and thus makes it possible to stabilize these particles at the surface in order to keep these particles in dispersion in the medium of the oily dispersion.

More particularly, the stabilizer(s) ii) are chosen from c) copolymers of: $c_1$) ($C_3$-$C_{12}$)cycloalkyl ($C_1$-$C_6$)(alkyl) acrylate; and

> $c_2$) ($C_1$-$C_4$)alkyl ($C_1$-$C_4$)(alkyl)acrylate;
> with from 80% to 95% by weight of ingredient $c_1$), notably of isobornyl (meth)acrylate, and from 5% to 20% by weight of ingredient $c_2$), notably methyl acrylate and ethyl acrylate, relative to the total weight of the stabilizer.

More preferentially, the stabilizer(s) ii) comprise from 85% to 94% by weight, more particularly 87% to 93% by weight of ingredient $c_1$), notably of isobornyl (meth)acrylate, and from 10% to 20% by weight of ingredient $c_2$), notably methyl acrylate and/or ethyl acrylate, relative to the total weight of the polymer.

Advantageously, the combination of ii) stabilizer(s) + i) polymer particle(s) present in the oily dispersion comprises from 5% to 60%, in particular comprises from 10% to 40% by weight of polymers b) or c) and from 60% to 90%, in particular from 61% to 89% by weight of polymers a), relative to the total weight of the combination of ii) stabilizer(s) + i) polymer particle(s).

Preferentially, the combination of ii) stabilizer(s) + i) polymer particle(s) present in the dispersion comprises from 15% to 30% by weight of polymers b) or c) and from 70% to 85% by weight of polymers a), relative to the total weight of the combination of ii) stabilizer(s) + i) polymer particle(s).

Preferably, the stabilizer(s) ii) and the particle(s) i) have a number-average molecular weight (Mn) of between 1000 and 1000 000 g/mol, notably between 5000 and 500 000 g/mol and better still between 10 000 and 300 000 g/mol.

The oily dispersion according to the invention is finally formed from polymer particles, of relatively large diameter, i.e. preferably greater than 100 nm, and leads to shiny film-forming deposits which are resistant to fatty substances at room temperature (25° C), and which are notably advantageous for makeup applications.

The final particle size is greater than 100 nm. In particular, they have a number-average size ranging from 100 nm to 600 nm, more particularly ranging from 150 nm to 500 nm and even more particularly ranging from 160 nm to 400 nm.

The average size of the particles is determined via conventional methods known to those skilled in the art. A Malvern brand NanoZS model laser particle size analyzer (which is particularly suitable for submicron dispersions) makes it possible to measure the size distribution of these samples. The operating principle of this type of machine is based on dynamic light scattering (DLS), also known as quasi-elastic light scattering (QELS) or photon correlation spectroscopy (PCS).

The sample is pipetted into a disposable plastic cuvette (four transparent faces, side length of 1 cm and volume of 4 mL) placed in the measuring cell. The data are analyzed on the basis of a cumulant fit method which leads to a monomodal particle size distribution characterized by an intensity-weighted mean diameter d (nm) and a size polydispersity factor Q. The results may also be expressed in the form of statistical data such as D10; D50 (median), D90 and the mode.

Other particle size analysis techniques make it possible to obtain this type of information, such as analysis of the individual tracking of particles (Nanoparticle Tracking Analysis, NTA), laser scattering (LS), acoustic extinction spectroscopy (AES), spatial-filter Doppler velocimetry or image analysis.

*The hydrocarbon-based oil(s) iii)*

The oily medium of the polymer dispersion comprises at least one hydrocarbon-based oil iii).

The hydrocarbon-based oil is an oil that is liquid at room temperature (25° C) and at atmospheric pressure.

The term "hydrocarbon-based oil" means an oil formed essentially from, or even constituted of, carbon and hydrogen atoms, and optionally oxygen and nitrogen atoms, and not containing any silicon or fluorine atoms. It can contain alcohol, ester, ether, carboxylic acid, amine and/or amide groups.

Advantageously, the hydrocarbon-based oil(s) of the invention are formed solely of carbon and hydrogen atoms.

The hydrocarbon-based oil may be chosen from nonvolatile hydrocarbon-based oils.

The term "nonvolatile oil" means an oil that remains on the keratin fiber at room temperature (25° C) and atmospheric pressure for at least several hours and that notably has a vapor pressure of less than $10^{-3}$ mmHg (0.13 Pa).

These oils may be hydrocarbon-based oils.

The term "hydrocarbon-based oil" means an oil mainly containing hydrogen and carbon atoms and optionally oxygen, nitrogen, sulfur or phosphorus atoms.

Nonvolatile hydrocarbon-based oils that may notably be mentioned include:

- hydrocarbon-based oils of plant origin, such as fatty acid triesters of glycerol, the fatty acids of which may have varied chain lengths from $C_4$ to $C_{24}$, these chains possibly being linear or branched, and saturated or unsaturated; these oils are notably wheatgerm oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, sesame seed oil, marrow oil, rapeseed oil, blackcurrant oil, evening primrose oil, millet oil, barley oil, quinoa oil, rye oil, safflower oil, candlenut oil, passion flower oil and musk rose oil; or alternatively caprylic/capric acid triglycerides such as those sold by the company Stearinerie Dubois or those sold under the names Miglyol 810®, 812® and 818® by the company Dynamit Nobel;
- linear or branched hydrocarbons of mineral or synthetic origin, such as liquid paraffins and derivatives thereof, polydecenes, polybutenes, hydrogenated polyisobutene such as Parleam, or squalane;
- synthetic ethers containing from 10 to 40 carbon atoms,
- synthetic esters such as oils of formula $R_1COOR_2$ in which $R_1$ represents a linear or branched fatty acid residue including from 1 to 40 carbon atoms and $R_2$ represents a, notably branched, hydrocarbon-based chain containing from 1 to 40 carbon atoms, on condition that $R_1 + R_2 \geq 10$, for instance purcellin oil (cetostearyl octanoate), isopropyl myristate, isopropyl palmitate, $C_{12}$ to $C_{15}$ alkyl benzoates, hexyl laurate, diisopropyl adipate, isononyl isononanoate, 2-ethylhexyl palmitate, isostearyl isostearate, alcohol or polyalcohol octanoates, decanoates or ricinoleates such as propylene glycol dioctanoate; hydroxylated esters such as isostearyl lactate and diisostearyl malate; and pentaerythritol esters;
- fatty alcohols that are liquid at room temperature, with a branched and/or unsaturated carbon-based chain containing from 12 to 26 carbon atoms, for instance octyldodecanol, isostearyl alcohol, oleyl alcohol, 2-hexyldecanol, 2-butyloctanol and 2-undecylpentadecanol;
- higher fatty acids such as oleic acid, linoleic acid or linolenic acid; and mixtures thereof.

According to a particularly preferred form of the invention, the hydrocarbon-based oil will be chosen from volatile hydrocarbon-based oils as defined previously. In particular, the volatile hydrocarbon-based oil is isododecane.

Preferably, the liquid hydrocarbon-based oil(s) iii) are present in the dispersion of the invention in an amount of between 15% and 80% by weight, more preferentially between 20% and 60% by weight, relative to the total weight of said oily dispersion. According to a particular embodiment of the invention, the weight ratio of the combination of the ingredients [i) + ii)] / iii) is less than or equal to 1, more particularly the [i) + ii)] / iii) weight ratio is between 0.5 and 1.

The polymer particles i) of the dispersion preferably have an average size, notably a number-average size, ranging from 50 to 500 nm, notably ranging from 75 to 400 nm and better still ranging from 100 to 250 nm.

In general, the dispersion according to the invention may be prepared in the following manner, which is given as an example.

*Method for preparing the dispersion*

Without this being limiting, in general, the dispersion according to the invention may be prepared in the following manner:

- The polymerization is performed in *dispersion* in nonaqueous medium, i.e. by precipitation of the polymer being formed, with protection of the formed particles with one or more stabilizers ii), preferably only one type of stabilizer ii) chosen from b) and c) as defined previously.
- In a first step, the stabilizing polymer (or stabilizer ii)) is prepared by mixing the constituent monomer(s) of the stabilizing polymer b) or c) with iv) a free-radical initiator, in a solvent known as the synthesis solvent, and by polymerizing these monomers; and then
- In a second step, the monomers constituting the polymer of the particles i) is added to the stabilizing polymer ii) formed in the preceding step and polymerization of these added monomers is performed in the presence of the radical initiator.
- In a third step, water is added and the combination of ingredients i) + ii) is stirred in the reactor before taking out the dispersion. When the nonaqueous medium is a nonvolatile hydrocarbon-based oil iii), the polymerization may be

performed in an apolar organic solvent (synthesis solvent), followed by adding the nonvolatile hydrocarbon-based oil (which should be miscible with said synthesis solvent) and selectively distilling off the synthesis solvent.

The synthesis solvent may be constituted of hydrocarbon-based oil iii) combined with an additional solvent notably chosen from linear or branched hydrocarbon-based aliphatic-chain esters containing from 3 to 8 carbon atoms in total, such as ethyl acetate, methyl acetate, propyl acetate or n-butyl acetate.

At the end of step 1, when the synthesis solvent is a mixture, the additional solvent including the hydrocarbon-based aliphatic-chain esters as defined previously is removed via a method that is conventional to those skilled in the art, such as distillation. The polymers of the particles i) are found in the hydrocarbon-based oil iii).

A synthesis solvent which is such that the monomers of the polymeric stabilizer(s) ii) and the free-radical initiator iv) are soluble therein, and such that the polymer particles i) obtained are insoluble therein, so that they precipitate therefrom during their formation, is thus chosen.

In particular, the synthesis solvent chosen is one which is apolar, organic and volatile, preferably chosen from volatile hydrocarbon-based oils iii), notably alkanes such as heptane, cyclohexane or isododecane, preferably isododecane.

When the nonaqueous medium is a volatile hydrocarbon-based oil iii), the polymerization may be performed directly in said oil, which thus also acts as synthesis solvent. The monomers should also be soluble therein, as should the free-radical initiator, and the polymer of the particles i) which is obtained should be insoluble therein.

The monomers are preferably present in the synthesis solvent, before polymerization, in a proportion of from 15% to 45% by weight. The total amount of the monomers may be present in the solvent before the start of the reaction, or a portion of the monomers may be added gradually as the polymerization reaction proceeds.

The polymerization is preferably performed in the presence iv) of one or more free-radical initiators which may be any initiator known to those skilled in the art for radical polymerization, such as peroxide or azo initiators, redox couples and photochemical initiators.

The following types of initiator may notably be mentioned:

- peroxide, in particular chosen from tert-butyl peroxy-2-ethylhexanoate: Trigonox 21S; 2,5-dimethyl-2,5-bis(2-ethylhexanoylperoxy)hexane: Trigonox 141; tert-butyl peroxypivalate: Trigonox 25C75 from AkzoNobel; or
- azo, in particular chosen from AIBN: azobisisobutyronitrile; V50: 2,2'-azobis(2-amidinopropane) dihydrochloride.

The polymerization is preferably performed at a temperature ranging from 70 to 110° C and at atmospheric pressure.

The polymer particles i) are surface-stabilized, when they are formed during the polymerization, by means of the stabilizer ii).

The stabilization may be performed by any known means, and in particular by direct addition of the stabilizer ii), during the polymerization.

The stabilizer ii) is preferably also present in the mixture before polymerization of the monomers of the polymer of the particles i). However, it is also possible to add it continuously, notably when the monomers of the particles i) are also added continuously.

From 2% to 40% by weight, particularly from 3% to 30% by weight and more particularly from 4% to 25% by weight of the stabilizer(s) ii) may be used relative to the total weight of monomers used (stabilizers ii) + polymer particles i), and preferably from 4.5% to 20% by weight.

The dispersion of polymer particles i) advantageously comprises from 30% to 65% by weight of solids relative to the total weight of said dispersion and preferably from 40% to 60% by weight relative to the total weight of said dispersion.

According to a preferred embodiment of the invention, the dispersion of the invention does not comprise more than 3% by weight of surfactants relative to the total weight of the dispersion, preferentially not more than 2% by weight of surfactants relative to the total weight of the dispersion, more particularly not more than 1% by weight of surfactants relative to the total weight of the dispersion; even more preferentially, the composition does not comprise more than 0.5% by weight of surfactants relative to the total weight of the dispersion, and better still the mixture does not comprise any surfactant.

In a particular preparation method, the statistical stabilizing polymer ii) is prepared in a first step. This stabilizing polymer is soluble in an apolar organic solvent of alkane type, such as isododecane.

Next, in a second step, the polymer particles i) are synthesized in the presence of the stabilizing polymer ii).

Preferentially, a solution of stabilizing polymer ii) in the hydrocarbon-based oil(s) iii) is prepared for the final dispersion, and polymerization of the monomers which form the core of the particle is performed in the presence of this stabilizer ii).

The stabilizing polymer ii) may be prepared by radical polymerization optionally in the presence of a polymerization initiator iv) as defined previously.

In a second step, the monomers which form the core of the particle i) may be polymerized in the presence of said

stabilizing polymer ii). This second step may be a conventional radical polymerization.

The dispersions are prepared in the presence of one or more hydrocarbon-based oils iii), preferably in an apolar organic solvent, in particular of alkane type such as isododecane, according to an industrially feasible process.

The dispersions according to the invention are thus finally formed from polymer particles, of relatively large diameter (preferably greater than 100 nm), and give glossy film-forming deposits that are resistant to fatty substances at the observation temperature (25° C).

Advantageously, the oily dispersion may comprise a plasticizer chosen from tri-n-butyl citrate, tripropylene glycol monomethyl ether (INCI name: PPG-3 Methyl Ether) and trimethyl pentaphenyl trisiloxane (sold under the name Dow Corning PH-1555 HRI Cosmetic Fluid by the company Dow Corning). These plasticizers make it possible to improve the mechanical strength of the polymer film.

The plasticizer is preferably present in the oily dispersion in a content ranging from 5% to 50% by weight, relative to the total weight of the polymer of the particles.

Preferentially, use will be made of a dispersion in iii), notably isododecane, of surface-stabilized polymer particles i) chosen from:

- a dispersion in isododecane of methyl acrylate/ethyl acrylate/acrylic acid (24.5/62.8/12.7) copolymer particles stabilized with an isobornyl acrylate/methyl acrylate/ethyl acrylate (92/4/4) statistical copolymer stabilizer; the oily dispersion containing in total (stabilizer + particles) 10% acrylic acid, 20% methyl acrylate, 50% ethyl acrylate and 20% isobornyl acrylate;
- a dispersion in isododecane of methyl acrylate/ethyl acrylate/acrylic acid (11.7/75.6/12.7) copolymer particles stabilized with an isobornyl acrylate/methyl acrylate/ethyl acrylate (92/4/4) statistical copolymer stabilizer; the oily dispersion containing in total (stabilizer + particles) 10% acrylic acid, 10% methyl acrylate, 60% ethyl acrylate and 20% isobornyl acrylate;
- a dispersion in isododecane of methyl acrylate/ethyl acrylate/maleic anhydride (50/37.2/12.8) copolymer particles stabilized with an isobornyl acrylate/methyl acrylate/ethyl acrylate (92/4/4) statistical copolymer stabilizer; the oily dispersion containing in total (stabilizer ii) + particles i)) 10% maleic anhydride, 30% methyl acrylate, 40% ethyl acrylate and 20% isobornyl acrylate.

More preferentially, use will be made of a dispersion in isododecane of methyl acrylate/ethyl acrylate/acrylic acid (11.7/75.6/12.7) copolymer particles stabilized with an isobornyl acrylate/methyl acrylate/ethyl acrylate (92/4/4) statistical copolymer stabilizer; the oily dispersion containing in total (stabilizer ii) + particles i)) 10% of acrylic acid, 10% of methyl acrylate, 60% of ethyl acrylate and 20% of isobornyl acrylate, such as the product having the INCI name: Acrylates/isobornyl acrylate copolymer sold under the trade name Mexomere PBM® from the company Noveal.

Composition (B) according to the invention preferably comprises a solids (or active material) content of polymers of particle i) + dispersing polymers ii) according to the invention ranging from 20% to 45% by weight, relative to the total weight of composition (B), more preferentially ranging from 25% to 40% by weight relative to the total weight of composition (B).

According to a particularly preferred mode of the invention, composition (B) comprises at least one hydrophobic film-forming polymer chosen from silicone polyamides and/or silicone resins, preferably a Nylon-611/dimethicone copolymer silicone polyamide and/or a Trimethoxysiloxysilicate MQ silicone resin.

More particularly, composition (B) comprises at least a mixture of silicone polyamide and silicone resin, and even more particularly a Nylon-611/dimethicone copolymer silicone polyamide and a Trimethoxysiloxysilicate silicone resin.

According to a particular embodiment of the invention, composition (B) will be anhydrous comprising the oily phase as described previously.

According to another particular form of the invention, composition (B) is a water-in-oil emulsion.

<u>Emulsifiers</u>

Composition (B) in the form of water-in-oil emulsions preferably comprises one or more emulsifying surfactants, more preferentially nonionic emulsifying surfactants.

For the purposes of the present invention, the term "emulsifying surfactant" refers to an amphiphilic surfactant compound, i.e. one which contains two parts of different polarity. Generally, one is lipophilic (soluble or dispersible in an oily phase). The other is hydrophilic (soluble or dispersible in water). Emulsifying surfactants are characterized by the value of their HLB (Hydrophilic Lipophilic Balance), the HLB being the ratio of the hydrophilic part to the lipophilic part in the molecule. The term "HLB" is well known to those skilled in the art and is described, for example, in "The HLB System. A Time-Saving Guide to Emulsifier Selection" (published by ICI Americas Inc.; 1984). For the emulsifying surfactants used for the preparation of water-in-oil emulsions, their HLB value is generally less than or equal to 8 and more particularly ranges from 3 to 8. The HLB value may be determined via the Griffin method or the Davies method.

As examples of W/O emulsifying surfactants, mention may be made of alkyl esters or ethers of sorbitan, of glycerol, of polyol or of sugars; silicone surfactants, such as dimethicone copolyols, such as the product having the INCI name Dimethicone (and) PEG/PPG-18/18 Dimethicone sold under the brand name X-22-6711D® by the company Shin-Etsu, the mixture of cyclomethicone and of dimethicone copolyol, sold under the name DC 5225 C® by the company Dow Corning, and alkyldimethicone copolyols such as laurylmethicone copolyol sold under the name Dow Corning 5200 Formulation Aid by the company Dow Corning; cetyl dimethicone copolyol, such as cetyl PEG/PPG-10/1 dimethicone, such as the product sold under the name Abil EM 90® by the company Evonik Goldschmidt, and the mixture of cetyl dimethicone copolyol, of polyglyceryl isostearate (4 mol) and of hexyl laurate, sold under the name Abil WE 09® by the company Goldschmidt. One or more coemulsifiers, which may be chosen advantageously from the group comprising polyol alkyl esters, may also be added thereto.

Mention may also be made of nonsilicone emulsifying surfactants, notably alkyl esters or ethers of sorbitan, of glycerol, of polyol or of sugars.

As polyol alkyl esters, mention may be made notably of polyethylene glycol esters, such as PEG-30 dipolyhydroxystearate, such as the product sold under the name Cithrol DPHS-SO-(MV) by the company Croda.

Examples of glycerol and/or sorbitan esters that may be mentioned include polyglyceryl isostearate (INCI name: Polyglyceryl-4 Isostearate), such as the product sold under the name Isolan GI 34® by the company Evonik Goldschmidt; sorbitan isostearate, such as the product sold under the name Arlacel 987® by the company ICI; sorbitan glyceryl isostearate, such as the product sold under the name Arlacel 986® by the company ICI, the diester of a mixture of isostearic, polyhydroxystearic and sebacic acids with Polyglycerin-4 (INCI name: Polyglyceryl-4 Diisostearate/Polyhydroxystearate/Sebacate), such as the product sold under the name Isolan GPS® by the company Evonik, and mixtures thereof.

Dyestuffs

According to a particular form of the invention, composition (A) and/or (B) according to the invention also comprises at least one dyestuff, in particular chosen from pulverulent dyestuffs.

The pulverulent dyestuffs may be chosen from pigments and nacres.

The term "pigments" means white or colored, mineral or organic particles, which are insoluble in an aqueous medium, and which are intended to color and/or opacify the resulting composition and/or deposit. These pigments may be white or colored, and mineral and/or organic.

According to a particular embodiment, the pigments used according to the invention are chosen from mineral pigments.

The term "mineral pigment" refers to any pigment that satisfies the definition in Ullmann's encyclopedia in the chapter on inorganic pigments. Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminum powder and copper powder. The following mineral pigments may also be used: $Ta_2O_5$, $Ti_3O_5$, $Ti_2O_3$, TiO, $ZrO_2$ as a mixture with $TiO_2$, $ZrO_2$, $Nb_2O_5$, $CeO_2$, ZnS.

The size of the pigment that is useful in the context of the present invention is generally greater than 100 nm and may range up to 10 μm, preferably from 200 nm to 5 μm and more preferentially from 300 nm to 1 μm.

According to a particular form of the invention, the pigments have a size characterized by a D[50] of greater than 100 nm and possibly ranging up to 10 μm, preferably from 200 nm to 5 μm and more preferentially from 300 nm to 1 μm.

The sizes are measured by static light scattering using a commercial MasterSizer 3000 particle size analyzer from Malvern, which makes it possible to determine the particle size distribution of all of the particles over a wide range which may extend from 0.01 μm to 1000 μm. The data are processed on the basis of the standard Mie scattering theory. This theory is the most suitable for size distributions ranging from submicron to multimicron; it allows an "effective" particle diameter to be determined. This theory is notably described in the publication by Van de Hulst, H.C., Light Scattering by Small Particles, Chapters 9 and 10, Wiley, New York, 1957.

D[50] represents the maximum size that 50% by volume of the particles have.

According to a particular form of the invention, the mineral pigment comprises a lipophilic or hydrophobic coating, said coating preferably being present in the fatty phase of the composition according to the invention.

According to a particular embodiment of the invention, the pigments may be coated according to the invention with at least one compound chosen from silicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

According to a preferential embodiment, the pigments may be coated according to the invention with an N-acylamino acid or a salt thereof, which may comprise an acyl group containing from 8 to 22 carbon atoms, for instance a 2-ethylhexanoyl, caproyl, lauroyl, myristoyl, palmitoyl, stearoyl or cocoyl group. The amino acid may be, for example, lysine,

glutamic acid or alanine. The salts of these compounds may be the aluminum, magnesium, calcium, zirconium, zinc, sodium or potassium salts. Thus, according to a particularly preferred embodiment, the pigments may be coated with an N-acylamino acid derivative which may notably be a glutamic acid derivative and/or a salt thereof, and more particularly a stearoyl glutamate, for instance aluminum stearoyl glutamate. As examples of pigments treated with aluminum stearoyl glutamate, mention may be made of titanium dioxide pigments and black, red and yellow iron oxide pigments sold under the trade name NAI® by the company Miyoshi Kasei.

According to a preferential embodiment, the pigments may be coated according to the invention with isopropyl triisostearyl titanate. As examples of isopropyl titanium triisostearate (ITT)-treated pigments, mention may be made of those sold under the commercial references BWBO-I2® (Iron oxide CI77499 and isopropyl titanium triisostearate), BWYO-I2® (Iron oxide CI77492 and isopropyl titanium triisostearate) and BWRO-I2® (Iron oxide CI77491 and isopropyl titanium triisostearate) by the company Kobo.

The pigments that may be used according to the invention may also be organic pigments.

The term "organic pigment" refers to any pigment that satisfies the definition in Ullmann's encyclopedia in the chapter on organic pigments. The organic pigment may notably be chosen from nitroso, nitro, azo, xanthene, quinoline, anthraquinone, phthalocyanine, metal complex type, isoindolinone, isoindoline, quinacridone, perinone, perylene, diketopyrrolopyrrole, thioindigo, dioxazine, triphenylmethane and quinophthalone compounds.

The organic pigment(s) may be chosen, for example, from carmine, carbon black, aniline black, melanin, azo yellow, quinacridone, phthalocyanine blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenol derivatives as described in patent FR 2 679 771.

These pigments may also be in the form of composite pigments as described in patent EP 1 184426. These composite pigments may notably be composed of particles including a mineral core at least partially covered with an organic pigment and at least one binder for fixing the organic pigments to the core.

The pigment may also be a lake. The term "lake" means insolubilized dyes adsorbed onto insoluble particles, the assembly thus obtained remaining insoluble during use.

The mineral substrates onto which the dyes are adsorbed are, for example, alumina, silica, calcium sodium borosilicate or calcium aluminum borosilicate and aluminum.

Among the organic dyes, mention may be made of cochineal carmine. Mention may also be made of the products known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green 5 (CI 61 570), D&C Yellow 10 (CI 77 002), D&C Green 3 (CI 42 053), D&C Blue 1 (CI 42 090).

An example of a lake that may be mentioned is the product known under the name D&C Red 7 (CI 15 850:1).

Preferably, the composition according to the invention comprises a pulverulent dyestuff, preferably of mineral pigment type, in particular metal oxides, notably coated or uncoated titanium dioxides or iron oxides and mixtures thereof.

The nacres may be chosen from white nacreous pigments such as mica coated with titanium or with bismuth oxychloride, colored nacreous pigments such as titanium mica with iron oxides, titanium mica notably with ferric blue or chromium oxide, titanium mica with an organic pigment of the abovementioned type, and also nacreous pigments based on bismuth oxychloride.

Preferably, said dyestuff is present in the composition in a content ranging from 0.2% to 25.0% by weight, preferably from 0.5% to 20.0% by weight, more particularly from 1% to 15.0% by weight, relative to the total weight of the composition.

According to a particular form of the invention, the makeup kit according to the invention contains at least:

i) a base-coat makeup composition (A) comprising at least one direct acid dye chosen from Acid Violet 43 (CI 60730), Acid Green 25 (CI 61570), Acid Blue 80 (CI 61785), Acid Blue 62 (CI 62045), Acid Violet 50 (CI 50325), FD & C Red 4 (CI 14700), Acid Red 4 (CI 14720), Acid Red 88 (CI 15620), D & C Red 7 (CI 15850), FD & C Red 40 (CI 16035), Acid Red 27 (CI 16185), Acid Red 28 (CI 16255), Acid Red 41 (CI 16290), Acid Red 33 (CI 17200), Acid Red 1 (CI 18050), Acid Red 155 (CI 18130), Acid Red 180 (CI 18736), Acid Red 163 (CI 24790), Acid Red 73 (CI 27290), Acid Orange 6 (CI 14270), Acid Orange 7 (CI 15510), Food Orange 2 (CI 15980), Food Yellow 3 (CI 15985), Acid Orange 10 (CI 16230), Acid Orange 24 (CI 20170), Acid Black 1 (CI 20470), Food Black 2 (CI 27755), Food Black 1 (CI 28440), Acid Black 2 (CI 50420), Acid Yellow 9 (CI 13015), Acid Yellow 11 (CI 18820), Acid Yellow 17 (CI 18965), Acid Yellow 23 (CI 19140), Betanin, Bromocresol Green, Food Green 3 (CI 42053), Acid Green 9 (CI 42100), Acid Green 22 (CI 42170),

Acid Green 50 (CI 44090), Bromothymol Blue, Acid Blue 1 (CI 42045), Acid Blue 7 (CI 42080), Acid Blue 9 (CI 42090), Acid Blue 104 (CI 42735), Acid Violet 9 (CI 45190), Acid Red 52 (CI 45100), Acid Red 50 (CI 45220), Acid Red 87 (CI 45380), Acid Red 98 (CI 45405), Acid Red 92 (CI 45410), Acid Red 95 (CI 45425), Acid Red 51 (CI 45430), Acid Orange 11 (CI 45370), Solvent Orange 16 (CI 45396), Acid Yellow 73 (CI 45350), Acid Yellow 73 Fluorescein (CI 45340), Acid Yellow 1 (CI 10316) and more particularly one or more dyes chosen from FD & C Red 40 (CI 16035), Acid Red 33 (CI 17200), Acid Blue 1 (CI 42045), Acid Yellow 23 (CI 19140), and mixtures thereof; and

ii) a top-coat fixing composition (B) comprising at least one silicone resin and/or one silicone polyamide as described previously, and more particularly a mixture of silicone resin and silicone polyamide.

According to another particular form, a makeup kit according to the invention contains at least:

i) a "base-coat" makeup composition (A) comprising at least one direct acid dye chosen from Acid Violet 43 (CI 60730), Acid Green 25 (CI 61570), Acid Blue 80 (CI 61785), Acid Blue 62 (CI 62045), Acid Violet 50 (CI 50325), FD & C Red 4 (CI 14700), Acid Red 4 (CI 14720), Acid Red 88 (CI 15620), D & C Red 7 (CI 15850), FD & C Red 40 (CI 16035), Acid Red 27 (CI 16185), Acid Red 28 (CI 16255), Acid Red 41 (CI 16290), Acid Red 33 (CI 17200), Acid Red 1 (CI 18050), Acid Red 155 (CI 18130), Acid Red 180 (CI 18736), Acid Red 163 (CI 24790), Acid Red 73 (CI 27290), Acid Orange 6 (CI 14270), Acid Orange 7 (CI 15510), Food Orange 2 (CI 15980), Food Yellow 3 (CI 15985), Acid Orange 10 (CI 16230), Acid Orange 24 (CI 20170), Acid Black 1 (CI 20470), Food Black 2 (CI 27755), Food Black 1 (CI 28440), Acid Black 2 (CI 50420), Acid Yellow 9 (CI 13015), Acid Yellow 11 (CI 18820), Acid Yellow 17 (CI 18965), Acid Yellow 23 (CI 19140), Betanin, Bromocresol Green, Food Green 3 (CI 42053), Acid Green 9 (CI 42100), Acid Green 22 (CI 42170), Acid Green 50 (CI 44090), Bromothymol Blue, Acid Blue 1 (CI 42045), Acid Blue 7 (CI 42080), Acid Blue 9 (CI 42090), Acid Blue 104 (CI 42735), Acid Violet 9 (CI 45190), Acid Red 52 (CI 45100), Acid Red 50 (CI 45220), Acid Red 87 (CI 45380), Acid Red 98 (CI 45405), Acid Red 92 (CI 45410), Acid Red 95 (CI 45425), Acid Red 51 (CI 45430), Acid Orange 11 (CI 45370), Solvent Orange 16 (CI 45396), Acid Yellow 73 (CI 45350), Acid Yellow 73 Fluorescein (CI 45340), Acid Yellow 1 (CI 10316) and more particularly one or more dyes chosen from FD & C Red 40 (CI 16035), Acid Red 33 (CI 17200), Acid Blue 1 (CI 42045), Acid Yellow 23 (CI 19140), and mixtures thereof; and

ii) a "top-coat" fixing composition (B) as described previously comprising a dispersion in isododecane of methyl acrylate/ethyl acrylate/acrylic acid (11.7/75.6/12.7) copolymer particles stabilized with an isobornyl acrylate/methyl acrylate/ethyl acrylate (92/4/4) statistical copolymer stabilizer; the oily dispersion containing in total (stabilizer ii) + particles i)) 10% of acrylic acid, 10% of methyl acrylate, 60% of ethyl acrylate and 20% of isobornyl acrylate, such as the product having the INCI name: Acrylates/isobornyl acrylate copolymer sold under the trade name Mexomere PBM® from the company Chimex.

## Cosmetic compositions (A) and (B)

According to a particular embodiment, compositions (A) and (B) in accordance with the present invention comprise a physiologically acceptable medium.

Each of the compositions (A) and (B) may also comprise a cosmetic additive usually used in anhydrous or aqueous supports, respectively, notably chosen from preserving agents, bactericidal agents, antioxidants, fragrances, fillers, hydrophilic thickeners or gelling agents, lipophilic thickeners or gelling agents, lipophilic active agents, hydrophilic active agents, liposoluble dyes, moisturizers, emollients, surfactants, sunscreens, odor absorbers, and mixtures thereof.

It is a matter of routine practice for those skilled in the art to adjust the nature and amount of the additives present in the compositions in accordance with the invention such that the desired cosmetic properties thereof are not thereby affected.

Preferentially, the base-coat composition (A) according to the invention may comprise at least one hydrophilic aqueous-phase thickener and/or gelling agent.

### Hydrophilic gelling agent

According to a particular form of the invention, the makeup composition (A) according to the invention also comprises one or more hydrophilic gelling agent(s).

For the purposes of the present invention, the term "hydrophilic gelling agent" means a compound that is capable of gelling the aqueous phase of the compositions according to the invention. More particularly, the function of these hydrophilic gelling agents is to structure the aqueous phase of the composition. The gelling agent is advantageously soluble in the aqueous phase of the composition. The gelling agent that may be used according to the invention may notably be characterized by its ability to form, in water, beyond a certain concentration C*, a gel characterized by oscillatory rheology ($\mu$ = 1 Hz) by a yield point $\tau$ c at least equal to 10 Pa. This concentration C* can vary widely depending on the nature of the gelling agent under consideration.

The hydrophilic gelling agents may be chosen from carboxyvinyl polymers, such as Carbopols® (Carbomers) and Pemulens® (acrylate/C10-C30 alkyl acrylate copolymer); polyacrylamides, for instance the crosslinked copolymers sold under the names Sepigel 305® (INCI name: polyacrylamide/$C_{13}$-$C_{14}$ isoparaffin/Laureth 7) or Simulgel 600 (INCI name: Acrylamide/Sodium acryloyldimethyl taurate copolymer/ Isohexadecane/Polysorbate-80) by the company SEPPIC; optionally crosslinked and/or neutralized polymers and copolymers of 2-acrylamido-2-methylpropanesulfonic acid, such as the poly(2-acrylamido-2-methylpropanesulfonic acid) sold by the company Hoechst under the trade name Hostacerin AMPS® (INCI name: Ammonium Polyacryloyldimethyl Taurate) or Simulgel 800® sold by the company SEPPIC (INCI name: Sodium Polyacryolyldimethyl Taurate/Polysorbate 80/Sorbitan Oleate); copolymers of 2-acrylamido-2-methylpropanesulfonic acid and of hydroxyethyl acrylate, such as Simulgel NS® and Sepinov EMT 10®, which are sold by the company SEPPIC; cellulose compounds, such as hydroxyethylcellulose; polysaccharides and notably gums, such as xanthan gum or hydroxypropylated guar gums.
Preferably, use will be made of cellulose compounds, in particular hydroxyethylcellulose.
The hydrophilic gelling agent(s) are preferably present in the composition in a total content ranging from 0.1% to 10%, better still from 0.5% to 5% by weight, relative to the total weight of said composition (A).
Preferentially, the top-coat composition (B) according to the invention may also comprise at least one oil thickener, gelling agent and/or structuring agent.
This (these) compounds may be chosen notably from lipophilic gelling agents, for instance hydrophobic-modified clays, such as modified magnesium silicate (Bentone Gel VS38® from Rheox), hectorite modified with distearyldimethylammonium chloride (CTFA name: Disteardimonium hectorite) sold notably under the name Bentone 38 CE® by the company Rheox or under the name Bentone 38 VCG® by the company Elementis.

## Eyebrow makeup process

One subject of the present patent application is a cosmetic process for making up keratin materials, in particular the eyebrows and notably the skin around the eyebrows, which consists in applying to said keratin materials:

- at least one first step of applying a first coat of a makeup composition (A) as defined previously to said keratin materials, and
- at least one second step of applying, to the coat formed by composition (A), at least one second coat of a fixing composition (B) as defined previously.

Between the application of the first coat with the base-coat composition (A) and the application of the second coat with the top-coat composition (B), the drying time varies as a function of the applicator and of the amount deposited, preferably from 1 to 20 minutes, more preferentially from 3 to 15 minutes.

## Packaging and applicators

Compositions (A) and (B) according to the invention may each be packaged in a container delimiting at least one compartment that comprises said composition (A) or (B), said container being closed by a closing member.
The container may be in any suitable form. It may notably be in the form of a bottle, a tube, a jar or a case.
The closing member may be in the form of a removable stopper, a lid or a cover, notably of the type including a body fixed to the container and a cap articulated on the body. It may also be in the form of a member for selectively closing the container, notably a pump, a valve or a flap valve.
The container may be combined with an applicator, notably in the form of a brush including an arrangement of bristles maintained by a twisted wire. Such a twisted brush is described notably in patent US 4 887 622. It may also be in the form of a comb including a plurality of application members, obtained notably by molding. Such combs are described, for example, in patent FR 2 796 529. The applicator may be in the form of a fine brush, as described, for example, in patent FR 2 722 380. The applicator may be in the form of a block of foam or of elastomer. The applicator may be free (sponge) or securely fastened to a rod borne by the closing member, as described, for example, in patent US 5 492 426. The applicator may be securely fastened to the container, as described, for example, in patent FR 2 761 959.
The product may be contained directly in the container, or indirectly.
The closing member may be coupled to the container by screwing. Alternatively, the coupling between the closing member and the container occurs other than by screwing, notably via a bayonet mechanism, by click-fastening or by gripping. The term "click-fastening" in particular means any system involving the crossing of a bead or cord of material by elastic deformation of a portion, notably of the closing member, followed by return to the elastically unconstrained position of said portion after the bead or cord has been crossed.
The container may be at least partially made of thermoplastic material. Examples of thermoplastic materials that may be mentioned include polypropylene and polyethylene.

The container may have rigid or deformable walls, notably in the form of a tube or a tube bottle.

The container may comprise means intended to bring about or facilitate the dispensing of the composition. By way of example, the container may have deformable walls so as to make the composition exit in response to excess pressure inside the container, which excess pressure is brought about by the elastic (or non-elastic) squeezing of the walls of the container.

The container may be equipped with a drainer positioned in the vicinity of the opening of the container. Such a drainer makes it possible to wipe the applicator and possibly the rod to which it may be securely fastened. Such a drainer is described, for example, in patent FR 2 792 618.

Throughout the description, including the claims, the term "including a" should be understood as being synonymous with "including at least one", unless otherwise specified.

The expressions "between ... and ...", and "ranging from ... to ..." should be understood as meaning limits included, unless otherwise specified.

The invention will now be described by means of examples which are presented for purely illustrative purposes and should not be interpreted as examples that limit the invention.

In the description and the examples, unless otherwise indicated, the amounts indicated are expressed as mass percentages. The ingredients are mixed in the order and under the conditions that are readily determined by a person skilled in the art.

**Polymer Preparation Example $P_1$**

A dispersion of polymer particles i) in isododecane was prepared according to the preparation method of Example 1, using:

Step 1: 315.2 g of isobornyl acrylate, 12.5 g of methyl acrylate, 12.5 g of ethyl acrylate, 3.4 g of Trigonox 21, 540 g of isododecane, 360 g of ethyl acetate;
followed by addition, after reaction, of 540 g of isododecane and 360 g of ethyl acetate.
Step 2: 145 g of methyl acrylate, 934 g of ethyl acrylate, 157 g of acrylic acid, 12.36 g of Trigonox 21S, 741.6 g of isododecane and 494.4 g of ethyl acetate.

After reaction, 3 litres of an isododecane/ethyl acetate mixture (60/40 weight/weight) were added and total evaporation of the ethyl acetate and partial evaporation of the isododecane was performed to obtain a solids content of 44% by weight.

A dispersion in isododecane of methyl acrylate/ethyl acrylate/acrylic acid (11.7/75.6/12.7) copolymer particles i) stabilized with an isobornyl acrylate/methyl acrylate/ethyl acrylate (92/4/4) statistical copolymer stabilizer ii) was obtained. The oily dispersion contains in total (particles i) + stabilizer ii) 10% acrylic acid, 10% methyl acrylate, 60% ethyl acrylate and 20% isobornyl acrylate.

**Makeup Kit Examples 1 to 4**

**Kit Example 1 (according to the invention)**

**Base-coat makeup composition (A1)**

[Tables 1]

| Ingredients | Amount (weight %) | Phase |
|---|---|---|
| Hydroxyethylcellulose | 1.35 | (a) |
| Water | qs 100 | |
| FD&C Red 40 | 0.25 | (b) |
| CI 16035 | | |
| Polysorbate-80 | 0.5 | |
| Citric acid | 1.5 | |
| Propylene carbonate | 9.0 | (c) |
| Denat. alcohol | 10.00 | |

(continued)

| Ingredients | Amount (weight %) | Phase |
|---|---|---|
| Sodium hydroxide | qs pH = 3 | **(d)** |

**Preparation protocol**

The hydroxyethylcellulose gel of phase (a) was developed in water at a temperature of 70° C until homogenization. The mixture was then cooled with stirring to a room temperature of 25° C. Phase (b) was introduced into the mixture, followed by phase (c). Next, phase (d) was added to adjust the pH of the composition to 3.

**Top-coat fixation composition ($B_1$)**

[Tables 2]

| Ingredients (INCI name) | Amount (weight %) | Phase |
|---|---|---|
| Isododecane | qs 100 | **(a)** |
| Trimethyl siloxysilicate (SR 1000® from Momentive Performance Materials) | 17.5 | |
| Disteardimonium Hectorite (and) Propylene Carbonate (Bentone Gel ISD V® from Elementis) | 26 | **(b)** |
| Isododecane | 20 | |
| Nylon-611/dimethicone copolymer (Dow Corning 2-8179 Gellant®) | 11 | **(c)** |

**Preparation method**

Phase (a) was prepared by mixing the ingredients composing this phase at room temperature until a transparent homogeneous phase was obtained. The ingredients of phases (b) and (c) were melted in a pan at a temperature of 95° C until a homogeneous mixture was obtained.

Phase (a) was added and the stirring and heating were maintained until a homogeneous mixture was obtained. The mixture was then cooled with stirring until it returned to room temperature (25° C).

**Kit Example 2 (according to the invention)**

The base-coat makeup composition is composition ($A_1$) as described in Kit Example 1.

**Top coat composition ($B_2$)**

[Tables 3]

| Ingredients (INCI name) | Amount (weight %) |
|---|---|
| Oily dispersion of Example $P_1$ | 70 |
| Disteardimonium hectorite (Bentone 38 VCG from Elementis) | 5 |
| Isododecane | qs 100 |
| Propylene carbonate | 1.6 |

**Preparation protocol**

The oily dispersion of Example $P_1$ was diluted with isododecane. The bentone was then dispersed in this solution until a homogeneous mixture was obtained. The propylene carbonate was then added to this homogeneous mixture so as to activate the bentone and thus to obtain a thick mixture.

**Kit Example 3 (comparative kit outside the invention)**

The base-coat makeup composition is composition ($A_1$) as described in Kit Example 1.

**Top coat composition ($B_3$)**

[Tables 4]

| Ingredients (INCI name) | Amount (weight %) |
|---|---|
| Acrylates/polytrimethyl siloxymethacrylate copolymer (Dowsil FA 4002 ID Silicone Acrylate from Dow Corning) | 85 |
| Disteardimonium hectorite (Bentone 38 VCG from Elementis) | 5 |
| Isododecane | qs 100 |
| Propylene carbonate | 1.6 |

**Preparation protocol**

The Acrylates/polytrimethyl siloxymethacrylate polymer was diluted with isododecane. The bentone was then dispersed in this solution until a homogeneous mixture was obtained. The propylene carbonate was then added to this homogeneous mixture so as to activate the bentone and thus to obtain a thick mixture.

**Kit Example 4 (comparative kit outside the invention)**

The base-coat makeup composition is composition ($A_1$) as described in Kit Example 1.

**Top coat composition ($B_4$) (outside the invention)**

[Tables 5]

| Ingredients (INCI name) | Amount (weight %) |
|---|---|
| Acrylates copolymer (Daitosol 5000 AD from Daito Kasei Kogyo) | 100 |

**Comparative tests of resistance of the base-coat and top-coat combination to dye diffusion in the presence of aqueous phase.**

Transparent square PET plates with a side length of 6 cm were cut.

An adhesive disc (Monaderm® ref. PA22/36 double-sided disc, diameter 22/36), the inner circle of which was 22 mm in diameter, was applied, making it possible to control and to delimit the application area. The same amount of product per unit area was thus applied. 0.15 g of the makeup composition (base-coat) was placed in this circle. The deposit was left to dry for 6 hours. One sample was kept for use as a reference (without application of a top-coat fixing composition). On the other samples, 0.15 g of the top-coat fixing composition was applied to cover the first coat of makeup. Each sample was left to dry for 12 hours at room temperature (25° C). The adhesive disc was then removed. The plate covered with this deposit was then dipped in 150 ml of water for 20 minutes. The PET plate was then removed to evaluate the red marks which had diffused through the water and the amount of product remaining on the PET plate. This test makes it possible to evaluate the resistance of the makeup applied to the PET plate after successive applications of the makeup composition (base-coat) and of the fixing composition (top-coat) by observing the ability of the dye to diffuse into the volume of water.

Results:

It was observed that the deposit of the reference sample without a fixing composition (top-coat) dissolved completely in the volume of water after two minutes. It was found that no color remained on the PET plate and that the volume of water was completely colored. This showed that the absence of application of a top-coat composition resulted in

an absence of resistance of the makeup in the presence of water.

The tests showed that the samples treated with the makeup composition (base-coat) followed by the fixing composition (top-coat) according to Kit Examples 1 and 2 according to the invention led to perfect makeup resistance since no dye molecules were found in the water.

On the other hand, Kit Example 3 outside the invention in which the top-coat fixing composition comprises a film-forming polymer of the silicone acrylate copolymer type showed insufficient water resistance, which was reflected by diffusion of the dye in the volume of water, which turned pink. The same is true for Kit Example 4, in which the fixing composition (top-coat) comprises a film-forming latex.

Consequently, the process involving the application of the aqueous makeup composition (base-coat) followed by the application of the anhydrous fixing composition (top-coat) comprising a film-forming polymer consisting of the silicone polyamide/silicone resin mixture or consisting of the dispersion of Example $P_1$ leads to makeup which has good water resistance.

## Claims

**1.** A kit for making up keratin materials, in particular the eyebrows and the skin around the eyebrows, comprising:

a) a first aqueous base-coat makeup composition (A) comprising at least one water-soluble dye, in particular a direct acid dye; and
b) a second top-coat fixing composition (B) comprising a continuous oily phase containing:

i) at least one hydrophobic film-forming polymer other than silicone acrylate copolymers; and
ii) at least one volatile hydrocarbon-based oil.

**2.** The makeup kit as claimed in claim 1, in which the direct acid dye(s) are present in a total content of less than 5%, preferably less than 3%, and more preferentially between 0.01% and 3% by weight relative to the total weight of composition (A).

**3.** The makeup kit as claimed in either of the preceding claims, in which the direct acid dye is chosen from Acid Violet 43 (CI 60730), Acid Green 25 (CI 61570), Acid Blue 80 (CI 61785), Acid Blue 62 (CI 62045), Acid Violet 50 (CI 50325), FD & C Red 4 (CI 14700), Acid Red 4 (CI 14720), Acid Red 88 (CI 15620), D & C Red 7 (CI 15850), FD & C Red 40 (CI 16035), Acid Red 27 (CI 16185), Acid Red 28 (CI 16255), Acid Red 41 (CI 16290), Acid Red 33 (CI 17200), Acid Red 1 (CI 18050), Acid Red 155 (CI 18130), Acid Red 180 (CI 18736), Acid Red 163 (CI 24790), Acid Red 73 (CI 27290), Acid Orange 6 (CI 14270), Acid Orange 7 (CI 15510), Food Orange 2 (CI 15980), Food Yellow 3 (CI 15985), Acid Orange 10 (CI 16230), Acid Orange 24 (CI 20170), Acid Black 1 (CI 20470), Food Black 2 (CI 27755), Food Black 1 (CI 28440), Acid Black 2 (CI 50420), Acid Yellow 9 (CI 13015), Acid Yellow 11 (CI 18820), Acid Yellow 17 (CI 18965), Acid Yellow 23 (CI 19140), Betanin, Bromocresol Green, Food Green 3 (CI 42053), Acid Green 9 (CI 42100), Acid Green 22 (CI 42170), Acid Green 50 (CI 44090), Bromothymol Blue, Acid Blue 1 (CI 42045), Acid Blue 7 (CI 42080), Acid Blue 9 (CI 42090), Acid Blue 104 (CI 42735), Acid Violet 9 (CI 45190), Acid Red 52 (CI 45100), Acid Red 50 (CI 45220), Acid Red 87 (CI 45380), Acid Red 98 (CI 45405), Acid Red 92 (CI 45410), Acid Red 95 (CI 45425), Acid Red 51 (CI 45430), Acid Orange 11 (CI 45370), Solvent Orange 16 (CI 45396), Acid Yellow 73 (CI 45350), Acid Yellow 73 Fluorescein (CI 45340), Acid Yellow 1 (CI 10316) and more particularly one or more dyes chosen from FD & C Red 40 (CI 16035), Acid Red 33 (CI 17200), Acid Blue 1 (CI 42045), Acid Yellow 23 (CI 19140), and mixtures thereof.

**4.** The makeup kit as claimed in any one of the preceding claims, in which, in composition (A), water is present in a content of greater than or equal to 40% by weight, more preferentially ranging from 45% to 90% by weight, in particular ranging from 50% to 85% by weight relative to the total weight of composition (A).

**5.** The makeup kit as claimed in any one of the preceding claims, in which, in composition (A), the aqueous phase comprises at least one pro-penetrating solvent, preferably chosen from glycols such as propanediol, propylene glycol, butylene glycol, pentylene glycol; cyclic glycol esters such as propylene carbonate, butylene carbonate, benzyl alcohol, and mixtures thereof.

**6.** The makeup kit as claimed in claim 5, in which, in composition (A), the pro-penetrating solvent is present in a content of less than or equal to 30%, preferably less than or equal to 25%, and more preferentially ranging from 4% to 25% by weight relative to the total weight of composition (A).

**7.** The makeup kit as claimed in any one of the preceding claims, in which, in composition (A), the pH is less than 6, more preferentially ranging from 2 to less than 6, and better still ranging from 2 to 5.

**8.** The makeup kit as claimed in any one of claims 1 to 7, in which composition (B) comprises at least one hydrophobic film-forming polymer chosen from:

i) a silicone resin
ii) a silicone polyamide
iii) a block ethylenic copolymer, containing at least one first block with a glass transition temperature (Tg) of greater than or equal to 40° C and being totally or partly derived from one or more first monomers, which are such that the homopolymer prepared from these monomers has a glass transition temperature of greater than or equal to 40° C, and at least one second block with a glass transition temperature of less than or equal to 20° C and being derived totally or partly from one or more second monomers, which are such that the homopolymer prepared from these monomers has a glass transition temperature of less than or equal to 20° C, said first block and said second block being connected together via a statistical intermediate segment comprising at least one of said first constituent monomers of the first block and at least one of said second constituent monomers of the second block, and said block copolymer having a polydispersity index I of greater than 2;
iv) an oily dispersion comprising at least:

i) particle(s) including:
a) at least one ethylenic copolymer of $a_1$) ($C_1$-$C_4$)alkyl ($C_1$-$C_4$)(alkyl)acrylate, and of $a_2$) ethylenic monomers comprising one or more carboxyl, anhydride, phosphoric acid, sulfonic acid and/or aryl, such as benzyl, groups; in particular, $a_2$) is a ($C_1$-$C_4$)(alkyl)acrylic acid, more particularly copolymers of ($C_1$-$C_4$)alkyl (meth) acrylate and of (meth)acrylic acid; and
ii) at least one polymeric stabilizer chosen from:

b) polymers of ($C_3$-$C_{12}$)cycloalkyl ($C_1$-$C_6$)(alkyl)acrylate monomers such as isobornyl (meth)acrylates; and
c) copolymers of $c_1$) ($C_3$-$C_{12}$)cycloalkyl ($C_1$-$C_6$)(alkyl)acrylate such as ($C_1$-$C_4$)alkyl (meth)acrylate; and

iii) at least one hydrocarbon-based oil, preferably a volatile hydrocarbon-based oil.

**9.** The makeup kit as claimed in claim 8, in which the silicone resin is of the MQ type and chosen from a resin of trimethyl siloxysilicate type.

**10.** The makeup kit as claimed in claim 8 or 9, in which the silicone resin is present in composition (B) in a resin solids content ranging from 4% to 35% by weight relative to the total weight of the composition, or ranging from 6% to 30% by weight or ranging from 8% to 25% by weight relative to the weight of composition (B).

**11.** The makeup kit as claimed in claim 8, in which the silicone polyamide has the INC name: Nylon-611/dimethicone copolymer.

**12.** The makeup kit as claimed in claim 8 or 11, in which the silicone polyamide content ranges from 5% to 30% by weight; or from 8% to 25% by weight; or from 10% to 25% by weight relative to the total weight of composition (B).

**13.** The makeup kit as claimed in claim 8, in which composition (B) comprises at least one dispersion in isododecane of methyl acrylate/ethyl acrylate/acrylic acid (11.7/75.6/12.7) copolymer particles stabilized with an isobornyl acrylate/methyl acrylate/ethyl acrylate (92/4/4) statistical copolymer stabilizer; the oily dispersion containing in total (stabilizer ii) + particles i)) 10% of acrylic acid, 10% of methyl acrylate, 60% of ethyl acrylate and 20% of isobornyl acrylate.

**14.** The makeup kit as claimed in claim 8 or 13, in which the amount of oily dispersion in composition (B) according to the invention comprises a solids (or active material) content of polymers of particle i) + dispersing polymers ii) ranging from 20% to 45% by weight, relative to the total weight of composition (B), and more preferentially ranging from 25% to 40% by weight relative to the total weight of composition (B).

**15.** The makeup kit as claimed in one of the preceding claims, in which the volatile hydrocarbon-based oil in composition (B) is chosen from:

- $C_8$-$C_{16}$ branched alkanes, for instance $C_8$-$C_{16}$ isoalkanes of petroleum origin;
- linear $C_{11}$-$C_{14}$ alkanes, and mixtures thereof; more preferentially isododecane.

**16.** The makeup kit as claimed in any one of the preceding claims, in which the content of volatile hydrocarbon-based oil(s) in composition (B) is greater than or equal to 20% by weight, or from 30% to 70%, or from 35% to 60% by weight, relative to the total weight of composition (B).

**17.** The makeup kit as claimed in any one of the preceding claims, in which composition (B) is anhydrous.

**18.** The makeup kit as claimed in any one of claims 1 to 16, in which composition (B) is a water-in-oil emulsion, preferably comprising at least one emulsifying surfactant with an HLB of less than or equal to 8, and more particularly ranging from 3 to 8.

**19.** The makeup kit as claimed in any one of the preceding claims, in which composition (A) and/or composition (B) also comprise at least one dyestuff, in particular chosen from pigments, nacres and mixtures thereof.

**20.** A cosmetic process for making up keratin materials, in particular the eyebrows and the skin around the eyebrows, comprising:

- at least a first step of applying a first coat of composition (A) as defined in any one of the preceding claims to said keratin materials, and
- at least a second step of applying, to the coat formed by composition (A), at least a second coat of composition (B) as defined in any one of the preceding claims.

## Patentansprüche

**1.** Kit zum Schminken von Keratinmaterialien, insbesondere der Augenbrauen und der Haut um die Augenbrauen, umfassend:

a) eine erste wässrige Base-Coat-Schminkzusammensetzung (A), umfassend mindestens einen wasserlöslichen Farbstoff, insbesondere einen direktziehenden Säurefarbstoff; und
b) eine zweite Top-Coat-Fixierzusammensetzung (B), umfassend eine kontinuierliche ölige Phase, enthaltend:

i) mindestens ein hydrophobes filmbildendes Polymer, das von Silikonacrylat-Copolymeren verschieden ist; und
ii) mindestens ein flüchtiges Öl auf Kohlenwasserstoffbasis.

**2.** Schminkkit nach Anspruch 1, wobei der direktziehende Säurefarbstoff bzw. die direktziehenden Säurefarbstoffe in einem Gesamtgehalt von weniger als 5 Gew.-%, vorzugsweise weniger als 3 Gew.-%% und weiter bevorzugt zwischen 0,01 und 3 Gew.-%, bezogen auf das Gesamtgewicht der Zusammensetzung (A), vorliegt bzw. vorliegen.

**3.** Schminkkit nach einem der vorhergehenden Ansprüche, wobei der direktziehende Säurefarbstoff ausgewählt ist aus Acid Violet 43 (CI 60730), Acid Green 25 (CI 61570), Acid Blue 80 (CI 61785), Acid Blue 62 (CI 62045), Acid Violet 50 (CI 50325), FD & C Red 4 (CI 14700), Acid Red 4 (CI 14720), Acid Red 88 (CI 15620), D & C Red 7 (CI 15850), FD & C Red 40 (CI 16035), Acid Red 27 (CI 16185), Acid Red 28 (CI 16255), Acid Red 41 (CI 16290), Acid Red 33 (CI 17200), Acid Red 1 (CI 18050), Acid Red 155 (CI 18130), Acid Red 180 (CI 18736), Acid Red 163 (CI 24790), Acid Red 73 (CI 27290), Acid Orange 6 (CI 14270), Acid Orange 7 (CI 15510), Food Orange 2 (CI 15980), Food Yellow 3 (CI 15985), Acid Orange 10 (CI 16230), Acid Orange 24 (CI 20170), Acid Black 1 (CI 20470), Food Black 2 (CI 27755), Food Black 1 (CI 28440), Acid Black 2 (CI 50420), Acid Yellow 9 (CI 13015), Acid Yellow 11 (CI 18820), Acid Yellow 17 (CI 18965), Acid Yellow 23 (CI 19140), Betanin, Bromkresolgrün, Food Green 3 (CI 42053), Acid Green 9 (CI 42100), Acid Green 22 (CI 42170), Acid Green 50 (CI 44090), Bromothymolblau, Acid Blue 1 (CI 42045), Acid Blue 7 (CI 42080), Acid Blue 9 (CI 42090), Acid Blue 104 (CI 42735), Acid Violet 9 (CI 45190), Acid Red 52 (CI 45100), Acid Red 50 (CI 45220), Acid Red 87 (CI 45380), Acid Red 98 (CI 45405), Acid Red 92 (CI 45410), Acid Red 95 (CI 45425), Acid Red 51 (CI 45430), Acid Orange 11 (CI 45370), Solvent Orange 16 (CI 45396), Acid Yellow 73 (CI 45350), Acid Yellow 73 Fluorescein (CI 45340), Acid Yellow 1 (CI 10316) und spezieller einem oder mehreren Farbstoffen ausgewählt aus FD & C Red 40 (CI 16035), Acid Red 33 (CI 17200), Acid Blue 1 (CI 42045), Acid Yellow 23 (CI 19140) und Mischungen davon.

4. Schminkkit nach einem der vorhergehenden Ansprüche, wobei in Zusammensetzung (A) Wasser in einem Gehalt größer oder gleich 40 Gew.-%, weiter bevorzugt im Bereich von 45 bis 90 Gew.-%, insbesondere im Bereich von 50 bis 85 Gew.-%, bezogen auf das Gesamtgewicht der Zusammensetzung (A), vorliegt.

5. Schminkkit nach einem der vorhergehenden Ansprüche, wobei in Zusammensetzung (A) die wässrige Phase mindestens ein penetrationsförderndes Lösungsmittel umfasst, das vorzugsweise aus Glykolen wie Propandiol, Propylenglykol, Butylenglykol, Pentylenglykol; cyclischen Glykolestern wie Propylencarbonat, Butylencarbonat, Benzylalkohol und Mischungen davon ausgewählt ist.

6. Schminkkit nach Anspruch 5, wobei in der Zusammensetzung (A) das penetrationsfördernde Lösungsmittel in einem Gehalt kleiner oder gleich 30 Gew.-%, vorzugsweise kleiner oder gleich 25 Gew.-% und weiter bevorzugt im Bereich von 4 bis 25 Gew.-%, bezogen auf das Gesamtgewicht der Zusammensetzung (A), vorliegt.

7. Schminkkit nach einem der vorhergehenden Ansprüche, wobei in Zusammensetzung (A) der pH-Wert kleiner als 6 ist, weiter bevorzugt im Bereich von 2 bis weniger als 6 liegt und noch besser im Bereich von 2 bis 5 liegt.

8. Schminkkit nach einem der Ansprüche 1 bis 7, wobei Zusammensetzung (B) mindestens ein hydrophobes filmbildendes Polymer umfasst, das ausgewählt ist aus:

i) einem Silikonharz
ii) einem Silikonpolyamid
iii) einem ethylenischen Blockcopolymer, das mindestens einen ersten Block mit einer Glasübergangstemperatur (Tg) größer oder gleich 40 °C, der sich ganz oder teilweise von einem oder mehreren ersten Monomeren ableitet, die so beschaffen sind, dass das aus diesen Monomeren hergestellte Homopolymer eine Glasübergangstemperatur größer oder gleich 40 °C aufweist, und mindestens einen zweiten Block mit einer Glasübergangstemperatur kleiner oder gleich 20 °C, der sich ganz oder teilweise von einem oder mehreren zweiten Monomeren ableitet, die derart beschaffen sind, dass das aus diesen Monomeren hergestellte Homopolymer eine Glasübergangstemperatur kleiner oder gleich 20 °C aufweist, enthält, wobei der erste Block und der zweite Block über ein statistisches Zwischensegment, das mindestens eines der ersten Aufbaumonomere des ersten Blocks und mindestens eines der zweiten Aufbaumonomere des zweiten Blocks umfasst, miteinander verbunden sind und wobei das Blockcopolymer einen Polydispersitätsindex I von mehr als 2 aufweist;
iv) eine ölige Dispersion, die mindestens Folgendes umfasst:

i) ein oder mehrere Teilchen, enthaltend:
a) mindestens ein ethylenisches Copolymer von $a_1$) ($C_1$-$C_4$)-Alkyl-($C_1$-$C_4$)(alkyl) acrylat und $a_2$) ethylenischen Monomeren mit einer oder mehreren Carboxylgruppen, Anhydridgruppen, Phosphonsäuregruppen, Sulfonsäuregruppen und/oder Arylgruppen, wie Benzylgruppen; wobei es sich bei $a_2$) insbesondere um eine ($C_1$-$C_4$)(Alkyl)acrylsäure handelt, spezieller Copolymere von ($C_1$-$C_4$)-Alkyl (meth) acrylat und (Meth) acrylsäure; und
ii) mindestens einen polymeren Stabilisator, ausgewählt aus:

b) Polymeren von ($C_3$-$C_{12}$)-Cycloalkyl-($C_1$-$C_6$)(alkyl)acrylatmonomeren wie Isobornyl(meth)acrylaten; und
c) Copolymeren von $c_1$) ($C_3$-$C_{12}$)-Cycloalkyl-($C_1$-$C_6$)(alkyl) acrylat, wie ($C_1$-$C_4$)-Alkyl (meth) acrylat; und
iii) mindestens einem Öl auf Kohlenwasserstoffbasis, vorzugsweise einem flüchtigen Öl auf Kohlenwasserstoffbasis.

9. Schminkkit nach Anspruch 8, wobei das Silikonharz vom MQ-Typ ist und aus einem Harz vom Trimethylsiloxysilikat-Typ ausgewählt ist.

10. Schminkkit nach Anspruch 8 oder 9, wobei das Silikonharz in Zusammensetzung (B) in einem Harzfeststoffgehalt im Bereich von 4 bis 35 Gew.-%, bezogen auf das Gesamtgewicht der Zusammensetzung, oder im Bereich von 6 bis 30 Gew.-% oder im Bereich von 8 bis 25 Gew.-%, bezogen auf das Gewicht der Zusammensetzung (B), vorliegt.

11. Schminkkit nach Anspruch 8, wobei das Silikonpolyamid den folgenden INC-Namen hat: Nylon-611/Dimethicone Copolymer.

12. Schminkkit nach Anspruch 8 oder 11, wobei der Gehalt an Silikonpolyamid im Bereich von 5 bis 30 Gew.-% oder von 8

bis 25 Gew.-% oder von 10 bis 25 Gew.-%, bezogen auf das Gesamtgewicht der Zusammensetzung (B), liegt.

**13.** Schminkkit nach Anspruch 8, wobei die Zusammensetzung (B) mindestens eine Dispersion von Teilchen aus Methylacrylat/Ethylacrylat/Acrylsäure(11,7/75,6/12,7)-Copolymer, die mit einem statistischen Isobornylacrylat/Methylacrylat/Ethylacrylat(92/4/4)-Copolymer als Stabilisator stabilisiert sind, in Isododecan umfasst; wobei die ölige Dispersion insgesamt (Stabilisator ii) + Teilchen i)) 10 % Acrylsäure, 10 % Methylacrylat, 60 % Ethylacrylat und 20 % Isobornylacrylat enthält.

**14.** Schminkkit nach Anspruch 8 oder 13, wobei die Menge an öliger Dispersion in der erfindungsgemäßen Zusammensetzung (B) einen Feststoffgehalt (oder Aktivsubstanzgehalt) an Polymeren von Teilchen i) + dispergierend wirkenden Polymeren ii) im Bereich von 20 bis 45 Gew.-%, bezogen auf das Gesamtgewicht der Zusammensetzung (B), und weiter bevorzugt im Bereich von 25 bis 40 Gew.-%, bezogen auf das Gesamtgewicht der Zusammensetzung (B), umfasst.

**15.** Schminkkit nach einem der vorhergehenden Ansprüche, wobei das flüchtige Öl auf Kohlenwasserstoffbasis in Zusammensetzung (B) ausgewählt ist aus:

- verzweigten $C_8$-$C_{16}$-Alkanen, z. B. $C_8$-$C_{16}$-Isoalkanen petrochemischen Ursprungs;
- linearen $C_{11}$-$C_{14}$-Alkanen und Gemischen davon; weiter bevorzugt Isododecan.

**16.** Schminkkit nach einem der vorhergehenden Ansprüche, wobei der Gehalt an flüchtigem Öl bzw. flüchtigen Ölen auf Kohlenwasserstoffbasis in Zusammensetzung (B) größer oder gleich 20 Gew.-% oder 30 bis 70 Gew.-% oder 35 bis 60 Gew.-%, bezogen auf das Gesamtgewicht der Zusammensetzung (B), beträgt.

**17.** Schminkkit nach einem der vorhergehenden Ansprüche, wobei Zusammensetzung (B) wasserfrei ist.

**18.** Schminkkit nach einem der Ansprüche 1 bis 16, wobei Zusammensetzung (B) eine Wasser-in-Öl-Emulsion ist, die vorzugsweise mindestens ein emulgierendes Tensid mit einem HLB-Wert kleiner oder gleich 8 und insbesondere im Bereich von 3 bis 8 umfasst.

**19.** Schminkkit nach einem der vorhergehenden Ansprüche, wobei Zusammensetzung (A) und/oder Zusammensetzung (B) außerdem mindestens einen Farbstoff enthalten, der insbesondere aus Pigmenten, Perlglanzmitteln und Mischungen davon ausgewählt ist.

**20.** Kosmetisches Verfahren zum Schminken von Keratinmaterialien, insbesondere der Augenbrauen und der Haut um die Augenbrauen, umfassend:

- mindestens einen ersten Schritt des Aufbringens einer ersten Schicht von Zusammensetzung (A) gemäß einem der vorhergehenden Ansprüche auf die Keratinmaterialien und
- mindestens einen zweiten Schritt des Aufbringens mindestens einer zweiten Schicht von Zusammensetzung (B) gemäß einem der vorhergehenden Ansprüche auf die durch Zusammensetzung (A) gebildete Schicht.

## Revendications

**1.** Kit pour le maquillage des matières kératiniques, en particulier les sourcils et la peau autour des sourcils, comprenant :

a) une première composition aqueuse de maquillage de couche de base (A) comprenant au moins un colorant hydrosoluble, en particulier un colorant acide direct ; et
b) une deuxième composition de fixation de couche supérieure (B) comprenant une phase huileuse continue contenant :

i) au moins un polymère filmogène hydrophobe autre que des copolymères silicone acrylate ; et
ii) au moins une huile volatile hydrocarbonée.

**2.** Kit de maquillage selon la revendication 1, dans lequel le ou les colorants acides directs sont présents en une teneur totale inférieure à 5 %, de préférence inférieure à 3 %, et plus préférentiellement entre 0,01 % et 3 % en poids par rapport au poids total de la composition (A).

**3.** Kit de maquillage selon l'une quelconque des revendications précédentes, dans lequel le colorant acide direct est choisi parmi Acid Violet 43 (CI 60730), Acid Green 25 (CI 61570), Acid Blue 80 (CI 61785), Acid Blue 62 (CI 62045), Acid Violet 50 (CI 50325), FD & C Red 4 (CI 14700), Acid Red 4 (CI 14720), Acid Red 88 (CI 15620), D & C Red 7 (CI 15850), FD & C Red 40 (CI 16035), Acid Red 27 (CI 16185), Acid Red 28 (CI 16255), Acid Red 41 (CI 16290), Acid Red 33 (CI 17200), Acid Red 1 (CI 18050), Acid Red 155 (CI 18130), Acid Red 180 (CI 18736), Acid Red 163 (CI 24790), Acid Red 73 (CI 27290), Acid Orange 6 (CI 14270), Acid Orange 7 (CI 15510), Food Orange 2 (CI 15980), Food Yellow 3 (CI 15985), Acid Orange 10 (CI 16230), Acid Orange 24 (CI 20170), Acid Black 1 (CI 20470), Food Black 2 (CI 27755), Food Black 1 (CI 28440), Acid Black 2 (CI 50420), Acid Yellow 9 (CI 13015), Acid Yellow 11 (CI 18820), Acid Yellow 17 (CI 18965), Acid Yellow 23 (CI 19140), Bétanine, Bromocresol Green, Food Green 3 (CI 42053), Acid Green 9 (CI 42100), Acid Green 22 (CI 42170), Acid Green 50 (CI 44090), Bromothymol Blue, Acid Blue 1 (CI 42045), Acid Blue 7 (CI 42080), Acid Blue 9 (CI 42090), Acid Blue 104 (CI 42735), Acid Violet 9 (CI 45190), Acid Red 52 (CI 45100), Acid Red 50 (CI 45220), Acid Red 87 (CI 45380), Acid Red 98 (CI 45405), Acid Red 92 (CI 45410), Acid Red 95 (CI 45425), Acid Red 51 (CI 45430), Acid Orange 11 (CI 45370), Solvent Orange 16 (CI 45396), Acid Yellow 73 (CI 45350), Acid Yellow 73 Fluorescein (CI 45340), Acid Yellow 1 (CI 10316) et plus particulièrement un ou plusieurs colorants choisis parmi FD & C Red 40 (CI 16035), Acid Red 33 (CI 17200), Acid Blue 1 (CI 42045), Acid Yellow 23 (CI 19140), et leurs mélanges.

**4.** Kit de maquillage selon l'une quelconque des revendications précédentes, dans lequel, dans la composition (A), l'eau est présente en une teneur supérieure ou égale à 40 % en poids, plus préférentiellement allant de 45 % à 90 % en poids, en particulier allant de 50 % à 85 % en poids par rapport au poids total de la composition (A).

**5.** Kit de maquillage selon l'une quelconque des revendications précédentes, dans lequel, dans la composition (A), la phase aqueuse comprend au moins un solvant pro-pénétrant, de préférence choisi parmi les glycols tels que le propanediol, le propylène glycol, le butylène glycol, le pentylène glycol ; les esters de glycol cycliques tels que le carbonate de propylène, le carbonate de butylène, l'alcool benzylique, et leurs mélanges.

**6.** Kit de maquillage selon la revendication 5, dans lequel, dans la composition (A), le solvant pro-pénétrant est présent en une teneur inférieure ou égale à 30 %, de préférence inférieure ou égale à 25 %, et plus préférentiellement allant de 4 % à 25 % en poids par rapport au poids total de la composition (A).

**7.** Kit de maquillage selon l'une quelconque des revendications précédentes, dans lequel, dans la composition (A), le pH est inférieur à 6, plus préférentiellement allant de 2 à moins de 6, et mieux allant de 2 à 5.

**8.** Kit de maquillage selon l'une quelconque des revendications 1 à 7, dans lequel la composition (B) comprend au moins un polymère filmogène hydrophobe choisi parmi :

i) une résine de silicone
ii) un silicone polyamide
iii) un copolymère éthylénique séquencé, contenant au moins un premier bloc ayant une température de transition vitreuse (Tg) supérieure ou égale à 40 °C et qui est totalement ou partiellement issu d'un ou plusieurs premiers monomères, qui sont tels que l'homopolymère préparé à partir de ces monomères a une température de transition vitreuse supérieure ou égale à 40 °C, et au moins un deuxième bloc ayant une température de transition vitreuse inférieure ou égale à 20 °C et qui est totalement ou partiellement issu d'un ou de plusieurs deuxièmes monomères, qui sont tels que l'homopolymère préparé à partir de ces monomères a une température de transition vitreuse inférieure ou égale à 20 °C, ledit premier bloc et ledit deuxième bloc étant reliés entre eux via un segment intermédiaire statistique comprenant au moins un desdits premiers monomères constitutifs du premier bloc et au moins un desdits seconds monomères constitutifs du deuxième bloc, et ledit copolymère séquencé ayant un indice de polydispersité I supérieur à 2 ;
iv) une dispersion huileuse comprenant au moins :

i) une ou plusieurs particule(s) comprenant :
a) au moins un copolymère éthylénique de $a_1$) ($C_1$-$C_4$)(alkyl)acrylate de ($C_1$-$C_4$)alkyle et de $a_2$) monomères éthyléniques comprenant un ou plusieurs groupes carboxyle, anhydride, acide phosphorique, acide sulfonique et/ou aryle, tel que benzyle ; en particulier, $a_2$) est un acide ($C_1$-$C_4$)(alkyl) acrylique, plus particulièrement des copolymères de (méth)acrylate de ($C_1$-$C_4$)alkyle et d'acide (méth)acrylique ; et
ii) au moins un stabilisant polymérique choisi parmi :

b) des polymères de monomères de ($C_1$-$C_6$)(alkyl)acrylate de ($C_3$-$C_{12}$)cycloalkyle tels que des (méth)acrylates d'isobornyle ; et

c) des copolymères de $c_1$) ($C_1$-$C_6$)(alkyl) acrylate de ($C_3$-$C_{12}$)cycloalkyle tels que (méth) acrylate de ($C_1$-$C_4$)alkyle ; et

iii) au moins une huile hydrocarbonée, de préférence une huile volatile hydrocarbonée.

**9.** Kit de maquillage selon la revendication 8, dans lequel la résine de silicone est du type MQ et choisie parmi une résine de type siloxysilicate de triméthyle.

**10.** Kit de maquillage selon la revendication 8 ou 9, dans lequel la résine de silicone est présente dans la composition (B) en une teneur de solides de résine allant de 4 % à 35 % en poids par rapport au poids total de la composition, ou allant de 6 % à 30 % en poids ou allant de 8 % à 25 % en poids par rapport au poids de la composition (B).

**11.** Kit de maquillage selon la revendication 8, dans lequel le silicone polyamide a le nom INC : Nylon-611/dimethicone copolymer.

**12.** Kit de maquillage selon la revendication 8 ou 11, dans lequel la teneur en silicone polyamide se situe dans la plage de 5 % à 30 % en poids ; ou de 8 % à 25 % en poids ; ou de 10 % à 25 % en poids par rapport au poids total de la composition (B).

**13.** Kit de maquillage selon la revendication 8, dans lequel la composition (B) comprend au moins une dispersion dans l'isododécane de particules de copolymère d'acrylate de méthyle/acrylate d'éthyle/acide acrylique (11,7/75,6/12,7) stabilisées par un stabilisant de copolymère statistique d'acrylate d'isobornyle/acrylate de méthyle/acrylate d'éthyle (92/4/4) ; la dispersion huileuse contenant au total (stabilisant ii) + particules i)) 10 % d'acide acrylique, 10 % d'acrylate de méthyle, 60 % d'acrylate d'éthyle et 20 % d'acrylate d'isobornyle.

**14.** Kit de maquillage selon la revendication 8 ou 13, dans lequel la quantité de dispersion huileuse dans la composition (B) selon l'invention comprend une teneur en solides (ou matière active) de polymères de particule i) + polymères dispersants ii) allant de 20 % à 45 % en poids par rapport au poids total de la composition (B), et plus préférentiellement allant de 25 % à 40 % en poids par rapport au poids total de la composition (B).

**15.** Kit de maquillage selon l'une des revendications précédentes, dans lequel l'huile hydrocarbonée volatile de la composition (B) est choisie parmi :

- des alcanes ramifiés en $C_8$-$C_{16}$, par exemple des isoalcanes en $C_8$-$C_{16}$ d'origine pétrolière ;
- des alcanes linéaires en $C_{11}$-$C_{14}$, et leurs mélanges ; plus préférentiellement l'isododécane.

**16.** Kit de maquillage selon l'une quelconque des revendications précédentes, dans lequel la teneur en huile(s) hydrocarbonée(s) volatile(s) dans la composition (B) est supérieure ou égale à 20 % en poids, ou de 30 % à 70 %, ou de 35 % à 60 % en poids, par rapport au poids total de la composition (B).

**17.** Kit de maquillage selon l'une quelconque des revendications précédentes, dans lequel la composition (B) est anhydre.

**18.** Kit de maquillage selon l'une quelconque des revendications 1 à 16, dans lequel la composition (B) est une émulsion eau-dans-huile, comprenant de préférence au moins un tensioactif émulsifiant doté d'un HLB inférieur ou égal à 8, et plus particulièrement allant de 3 à 8.

**19.** Kit de maquillage selon l'une quelconque des revendications précédentes, dans lequel la composition (A) et/ou la composition (B) comprennent également au moins une matière colorante, en particulier choisie parmi les pigments, les nacres et leurs mélanges.

**20.** Procédé cosmétique pour le maquillage de matières kératiniques, en particulier les sourcils et la peau autour des sourcils, comprenant :

- au moins une première étape d'application sur lesdites matières kératiniques d'un premier revêtement de composition (A) telle que définie dans l'une quelconque des revendications précédentes, et
- au moins une deuxième étape d'application, sur le revêtement formé par la composition (A), d'au moins une

deuxième couche de composition (B) telle que définie dans l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2936420 A1
- WO 2008155059 A
- US 2676182 A
- US 3627851 A
- US 3772247 A
- US 5248739 A
- US 5082706 A
- US 5319040 A
- US 5302685 A
- US 4935484 A
- US 5110890 A
- WO 2005075542 A
- US 5874069 A
- US 5919441 A
- US 6051216 A
- US 5981680 A
- EP 1411069 A
- EP 1882709 A
- FR 2679771
- EP 1184426 A
- US 4887622 A
- FR 2796529
- FR 2722380
- US 5492426 A
- FR 2761959
- FR 2792618


### Non-patent literature cited in the description


- Encyclopedia of Polymer Science and Engineering. John Wiley & Sons, 1989, vol. 15, 265-270
- Polymer Handbook. John Wiley, 1989
- The HLB System. A Time-Saving Guide to Emulsifier Selection. ICI Americas Inc., 1984
- **VAN DE HULST, H.C.** Light Scattering by Small Particles. Wiley, 1957, vol. 9,10